# EUROPEAN PATENT SPECIFICATION

(11) **EP 3 013 819 B1**
(45) Date of publication and mention of the grant of the patent: **15.08.2018**
(21) Application number: 14732176.4
(22) Date of filing: 23.06.2014
(51) Int. Cl.: C07D 417/10, A01N 43/80, C07D 495/10

(54) **ISOTHIAZOLINE COMPOUNDS FOR COMBATING INVERTEBRATE PESTS**
ISOTHIAZOLINVERBINDUNGEN ZUR BEKÄMPFUNG VON WIRBELLOSEN SCHÄDLINGEN
COMPOSÉS ISOTHIAZOLINIQUES POUR COMBATTRE LES INVERTÉBRÉS NUISIBLES

(30) Priority: 24.06.2013 US 201361838370 P; 28.10.2013 US 201361896137 P
(43) Date of publication of application: 04.05.2016
(73) Proprietor: Merial, Inc., Duluth, GA 30096 (US)
(72) Inventor: BINDSCHÄDLER, Pascal, 67354 Römerberg (DE); VON DEYN, Wolfgang, 67435 Neustadt (DE); KÖRBER, Karsten, 69214 Eppelheim (DE); CULBERTSON, Deborah L., Fuquay Varina, North Carolina 27526 (US); BRAUN, Franz Josef, Durham, North Carolina 27703 (US); GUNJIMA, Koshi, Toyohashi-city Aichi Prefecture 441-8021 (JP)
(74) Representative: D Young & Co LLP
(86) International application number: PCT/EP2014/063106
(87) International publication number: WO 2014/206911

(56) References cited:
- EP-A1- 1 731 512
- WO-A1-2011/092287
- WO-A1-2013/037626
- WO-A1-2013/092943

## Description

The present invention relates to isothiazoline compounds which are useful for combating or controlling invertebrate pests, in particular arthropod pests and nematodes. The invention also relates to a non-therapeutic method for controlling invertebrate pests by using these compounds and to plant propagation material and to an agricultural and a veterinary composition comprising said compounds.

The invention is defined by the claims only. The description which follows is subject to this limitation. All aspects and embodiments in the description which are not encompassed in the scope of the claims do not form part of the invention.

Invertebrate pests and in particular arthropods and nematodes destroy growing and harvested crops and attack wooden dwelling and commercial structures, causing large economic loss to the food supply and to property. While a large number of pesticidal agents are known, due to the ability of target pests to develop resistance to said agents, there is an on-going need for new agents for combating invertebrate pests, in particular insects, arachnids and nematodes.

Related insecticidal aryl isothiazoline compounds are described in WO 2013/037626. However, this document does not describe compounds having the characteristic substituents and substituents' arrangement as claimed in the present invention.

Related insecticidal aryl azoline compounds are further described in WO 2011/092287, WO 2011/073444, WO 2010/090344, WO 2009/112275 and WO 97/23212. These documents do not describe compounds having the characteristic substituents and substituents' arrangement as claimed in the present invention, either.

It is an object of the present invention to provide compounds that have a good pesticidal activity, in particular insecticidal activity, and show a broad activity spectrum against a large number of different invertebrate pests, especially against difficult to control arthropod pests and/or nematodes.

It has been found that these objectives can be achieved by isothiazoline compounds of the formula I below, by their steroisomers and by their salts, in particular their agriculturally or veterinarily acceptable salts.

Therefore, in a first aspect, the present description relates to isothiazoline compounds of formula I 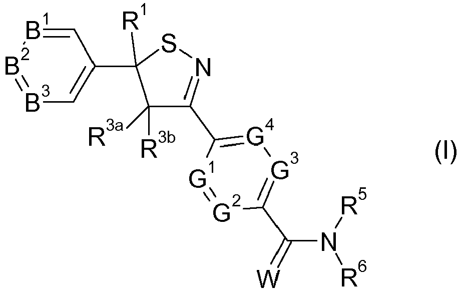 wherein
B¹, B² and B³ are each independently selected from the group consisting of N and CR², with the proviso that at most two of B¹, B² and B³ are N;
G¹, G², G³ and G⁴ are each independently selected from the group consisting of N and CR⁴, with the proviso that at most two of G¹, G², G³ and G⁴ are N;
W is O or S;
R¹ is selected from the group consisting of C₁-C₄-alkyl, C₁-C₄-haloalkyl, C₁-C₄-alkoxy-C₁-C₄-alkyl-, C₁-C₄-haloalkoxy-C₁-C₄-alkyl-, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl and -C(=O)OR¹⁵;
each R² is independently selected from the group consisting of hydrogen, halogen, cyano, azido, nitro, -SCN, -SF₅, C₁-C₆-alkyl, C₃-C₈-cycloalkyl, C₂-C₆-alkenyl, C₂-C₆-alkynyl, wherein the four last mentioned aliphatic and cycloaliphatic radicals may be partially or fully halogenated and/or may be substituted by one or more radicals R⁸;
   -Si(R¹²)₃, -OR⁹, -S(O)ₙR⁹, -NR^{10a}R^{10b},
   phenyl which may be substituted by 1, 2, 3, 4 or 5 radicals R¹¹, and a 3-, 4-, 5-, 6-, 7-, 8-, 9- or 10-membered saturated, partially unsaturated or maximally unsaturated heteromonocyclic or heterobicyclic ring containing 1, 2, 3 or 4 heteroatoms or heteroatom groups selected from N, O, S, NO, SO and SO₂, as ring members, where the heteromono- or heterobicyclic ring may be substituted by one or more radicals R¹¹;
R^{3a}, R^{3b} are each independently selected from the group consisting of hydrogen, halogen, hydroxyl, -CO₂R^{3d}, C₁-C₃-alkyl, C₁-C₃-haloalkyl, C₂-C₃-alkenyl, C₂-C₃-alkynyl, C₁-C₃-alkoxy, C₁-C₃-haloalkoxy, C₁-C₃-alkylthio, C₁-C₃-haloalkylthio, C₁-C₃-alkylsulfonyl and C₁-C₃-haloalkylsulfonyl; or
   R^{3a} and R^{3b} together form a group =O, =C(R^{3c})₂, =NOH or =NOCH₃;
each R^{3c} is independently selected from the group consisting of hydrogen, halogen, CH₃ and CF₃;
R^{3d} is selected from the group consisting of hydrogen, C₁-C₆-alkyl and C₁-C₃-alkyloxy-C₁-C₃-alkyl-;
each R⁴ is independently selected from the group consisting of hydrogen, halogen, cyano, azido, nitro, -SCN, -SF₅, C₁-C₆-alkyl which may be partially or fully halogenated and/or may be substituted by one or more radicals R⁸, C₃-C₈-cycloalkyl which may be partially or fully halogenated and/or may be substituted by one or more radicals R⁸, C₂-C₆-alkenyl which may be partially or fully halogenated and/or may be substituted by one or more radicals R⁸, C₂-C₆-alkynyl which may be partially or fully halogenated and/or may be substituted by one or more radicals R⁸,
   -Si(R¹²)₃, -OR⁹, -S(O)ₙR⁹, -NR^{10a}R^{10b},
   phenyl which may be substituted by 1, 2, 3, 4 or 5 radicals R¹¹, and a 3-, 4-, 5-, 6-, 7-, 8-, 9- or 10-membered saturated, partially unsaturated or maximally unsaturated heteromonocyclic or heterobicyclic ring containing 1, 2, 3 or 4 heteroatoms or heteroatom groups selected from N, O, S, NO, SO and SO₂, as ring members, where the heteromonocyclic or heterobicyclic ring may be substituted by one or more radicals R¹¹;
R⁵ and R⁶, together with the nitrogen atom to which they are bound, form a 3-, 4-, 5-, 6-, 7-, 8-, 9- or 10-membered saturated, partially unsaturated or maximally unsaturated heteromonocyclic or heterobicyclic ring [the ring being of course bound via this nitrogen atom to the group C(=W)], where the ring may further contain 1, 2, 3 or 4 heteroatoms or heteroatom-containing groups selected from O, S, N, NH, SO, and SO₂, as ring members, wherein the heterocyclic ring may be substituted with 1, 2, 3, 4, 5, 6, 7 or 8 substituents R⁷;
each R⁷ is independently selected from the group consisting of halogen, cyano, azido, nitro, -SCN, -SF₅, C₁-C₆-alkyl, C₃-C₈-cycloalkyl, C₂-C₆-alkenyl, C₂-C₆-alkynyl, wherein the four last-mentioned aliphatic and cycloaliphatic radicals may be partially or fully halogenated and/or may be substituted by one or more radicals R⁸;
   -Si(R¹²)₃, -OR⁹, -OSO₂R⁹, -S(O)ₙR⁹, -N(R^{10a})R^{10b}, -C(=O)N(R^{10a})R^{10b},
   -C(=S)N(R^{10a})R^{10b}, -C(=O)OR⁹, -C(=O)R⁸,
   phenyl which may be substituted with 1, 2, 3, 4 or 5 substituents R¹¹; and
   a 3-, 4-, 5-, 6-, 7-, 8-, 9- or 10-membered saturated, partially unsaturated or maximally unsaturated heteromonocyclic or heterobicyclic ring containing 1, 2, 3 or 4 heteroatoms or heteroatom groups selected from N, O, S, NO, SO and SO₂, as ring members, where the heteromonocyclic or heterobicyclic ring may be substituted by one or more radicals R¹¹; and additionally or alternatively, two radicals R⁷ present on the same carbon atom of the 3-, 4-, 5-, 6-, 7-, 8-, 9- or 10-membered saturated, partially unsaturated or maximally unsaturated heteromonocyclic or heterobicyclic ring may together form a group =O (this also being called an oxo substituent) or =S;
each R⁸ is independently selected from the group consisting of cyano, azido, nitro, -SCN, -SF₅, C₃-C₈-cycloalkyl, C₃-C₈-halocycloalkyl, where the cycloaliphatic moieties in the two last-mentioned radicals may be substituted by one or more radicals R¹³;
   -Si(R¹²)₃, -OR⁹, -OSO₂R⁹, -S(O)ₙR⁹, -N(R^{10a})R^{10b}, -C(=O)N(R^{10a})R^{10b},
   -C(=S)N(R^{10a})R^{10b}, -C(=O)OR⁹,
   phenyl, optionally substituted with 1, 2, 3, 4 or 5 substituents R¹⁶, and a 3-, 4-, 5-, 6- or 7-membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶,
   or
   two R⁸ present on the same carbon atom of an alkyl, alkenyl, alkynyl or cycloalkyl group together form a group =O, =C(R¹³)₂; =S; =S(O)ₘ(R¹⁵)₂, =S(O)ₘR¹⁵N(R^{14a})R^{14b}, =NR^{10a}, =NOR⁹; or =NN(R^{10a})R^{10b};
   or
   two radicals R⁸, together with the carbon atoms of an alkyl, alkenyl, alkynyl or cycloalkyl group which they are bonded to, form a 3-, 4-, 5-, 6-, 7- or 8-membered saturated or partially unsaturated carbocyclic or heterocyclic ring, where the heterocyclic ring comprises 1, 2, 3 or 4 heteroatoms or heteroatom groups independently selected from N, O, S, NO, SO and SO₂, as ring members, and where the carbocyclic or heterocyclic ring is optionally substituted with one or more substituents R¹⁶; and
   R⁸ as a substituent on a cycloalkyl ring is additionally selected from the group consisting of C₁-C₆-alkyl, C₁-C₆-haloalkyl, C₂-C₆-alkenyl, C₂-C₆-haloalkenyl, C₂-C₆-alkynyl and C₂-C₆-haloalkynyl, where the aliphatic moieties in these six radicals may be substituted by one or more radicals R¹³; and R⁸ in the group -C(=O)R⁸ is additionally selected from the group consisting of hydrogen, halogen, C₁-C₆-alkyl, C₁-C₆-haloalkyl, C₂-C₆-alkenyl, C₂-C₆-haloalkenyl, C₂-C₆-alkynyl and C₂-C₆-haloalkynyl, where the aliphatic moieties in the six last-mentioned radicals may be substituted by one or more radicals R¹³;
each R⁹ is independently selected from the group consisting of hydrogen, cyano, C₁-C₆-alkyl, C₁-C₆-haloalkyl, C₃-C₈-cycloalkyl, C₃-C₈-cycloalkyl-C₁-C₄-alkyl-, C₃-C₈-halocycloalkyl, C₂-C₆-alkenyl, C₂-C₆-haloalkenyl, C₂-C₆-alkynyl, C₂-C₆-haloalkynyl, where the aliphatic and cycloaliphatic moieties in the nine last-mentioned radicals may be substituted by one or more radicals R¹³,
   -C₁-C₆-alkyl-C(=O)OR¹⁵, -C₁-C₆-alkyl-C(=O)N(R^{14a})R^{14b},
   -C₁-C₆-alkyl-C(=S)N(R^{14a})R^{14b}, -C₁-C₆-alkyl-C(=NR¹⁴)N(R^{14a})R^{14b},
   -Si(R¹²)₃, -S(O)ₙR¹⁵, -S(O)ₙN(R^{14a})R^{14b}, -N(R^{10a})R^{10b}, -N=C(R¹³)₂, -C(=O)R¹³, -C(=O)N(R^{14a})R^{14b}, -C(=S)N(R^{14a})R^{14b}, -C(=O)OR¹⁵,
   phenyl, optionally substituted with one or more substituents R¹⁶; and
   a 3-, 4-, 5-, 6- or 7-membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶; and R⁹ in the groups -S(O)ₙR⁹ and -OSO₂R⁹ is additionally selected from the group consisting of C₁-C₆-alkoxy and C₁-C₆-haloalkoxy;
R^{10a}, R^{10b} are selected independently from one another from the group consisting of hydrogen, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, where the aliphatic and cycloaliphatic moieties in the eight last-mentioned radicals may be substituted by one or more radicals R¹³;
   -C₁-C₆-alkyl-C(=O)OR¹⁵, -C₁-C₆-alkyl-C(=O)N(R^{14a})R^{14b}, -C₁-C₆-alkyl-C(=S)N(R^{14a})R^{14b}, -C₁-C₆-alkyl-C(=NR¹⁴)N(R^{14a})R^{14b}, C₁-C₆-alkoxy, C₁-C₆-haloalkoxy, C₁-C₆-alkylthio, C₁-C₆-haloalkylthio,
   -S(O)ₙR¹⁵, -S(O)ₙN(R^{14a})R^{14b}, -C(=O)R¹³, -C(=O)OR¹⁵, -C(=O)N(R^{14a})R^{14b}, -C(=S)R¹³, -C(=S)SR¹⁵, -C(=S)N(R^{14a})R^{14b}, -C(=NR¹⁴)R¹³;
   phenyl, optionally substituted with 1, 2, 3 or 4 substituents R¹⁶; and a 3-, 4-, 5-, 6- or 7-membered saturated, partially unsaturated or maximally unsaturated heterocyclic ring comprising 1, 2, 3 or 4 heteroatoms or heteroatom groups selected from N, O, S, NO, SO and SO₂, as ring members, where the heterocyclic ring is optionally substituted with one or more substituents R¹⁶;
   or
   R^{10a} and R^{10b} form together with the nitrogen atom they are bonded to a 3-, 4-, 5-, 6-, 7- or 8-membered saturated, partially unsaturated or maximally unsaturated heterocyclic ring, wherein the heterocyclic ring may additionally contain one or two heteroatoms or heteroatom groups selected from N, O, S, NO, SO and SO₂, as ring members, where the heterocyclic ring optionally carries one or more substituents selected from halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl, optionally substituted with 1, 2, 3, 4 or 5 substituents R¹⁶, and a 3-, 4-, 5-, 6,- or 7-membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring optionally carries one or more substituents R¹⁶;
   or R^{10a} and R^{10b} together form a group =C(R¹³)₂, =S(O)ₘ(R¹⁵)₂, =S(O)ₘR¹⁵N(R^{14a})R^{14b}, =NR¹⁴ or =NOR¹⁵;
R¹¹ is independently selected from the group consisting of halogen, cyano, azido, nitro, -SCN, -SF₅, C₁-C₁₀-alkyl, C₃-C₈-cycloalkyl, C₂-C₁₀-alkenyl, C₂-C₁₀-alkynyl, wherein the four last-mentioned aliphatic and cycloaliphatic radicals may be partially or fully halogenated and/or may be substituted with one or more radicals R8; -OR⁹, -NR^{10a}R^{10b}, -S(O)ₙR⁹, -Si(R¹²)₃;
   phenyl, optionally substituted with 1, 2, 3, 4, or 5 substituents selected independently from R¹⁶; and
   a 3-, 4-, 5-, 6- or 7-membered saturated, partially unsaturated or maximally unsaturated aromatic heterocyclic ring comprising 1, 2, 3 or 4 heteroatoms or heteroatom groups selected from N, O, S, NO, SO and SO₂, as ring members, where the heterocyclic ring is optionally substituted with one or more substituents selected independently from R¹⁶;
   or
   two R¹¹ present on the same ring carbon atom of an unsaturated or partially unsaturated heterocyclic ring may together form a group =O, =C(R¹³)₂; =S; =S(O)ₘ(R¹⁵)₂; -S(O)ₘR¹⁵N(R^{14a})R^{14b}, =NR¹⁴, =NOR¹⁵, or =NN(R^{14a})R^{14b};
   or
   two R¹¹ bound on adjacent ring atoms form together with the ring atoms to which they are bound a saturated 3-, 4-, 5-, 6-, 7-, 8- or 9-membered ring, wherein the ring may contain 1 or 2 heteroatoms or heteroatom groups selected from O, S, N, NR¹⁴, NO, SO and SO₂ and/or 1 or 2 groups selected from C=O, C=S and C=NR¹⁴ as ring members, and wherein the ring may be substituted by one or more radicals selected from the group consisting of halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted by 1, 2, 3, 4 or 5 radicals R¹⁶, and a 3-, 4-, 5-, 6- or 7-membered saturated, partially unsaturated or maximally unsaturated heterocyclic ring containing 1, 2 or 3 heteroatoms or heteroatom groups selected from N, O, S, NO, SO and SO₂, as ring members, where the heterocyclic ring may be substituted by one or more radicals R¹⁶;
each R¹² is independently selected from the group consisting of hydrogen, halogen, C₁-C₆-alkyl, C₁-C₆-haloalkyl, C₁-C₆-alkoxy, C₁-C₆-haloalkoxy, C₂-C₆-alkenyl, C₂-C₆-haloalkenyl, C₂-C₆-alkynyl, C₂-C₆-haloalkynyl, C₃-C₈-cycloalkyl, C₃-C₈-halocycloalkyl, C₁-C₆-alkoxy-C₁-C₆-alkyl, C₁-C₆-haloalkoxy-C₁-C₆-alkyl, and phenyl, optionally substituted with 1, 2, 3, 4, or 5 substituents R¹⁶;
each R¹³ is independently selected from the group consisting of cyano, nitro, -OH, -SH, -SCN, -SF₅, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, trimethylsilyl, triethylsilyl, *tert*-butyldimethylsilyl, C₃-C₈-cycloalkyl which may be unsubstituted, partially or fully halogenated and/or may carry 1 or 2 radicals selected from C₁-C₄-alkyl, C₃-C₄-cycloalkyl, C₁-C₄-alkoxy, C₁-C₄-haloalkoxy and oxo; phenyl, benzyl, phenoxy, where the phenyl moiety in the three last-mentioned radicals may be unsubstituted or carry 1, 2, 3, 4 or 5 substituents R¹⁶; and a 3-, 4-, 5-, 6- or 7-membered saturated, partially unsaturated or maximally unsaturated heterocyclic ring containing 1, 2 or 3 heteroatoms or heteroatom groups selected from N, O, S, NO, SO and SO₂, as ring members, where the heterocyclic ring may be substituted by 1, 2 or 3 substituents R¹⁶;
   or
   two R¹³ present on the same carbon atom of an alkyl, alkenyl, alkynyl or cycloalkyl group may together be =O, =CH(C₁-C₄-alkyl), =C(C₁-C₄-alkyl)C₁-C₄-alkyl, =N(C₁-C₆-alkyl) or =NO(C₁-C₆-alkyl);
   and
   R¹³ as a substituent on a cycloalkyl ring is additionally selected from the group consisting of C₁-C₆-alkyl, C₂-C₆-alkenyl and C₂-C₆-alkynyl, wherein the three last-mentioned aliphatic radicals may be unsubstituted, partially or fully halogenated and/or may carry 1 or 2 substituents selected from CN, C₃-C₄-cycloalkyl, C₁-C₄-alkoxy, C₁-C₄-haloalkoxy and oxo;
   and
   R¹³ in the groups =C(R¹³)₂, -N=C(R¹³)₂, -C(=O)R¹³, -C(=S)R¹³ and -C(=NR¹⁴)R¹³ is additionally selected from the group consisting of hydrogen, halogen, C₁-C₆-alkyl, C₂-C₆-alkenyl and C₂-C₆-alkynyl, wherein the three last-mentioned aliphatic radicals may be unsubstituted, partially or fully halogenated and/or may carry 1 or 2 radicals selected from CN, C₃-C₄-cycloalkyl, C₁-C₄-alkoxy, C₁-C₄-haloalkoxy and oxo;
each R¹⁴ is independently selected from the group consisting of hydrogen, cyano, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, trimethylsilyl, triethylsilyl, *tert*-butyldimethylsilyl,
   C₁-C₆-alkyl, C₂-C₆-alkenyl, C₂-C₆-alkynyl, wherein the three last-mentioned aliphatic radicals may be unsubstituted, partially or fully halogenated and/or may carry 1 or 2 radicals selected from CN, C₁-C₄-alkoxy, C₁-C₄-haloalkoxy, C₁-C₄-alkylthio, C₁-C₄-alkylsulfinyl, C₁-C₄-alkylsulfonyl, C₃-C₄-cycloalkyl which may be substituted by 1 or 2 substituents selected from halogen and cyano; and oxo; C₃-C₈-cycloalkyl which may be unsubstituted, partially or fully halogenated and/or may carry 1 or 2 radicals selected from C₁-C₄-alkyl, C₁-C₄-alkoxy, C₁-C₄-haloalkoxy, C₁-C₄-alkylthio, C₁-C₄-alkylsulfinyl, C₁-C₄-alkylsulfonyl, C₃-C₄-cycloalkyl, C₃-C₄-cycloalkyl-C₁-C₄-alkyl-, where the cycloalkyl moiety in the two last-mentioned radicals may be substituted by 1 or 2 substituents selected from halogen and cyano; and oxo;
   phenyl, benzyl, pyridyl, phenoxy, wherein the cyclic moieties in the four last-mentioned radicals may be unsubstituted or carry 1, 2 or 3 substituents selected from halogen, C₁-C₆-alkyl, C₁-C₆-haloalkyl, C₁-C₆-alkoxy, C₁-C₆-haloalkoxy and (C₁-C₆-alkoxy)carbonyl; and a 3-, 4-, 5- or 6-membered saturated, partially unsaturated or maximally unsaturated heterocyclic ring comprising 1 or 2 heteroatoms or heteroatom groups selected from N, O, S, NO, SO and SO₂, as ring members, where the heterocyclic ring is optionally substituted with one or more substituents R¹⁶;
R^{14a} and R^{14b}, independently of each other, have one of the meanings given for R¹⁴; or
   R^{14a} and R^{14b}, together with the nitrogen atom to which they are bound, form a 3-, 4-, 5-, 6- or 7-membered saturated, partially unsaturated or maximally unsaturated heterocyclic ring, wherein the heterocyclic ring may additionally contain 1 or 2 heteroatoms or heteroatom groups selected from N, O, S, NO, SO and SO₂, as ring members, where the heterocyclic ring optionally carries one or more substituents selected from halogen, C₁-C₄-alkyl, C₁-C₄-haloalkyl, C₁-C₄-alkoxy and C₁-C₄-haloalkoxy;
   or
   R^{14a} and R¹⁴ or R^{14b} and R¹⁴, together with the nitrogen atoms to which they are bound in the group -C(=NR¹⁴)N(R^{14a})R^{14b}, form a 3-, 4-, 5-, 6- or 7-membered partially unsaturated or maximally unsaturated heterocyclic ring, wherein the heterocyclic ring may additionally contain 1 or 2 heteroatoms or heteroatom groups selected from N, O, S, NO, SO and SO₂, as ring members, where the heterocyclic ring optionally carries one or more substituents selected from halogen, C₁-C₄-haloalkyl, C₁-C₄-alkoxy and C₁-C₄-haloalkoxy;
each R¹⁵ is independently selected from the group consisting of hydrogen, cyano, trimethylsilyl, triethylsilyl, *tert*-butyldimethylsilyl,
   C₁-C₆-alkyl, C₂-C₆-alkenyl, C₂-C₆-alkynyl, wherein the three last-mentioned aliphatic radicals may be unsubstituted, partially or fully halogenated and/or may carry 1 or 2 radicals selected from C₃-C₄-cycloalkyl, C₁-C₄-alkoxy, C₁-C₄-haloalkoxy, C₁-C₄-alkylthio, C₁-C₄-alkylsulfinyl, C₁-C₄-alkylsulfonyl and oxo; C₃-C₈-cycloalkyl which may be unsubstituted, partially or fully halogenated and/or may carry 1 or 2 radicals selected from C₁-C₄-alkyl, C₃-C₄-cycloalkyl, C₁-C₄-alkoxy, C₁-C₄-haloalkoxy, C₁-C₄-alkylthio, C₁-C₄-alkylsulfinyl, C₁-C₄-alkylsulfonyl and oxo;
   phenyl, benzyl, pyridyl and phenoxy, wherein the four last-mentioned radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆-haloalkoxy and (C₁-C₆-alkoxy)carbonyl;
each R¹⁶ is independently selected from the group consisting of halogen, nitro, cyano, -OH, -SH, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, trimethylsilyl, triethylsilyl, *tert*-butyldimethylsilyl; C₁-C₆-alkyl, C₂-C₆-alkenyl, C₂-C₆-alkynyl, wherein the three last-mentioned aliphatic radicals may be unsubstituted, partially or fully halogenated and/or may carry 1 or 2 radicals selected from C₃-C₄-cycloalkyl, C₁-C₄-alkoxy, C₁-C₄-haloalkoxy and oxo;
   C₃-C₈-cycloalkyl which may be unsubstituted, partially or fully halogenated and/or may carry 1 or 2 radicals selected from C₁-C₄-alkyl, C₃-C₄-cycloalkyl, C₁-C₄-alkoxy, C₁-C₄-haloalkoxy and oxo;
   phenyl, benzyl, pyridyl and phenoxy, wherein the four last mentioned radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆-haloalkoxy and (C₁-C₆-alkoxy)carbonyl;
   or
   two R¹⁶ present together on the same atom of an unsaturated or partially unsaturated ring may be =O, =S, =N(C₁-C₆-alkyl), =NO(C₁-C₆-alkyl), =CH(C₁-C₄-alkyl) or =C(C₁-C₄-alkyl)C₁-C₄-alkyl;
   or
   two R¹⁶ on two adjacent carbon atoms form together with the carbon atoms they are bonded to a 4-, 5-, 6-, 7- or 8-membered saturated, partially unsaturated or maximally unsaturated ring, wherein the ring may contain 1 or 2 heteroatoms or heteroatom groups selected from N, O, S, NO, SO and SO₂, as ring members, and wherein the ring optionally carries one or more substituents selected from halogen, C₁-C₄-alkyl, C₁-C₄-haloalkyl, C₁-C₄-alkoxy and C₁-C₄-haloalkoxy;
each n is independently 0, 1 or 2; and
each m is independently 0 or 1;
and the N-oxides, stereoisomers and agriculturally or veterinarily acceptable salts thereof;
except for compounds I wherein B¹ and B³ are C-CI, and simultaneously B² is C-H, G¹, G³ and G⁴ are C-H, G² is C-F, C-Cl, C-CH₃ or C-SCH₃, W is O, R¹ is CF₃, R^{3a} and R^{3b} are H, and R⁵ and R⁶, together with the nitrogen atom they are bound to, form a ring selected from aziridin-1-yl, azetidin-1-yl, pyrrolidin-1-yl, piperidin-1-yl, thiazolidin-3-yl, morpholin-4-yl, thiomorpholin-4-yl, 1-oxo-1,4-thiazinan-4-yl and 1,1-dioxo-1,4-thiazinan-4-yl.

The present description also provides an agricultural composition comprising at least one compound of the formula I as defined herein and/or an agriculturally acceptable salt thereof and at least one liquid and/or solid agriculturally acceptable carrier.

The present description also provides a veterinary composition comprising at least one compound of the formula I as defined herein and/or a veterinarily acceptable salt thereof and at least one liquid and/or solid veterinarily acceptable carrier.

The present description also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein.

The present description also relates to plant propagation material, in particular seed, comprising at least one compound of formula I and/or an agriculturally acceptable salt thereof as defined herein.

The present description further relates to a method for treating or protecting an animal from infestation or infection by parasites which comprises bringing the animal in contact with a parasiticidally effective amount of a compound of the formula I or a veterinarily acceptable salt thereof as defined herein. Bringing the animal in contact with the compound I, its salt or the veterinary composition of the invention means applying or administering it to the animal.

The term "steroisomers" encompasses both optical isomers, such as enantiomers or diastereomers, the latter existing due to more than one center of chirality in the molecule, as well as geometrical isomers (cis/trans isomers).

Depending on the substitution pattern, the compounds of the formula I may have one or more centers of chirality, in which case they are present as mixtures of enantiomers or diastereomers. One center of chirality is the carbon ring atom of the isothiazoline ring carrying radical R¹. The invention provides both the pure enantiomers or diastereomers and their mixtures and the use according to the invention of the pure enantiomers or diastereomers of the compound I or its mixtures. Suitable compounds of the formula I also include all possible geometrical stereoisomers (cis/trans isomers) and mixtures thereof.

The term N-oxides relates to a form of compounds I in which at least one nitrogen atom is present in oxidized form (as NO). To be more precise, it relates to any compound of the present invention which has at least one tertiary nitrogen atom that is oxidized to an N-oxide moiety. N-oxides of compounds I can in particular be prepared by oxidizing e.g. the ring nitrogen atom of the isothiazoline moiety and/or, if G¹, G², G³ or G⁴ is N, this nitrogen atom, and/or of the ring formed by R⁵ and R⁶ with a suitable oxidizing agent, such as peroxo carboxylic acids or other peroxides. The person skilled in the art knows if and in which positions compounds of the present invention may form N-oxides.

The compounds of the present invention may be amorphous or may exist in one ore more different crystalline states (polymorphs) which may have a different macroscopic properties such as stability or show different biological properties such as activities. The present invention includes both amorphous and crystalline compounds of the formula I, mixtures of different crystalline states of the respective compound I, as well as amorphous or crystalline salts thereof.

Salts of the compounds of the formula I are preferably agriculturally and veterinarily acceptable salts. They can be formed in a customary method, e.g. by reacting the compound with an acid of the anion in question if the compound of formula I has a basic functionality or by reacting an acidic compound of formula I with a suitable base.

Suitable agriculturally acceptable salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH⁴⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting a compound of formulae I with an acid of the corresponding anion, preferably of hydrochloric acid, hydrobromic acid, sulfuric acid, phosphoric acid or nitric acid.

By the term "veterinarily acceptable salts" is meant salts of those cations or anions which are known and accepted in the art for the formation of salts for veterinary use. Suitable acid addition salts, e.g. formed by compounds of formula I containing a basic nitrogen atom, e.g. an amino group, include salts with inorganic acids, for example hydrochlorids, sulphates, phosphates, and nitrates and salts of organic acids for example acetic acid, maleic acid, dimaleic acid, fumaric acid, difumaric acid, methane sulfenic acid, methane sulfonic acid, and succinic acid.

The term "invertebrate pest" as used herein encompasses animal populations, such as insects, arachnids and nematodes, which may attack plants, thereby causing substantial damage to the plants attacked, as well as ectoparasites which may infest animals, in particular warm blooded animals such as e.g. mammals or birds, or other higher animals such as reptiles, amphibians or fish, thereby causing substantial damage to the animals infested.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. The plant propagation materials may be treated prophylactically with a plant protection compound either at or before planting or transplanting. Said young plants may also be protected before transplantation by a total or partial treatment by immersion or pouring.

The term "plants" comprises any types of plants including "non-cultivated plants" and in particular "cultivated plants".

The term "non-cultivated plants" refers to any wild type species or related species or related genera of a cultivated plant.

The term "cultivated plants" is to be understood as including plants which have been modified by breeding, mutagenesis or genetic engineering including but not limiting to agricultural biotech products on the market or in development (cf. http://www.bio.org/speeches/pubs/er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxyl-phenylpyruvate dioxygenase (HPPD) inhibitors or phytoene desaturase (PDS) inhibit-tors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady® (glyphosate-tolerant, Monsanto, U.S.A.), Cultivance® (imidazolinone tolerant, BASF SE, Germany) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ -endotoxins, e. g. CryIA(b), CryIA(c), CrylF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called " pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora® potato, BASF SE, Germany).

The organic moieties mentioned in the above definitions of the variables are - like the term halogen - collective terms for individual listings of the individual group members. The prefix Cₙ-Cₘ indicates in each case the possible number of carbon atoms in the group.

The term halogen denotes in each case fluorine, bromine, chlorine or iodine, in particular fluorine, chlorine or bromine.

The term "alkyl" as used herein and in the alkyl moieties of alkoxy, alkylthio, alkylsulfinyl, alkylsulfonyl, alkylcarbonyl, alkoxycarbonyl and the like refers to saturated straight-chain or branched hydrocarbon radicals having 1 to 2 ("C₁-C₂-alkyl"), 1 to 3 ("C₁-C₃-alkyl"), 1 to 4 ("C₁-C₄-alkyl"), 1 to 6 ("C₁-C₆-alkyl"), 1 to 8 ("C₁-C₈-alkyl") or 1 to 10 ("C₁-C₁₀-alkyl") carbon atoms. C₁-C₂-Alkyl is methyl or ethyl. C₁-C₃-Alkyl is additionally propyl and isopropyl. C₁-C₄-Alkyl is additionally butyl, 1-methylpropyl (sec-butyl), 2-methylpropyl (isobutyl) or 1,1-dimethylethyl (tert-butyl). C₁-C₆-Alkyl is additionally also, for example, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl. C₁-C₈-Alkyl is additionally also, for example, heptyl, octyl, 2-ethylhexyl and positional isomers thereof. C₁-C₁₀-Alkyl is additionally also, for example, nonyl, decyl and positional isomers thereof.

The term "haloalkyl" as used herein, which is also expressed as "alkyl which is partially or fully halogenated", refers to straight-chain or branched alkyl groups having 1 to 2 ("C₁-C₂-haloalkyl"), 1 to 3 ("C₁-C₃-haloalkyl"), 1 to 4 ("C₁-C₄-haloalkyl"), 1 to 6 ("C₁-C₆-haloalkyl"), 1 to 8 ("C₁-C₈-haloalkyl") or 1 to 10 ("C₁-C₁₀-haloalkyl") carbon atoms (as mentioned above), where some or all of the hydrogen atoms in these groups are replaced by halogen atoms as mentioned above: in particular C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. C₁-C₃-haloalkyl is additionally, for example, 1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 3-chloropropyl and the like. Examples for C₁-C₄-haloalkyl are, apart those mentioned for C₁-C₃-haloalkyl, 4-chlorobutyl and the like.

"Halomethyl" is methyl in which 1, 2 or 3 of the hydrogen atoms are replaced by halogen atoms. Examples are bromomethyl, chloromethyl, fluoromethyl, dichloromethyl, trichloromethyl, difluoromethyl, trifluoromethyl, chlorofluoromethyl, dichlorofluoromethyl, chlorodifluoromethyl and the like.

The term "alkenyl" as used herein refers to monounsaturated straight-chain or branched hydrocarbon radicals having 2 to 3 ("C₂-C₃-alkenyl"), 2 to 4 ("C₂-C₄-alkenyl"), 2 to 6 ("C₂-C₆-alkenyl"), 2 to 8 ("C₂-C₈-alkenyl") or 2 to 10 ("C₂-C₁₀-alkenyl") carbon atoms and a double bond in any position, for example C₂-C₃-alkenyl, such as ethenyl, 1-propenyl, 2-propenyl or 1-methylethenyl; C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like, or C₂-C₁₀-alkenyl, such as the radicals mentioned for C₂-C₆-alkenyl and additionally 1-heptenyl, 2-heptenyl, 3-heptenyl, 1-octenyl, 2-octenyl, 3-octenyl, 4-octenyl, 1-nonenyl, 2-nonenyl, 3-nonenyl, 4-nonenyl, 1-decenyl, 2-decenyl, 3-decenyl, 4-decenyl, 5-decenyl and the positional isomers thereof.

The term "haloalkenyl" as used herein, which is also expressed as "alkenyl which is partially or fully halogenated", refers to unsaturated straight-chain or branched hydrocarbon radicals having 2 to 3 ("C₂-C₃-haloalkenyl"), 2 to 4 ("C₂-C₄-haloalkenyl"), 2 to 6 ("C₂-C₆-haloalkenyl"), 2 to 8 ("C₂-C₆-haloalkenyl") or 2 to 10 ("C₂-C₁₀-haloalkenyl") carbon atoms and a double bond in any position (as mentioned above), where some or all of the hydrogen atoms in these groups are replaced by halogen atoms as mentioned above, in particular fluorine, chlorine and bromine, for example chlorovinyl, chloroallyl and the like.

The term "alkynyl" as used herein refers to straight-chain or branched hydrocarbon groups having 2 to 3 ("C₂-C₃-alkynyl"), 2 to 4 ("C₂-C₄-alkynyl"), 2 to 6 ("C₂-C₆-alkynyl"), 2 to 8 ("C₂-C₈-alkynyl"), or 2 to 10 ("C₂-C₁₀-alkynyl") carbon atoms and one or two triple bonds in any position, for example C₂-C₃-alkynyl, such as ethynyl, 1-propynyl or 2-propynyl; C₂-C₄-alkynyl, such as ethynyl, 1-propynyl, 2-propynyl, 1-butynyl, 2-butynyl, 3-butynyl, 1-methyl-2-propynyl and the like, C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl and the like;

The term "haloalkynyl" as used herein, which is also expressed as "alkynyl which is partially or fully halogenated", refers to unsaturated straight-chain or branched hydrocarbon radicals having 2 to 3 ("C₂-C₃-haloalkynyl"), 2 to 4 ("C₂-C₄-haloalkynyl"), 3 to 4 ("C₃-C₄-haloalkynyl"), 2 to 6 ("C₂-C₆-haloalkynyl"), 2 to 8 ("C₂-C₈-haloalkynyl") or 2 to 10 ("C₂-C₁₀-haloalkynyl") carbon atoms and one or two triple bonds in any position (as mentioned above), where some or all of the hydrogen atoms in these groups are replaced by halogen atoms as mentioned above, in particular fluorine, chlorine and bromine;

The term "cycloalkyl" as used herein refers to mono- or bi- or polycyclic saturated hydrocarbon radicals having 3 to 8 ("C₃-C₈-cycloalkyl"), in particular 3 to 6 ("C₃-C₆-cycloalkyl") or 3 to 5 ("C₃-C₅-cycloalkyl") or 3 to 4 ("C₃-C₄-cycloalkyl") carbon atoms.

Examples of monocyclic radicals having 3 to 4 carbon atoms comprise cyclopropyl and cyclobutyl. Examples of monocyclic radicals having 3 to 5 carbon atoms comprise cyclopropyl, cyclobutyl and cyclopentyl. Examples of monocyclic radicals having 3 to 6 carbon atoms comprise cyclopropyl, cyclobutyl, cyclopentyl and cyclohexyl. Examples of monocyclic radicals having 3 to 8 carbon atoms comprise cyclopropyl, cyclobutyl, cyclopentyl, cyclohexyl, cycloheptyl and cyclooctyl. Examples of bicyclic radicals having 7 or 8 carbon atoms comprise bicyclo[2.2.1]heptyl, bicyclo[3.1.1]heptyl, bicyclo[2.2.2]octyl and bicyclo[3.2.1]octyl. Preferably, the term cycloalkyl denotes a monocyclic saturated hydrocarbon radical.

The term "halocycloalkyl" as used herein, which is also expressed as "cycloalkyl which is partially or fully halogenated", refers to mono- or bi- or polycyclic saturated hydrocarbon groups having 3 to 8 ("C₃-C₈-halocycloalkyl") or preferably 3 to 6 ("C₃-C₆-halocycloalkyl") or 3 to 5 ("C₃-C₅-halocycloalkyl") or 3 to 4 ("C₃-C₄-halocycloalkyl") carbon ring members (as mentioned above) in which some or all of the hydrogen atoms are replaced by halogen atoms as mentioned above, in particular fluorine, chlorine and bromine.

The term "cycloalkyl-C₁-C₄-alkyl" refers to a C₃-C₈-cycloalkyl group ("C₃-C₈-cycloalkyl-C₁-C₄-alkyl"), preferably a C₃-C₆-cycloalkyl group ("C₃-C₆-cycloalkyl-C₁-C₄-alkyl"), more preferably a C₃-C₄-cycloalkyl group ("C₃-C₄-cycloalkyl-C₁-C₄-alkyl") as defined above (preferably a monocyclic cycloalkyl group) which is bound to the remainder of the molecule via a C₁-C₄-alkyl group, as defined above. Examples for C₃-C₄-cycloalkyl-C₁-C₄-alkyl- are cyclopropylmethyl, cyclopropylethyl, cyclopropylpropyl, cyclobutylmethyl, cyclobutylethyl and cyclobutylpropyl, Examples for C₃-C₆-cycloalkyl-C₁-C₄-alkyl, apart those mentioned for C₃-C₄-cycloalkyl-C₁-C₄-alkyl, are cyclopentylmethyl, cyclopentylethyl, cyclopentylpropyl, cyclohexylmethyl, cyclohexylethyl and cyclohexylpropyl. Examples for C₃-C₈-cycloalkyl-C₁-C₄-alkyl, apart those mentioned for C₃-C₆-cycloalkyl-C₁-C₄-alkyl, are cycloheptylmethyl, cycloheptylethyl, cyclooctylmethyl and the like.

The term "C₃-C₈-halocycloalkyl-C₁-C₄-alkyl" refers to a C₃-C₈-halocycloalkyl group as defined above which is bound to the remainder of the molecule via a C₁-C₄-alkyl group, as defined above.

The term "cycloalkenyl" as used herein refers to monocyclic hydrocarbon radicals with at least one C-C double bond in the ring, which ring is however not aromatic, the hydrocarbon radicals having 3 to 8 ("C₃-C₈-cycloalkyl) carbon atoms. Examples are cyclopropenyl, such as cycloprop-1-enyl and cycloprop-2-yl, cyclobutenyl, such as cyclobut-1-enyl and cyclobut-2-enyl, cyclopentenyl, such as cyclopent-1-enyl, cyclopent-2-enyl and cyclopent-3-enyl, cyclopentadienyl, such as cyclopenta-1,3-dienyl, cyclpenta-1,4-dienyl and cyclpenta-2,4-dienyl, cyclohexenyl, such as cyclohex-1-enyl, cyclohex-2-enyl and cyclohex-3-enyl, cyclohexadienyl, such as cyclohexa-1,3-dienyl, cyclohexa-1,4-dienyl, cyclohexa-1,5-dienyl and cyclohexa-2,5-dienyl, cycloheptenyl, cycloheptadienyl, cycloheptatrienyl cyclooctenyl, cyclooctadieny, cyclooctatrienyl and cyclooctatetraenyl.

The term "halocycloalkenyl" as used herein refers to monocyclic hydrocarbon radicals with at least one C-C double bond in the ring, which ring is however not aromatic, the hydrocarbon radicals having 3 to 8 ("C₃-C₈-halocycloalkenyl") carbon atoms, and wherein some or all of the hydrogen atoms are replaced by halogen atoms as mentioned above, in particular fluorine, chlorine and bromine.

The term "C₁-C₂-alkoxy" is a C₁-C₂-alkyl group, as defined above, attached via an oxygen atom. The term "C₁-C₃-alkoxy" is a C₁-C₃-alkyl group, as defined above, attached via an oxygen atom. The term "C₁-C₄-alkoxy" is a C₁-C₄-alkyl group, as defined above, attached via an oxygen atom. The term "C₁-C₆-alkoxy" is a C₁-C₆-alkyl group, as defined above, attached via an oxygen atom. The term "C₁-C₁₀-alkoxy" is a C₁-C₁₀-alkyl group, as defined above, attached via an oxygen atom. C₁-C₂-Alkoxy is methoxy or ethoxy. C₁-C₃-Alkoxy is additionally, for example, n-propoxy and 1-methylethoxy (isopropoxy). C₁-C₄-Alkoxy is additionally, for example, butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy. C₁-C₈-Alkoxy is additionally, for example, heptyloxy, octyloxy, 2-ethylhexyloxy and positional isomers thereof. C₁-C₁₀-Alkoxy is additionally, for example, nonyloxy, decyloxy and positional isomers thereof.

The term "C₁-C₂-haloalkoxy" is a C₁-C₂-haloalkyl group, as defined above, attached via an oxygen atom. The term "C₁-C₃-haloalkoxy" is a C₁-C₃-haloalkyl group, as defined above, attached via an oxygen atom. The term "C₁-C₄-haloalkoxy" is a C₁-C₄-haloalkyl group, as defined above, attached via an oxygen atom. The term "C₁-C₆-haloalkoxy" is a C₁-C₆-haloalkyl group, as defined above, attached via an oxygen atom. The term "C₁-C₁₀-haloalkoxy" is a C₁-C₁₀-haloalkyl group, as defined above, attached via an oxygen atom. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₄-Haloalkoxy is additionally, for example, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "C₁-C₃-alkoxy-C₁-C₃-alkyl" as used herein, refers to a straight-chain or branched alkyl group having 1 to 3 carbon atoms, as defined above, where one hydrogen atom is replaced by a C₁-C₃-alkoxy group, as defined above. The term "C₁-C₄-alkoxy-C₁-C₄-alkyl" as used herein, refers to a straight-chain or branched alkyl group having 1 to 4 carbon atoms, as defined above, where one hydrogen atom is replaced by a C₁-C₄-alkoxy group, as defined above. The term "C₁-C₆-alkoxy-C₁-C₆-alkyl" as used herein, refers to a straight-chain or branched alkyl group having 1 to 6 carbon atoms, as defined above, where one hydrogen atom is replaced by a C₁-C₆-alkoxy group, as defined above. Examples are methoxymethyl, ethoxymethyl, propoxymethyl, isopropoxymethyl, n-butoxymethyl, sec-butoxymethyl, isobutoxymethyl, tert-butoxymethyl, 1-methoxyethyl, 1-ethoxyethyl, 1-propoxyethyl, 1-isopropoxyethyl, 1-n-butoxyethyl, 1-sec-butoxyethyl, 1-isobutoxyethyl, 1-tert-butoxyethyl, 2-methoxyethyl, 2-ethoxyethyl, 2-propoxyethyl, 2-isopropoxyethyl, 2-n-butoxyethyl, 2-sec-butoxyethyl, 2-isobutoxyethyl, 2-tert-butoxyethyl, 1-methoxypropyl, 1-ethoxypropyl, 1-propoxypropyl, 1-isopropoxypropyl, 1-n-butoxypropyl, 1-sec-butoxypropyl, 1-isobutoxypropyl, 1-tert-butoxypropyl, 2-methoxypropyl, 2-ethoxypropyl, 2-propoxypropyl, 2-isopropoxypropyl, 2-n-butoxypropyl, 2-sec-butoxypropyl, 2-isobutoxypropyl, 2-tert-butoxypropyl, 3-methoxypropyl, 3-ethoxypropyl, 3-propoxypropyl, 3-isopropoxypropyl, 3-n-butoxypropyl, 3-sec-butoxypropyl, 3-isobutoxypropyl, 3-tert-butoxypropyl and the like.

The term "C₁-C₄-alkoxy-methyl" as used herein, refers to methyl in which one hydrogen atom is replaced by a C₁-C₄-alkoxy group, as defined above. The term "C₁-C₆-alkoxymethyl" as used herein, refers to methyl in which one hydrogen atom is replaced by a C₁-C₆-alkoxy group, as defined above. Examples are methoxymethyl, ethoxymethyl, propoxymethyl, isopropoxymethyl, n-butoxymethyl, sec-butoxymethyl, isobutoxymethyl, tert-butoxymethyl, pentyloxymethyl, hexyloxymethyl and the like.

C₁-C₆-Haloalkoxy-C₁-C₆-alkyl is a straight-chain or branched alkyl group having from 1 to 6, especially 1 to 4 carbon atoms (=C₁-C₆-haloalkoxy-C₁-C₄-alkyl), wherein one of the hydrogen atoms is replaced by a C₁-C₆-alkoxy group and wherein at least one, e.g. 1, 2, 3, 4 or all of the remaining hydrogen atoms (either in the alkoxy moiety or in the alkyl moiety or in both) are replaced by halogen atoms. C₁-C₄-Haloalkoxy-C₁-C₄-alkyl is a straight-chain or branched alkyl group having from 1 to 4 carbon atoms, wherein one of the hydrogen atoms is replaced by a C₁-C₄-alkoxy group and wherein at least one, e.g. 1, 2, 3, 4 or all of the remaining hydrogen atoms (either in the alkoxy moiety or in the alkyl moiety or in both) are replaced by halogen atoms. Examples are difluoromethoxymethyl (CHF₂OCH₂), trifluoromethoxymethyl, 1-difluoromethoxyethyl, 1-trifluoromethoxyethyl, 2-difluoromethoxyethyl, 2-trifluoromethoxyethyl, difluoro-methoxy-methyl (CH₃OCF₂), 1,1-difluoro-2-methoxyethyl, 2,2-difluoro-2-methoxyethyl and the like.

The term "C₁-C₂-alkylthio" is a C₁-C₂-alkyl group, as defined above, attached via a sulfur atom. The term "C₁-C₃-alkylthio" is a C₁-C₃-alkyl group, as defined above, attached via a sulfur atom. The term "C₁-C₄-alkylthio" is a C₁-C₄-alkyl group, as defined above, attached via a sulfur atom. The term "C₁-C₆-alkylthio" is a C₁-C₆-alkyl group, as defined above, attached via a sulfur atom. The term "C₁-C₁₀-alkylthio" is a C₁-C₁₀-alkyl group, as defined above, attached via a sulfur atom. C₁-C₂-Alkylthio is methylthio or ethylthio. C₁-C₃-Alkylthio is additionally, for example, n-propylthio or 1-methylethylthio (isopropylthio). C₁-C₄-Alkylthio is additionally, for example, butylthio, 1-methylpropylthio (sec-butylthio), 2-methylpropylthio (isobutylthio) or 1,1-dimethylethylthio (tert-butylthio). C₁-C₆-Alkylthio is additionally, for example, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio. C₁-C₈-Alkylthio is additionally, for example, heptylthio, octylthio, 2-ethylhexylthio and positional isomers thereof. C₁-C₁₀-Alkylthio is additionally, for example, nonylthio, decylthio and positional isomers thereof.

The term "C₁-C₂-haloalkylthio" is a C₁-C₂-haloalkyl group, as defined above, attached via a sulfur atom. The term "C₁-C₃-haloalkylthio" is a C₁-C₃-haloalkyl group, as defined above, attached via a sulfur atom. The term "C₁-C₄-haloalkylthio" is a C₁-C₄-haloalkyl group, as defined above, attached via a sulfur atom. The term "C₁-C₆-haloalkylthio" is a C₁-C₆-haloalkyl group, as defined above, attached via a sulfur atom. The term "C₁-C₁₀-haloalkylthio" is a C₁-C₁₀-haloalkyl group, as defined above, attached via a sulfur atom. C₁-C₂-Haloalkylthio is, for example, SCH₂F, SCHF₂, SCF₃, SCH₂Cl, SCHCl₂, SCCl₃, chlorofluoromethylthio, dichlorofluoromethylthio, chlorodifluoromethylthio, 2-fluoroethylthio, 2-chloroethylthio, 2-bromoethylthio, 2-iodoethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio or SC₂F₅. C₁-C₃-Haloalkylthio is additionally, for example, 2-fluoropropylthio, 3-fluoropropylthio, 2,2-difluoropropylthio, 2,3-difluoropropylthio, 2-chloropropylthio, 3-chloropropylthio, 2,3-dichloropropylthio, 2-bromopropylthio, 3-bromopropylthio, 3,3,3-trifluoropropylthio, 3,3,3-trichloropropylthio, SCH₂-C₂F₅, SCF₂-C₂F₅, 1-(CH₂F)-2-fluoroethylthio, 1-(CH₂Cl)-2-chloroethylthio or 1-(CH₂Br)-2-bromoethylthio. C₁-C₄-Haloalkylthio is additionally, for example, 4-fluorobutylthio, 4-chlorobutylthio, 4-bromobutylthio or nonafluorobutylthio. C₁-C₆-Haloalkylthio is additionally, for example, 5-fluoropentylthio, 5-chloropentylthio, 5-brompentylthio, 5-iodopentylthio, undecafluoropentylthio, 6-fluorohexylthio, 6-chlorohexylthio, 6-bromohexylthio, 6-iodohexylthio or dodecafluorohexylthio.

The term "C₁-C₂-alkylsulfinyl" is a C₁-C₂-alkyl group, as defined above, attached via a sulfinyl [S(O)] group. The term "C₁-C₃-alkylsulfinyl" is a C₁-C₃-alkyl group, as defined above, attached via a sulfinyl [S(O)] group. The term "C₁-C₄-alkylsulfinyl" is a C₁-C₄-alkyl group, as defined above, attached via a sulfinyl [S(O)] group. The term "C₁-C₆-alkylsulfinyl" is a C₁-C₆-alkyl group, as defined above, attached via a sulfinyl [S(O)] group. The term "C₁-C₁₀-alkylsulfinyl" is a C₁-C₁₀-alkyl group, as defined above, attached via a sulfinyl [S(O)] group. C₁-C₂-Alkylsulfinyl is methylsulfinyl or ethylsulfinyl. C₁-C₃-Alkylsulfinyl is additionally, for example, n-propylsulfinyl and 1-methylethylsulfinyl (isopropylsulfinyl). C₁-C₄-Alkylsulfinyl is additionally, for example, butylsulfinyl, 1-methylpropylsulfinyl (sec-butylsulfinyl), 2-methylpropylsulfinyl (isobutylsulfinyl) or 1,1-dimethylethylsulfinyl (tert-butylsulfinyl). C₁-C₆-Alkylsulfinyl is additionally, for example, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl. C₁-C₈-Alkylsulfinyl is additionally, for example, heptylsulfinyl, octylsulfinyl, 2-ethylhexylsulfinyl and positional isomers thereof. C₁-C₁₀-Alkylsulfinyl is additionally, for example, nonylsulfinyl, decylsulfinyl and positional isomers thereof.

The term "C₁-C₂-haloalkylsulfinyl" is a C₁-C₂-haloalkyl group, as defined above, attached via a sulfinyl [S(O)] group. The term "C₁-C₃-haloalkylsulfinyl" is a C₁-C₃-haloalkyl group, as defined above, attached via a sulfinyl [S(O)] group. The term "C₁-C₄-haloalkylsulfinyl" is a C₁-C₄-haloalkyl group, as defined above, attached via a sulfinyl [S(O)] group. The term "C₁-C₆-haloalkylsulfinyl" is a C₁-C₆-haloalkyl group, as defined above, attached via a sulfinyl [S(O)] group. The term "C₁-C₁₀-haloalkylsulfinyl" is a C₁-C₁₀-haloalkyl group, as defined above, attached via a sulfinyl [S(O)] group. C₁-C₂-Haloalkylsulfinyl is, for example, S(O)CH₂F, S(O)CHF₂, S(O)CF₃, S(O)CH₂Cl, S(O)CHCl₂, S(O)CCl₃, chlorofluoromethylsulfinyl, dichlorofluoromethylsulfinyl, chlorodifluoromethylsulfinyl, 2-fluoroethylsulfinyl, 2-chloroethylsulfinyl, 2-bromoethylsulfinyl, 2-iodoethylsulfinyl, 2,2-difluoroethylsulfinyl, 2,2,2-trifluoroethylsulfinyl, 2-chloro-2-fluoroethylsulfinyl, 2-chloro-2,2-difluoroethylsulfinyl, 2,2-dichloro-2-fluoroethylsulfinyl, 2,2,2-trichloroethylsulfinyl or S(O)C₂F₅. C₁-C₃-Haloalkylsulfinyl is additionally, for example, 2-fluoropropylsulfinyl, 3-fluoropropylsulfinyl, 2,2-difluoropropylsulfinyl, 2,3-difluoropropylsulfinyl, 2-chloropropylsulfinyl, 3-chloropropylsulfinyl, 2,3-dichloropropylsulfinyl, 2-bromopropylsulfinyl, 3-bromopropylsulfinyl, 3,3,3-trifluoropropylsulfinyl, 3,3,3-trichloropropylsulfinyl, S(O)CH₂-C₂F₅, S(O)CF₂-C₂F₅, 1-(CH₂F)-2-fluoroethylsulfinyl, 1-(CH₂Cl)-2-chloroethylsulfinyl and 1-(CH₂Br)-2-bromoethylsulfinyl. C₁-C₄-Haloalkylsulfinyl is additionally, for example, 4-fluorobutylsulfinyl, 4-chlorobutylsulfinyl, 4-bromobutylsulfinyl or nonafluorobutylsulfinyl. C₁-C₆-Haloalkylsulfinyl is additionally, for example, 5-fluoropentylsulfinyl, 5-chloropentylsulfinyl, 5-brompentylsulfinyl, 5-iodopentylsulfinyl, undecafluoropentylsulfinyl, 6-fluorohexylsulfinyl, 6-chlorohexylsulfinyl, 6-bromohexylsulfinyl, 6-iodohexylsulfinyl or dodecafluorohexylsulfinyl.

The term "C₁-C₂-alkylsulfonyl" is a C₁-C₂-alkyl group, as defined above, attached via a sulfonyl [S(O)₂] group. The term "C₁-C₃-alkylsulfonyl" is a C₁-C₃-alkyl group, as defined above, attached via a sulfonyl [S(O)₂] group. The term "C₁-C₄-alkylsulfonyl" is a C₁-C₄-alkyl group, as defined above, attached via a sulfonyl [S(O)₂] group. The term "C₁-C₆-alkylsulfonyl" is a C₁-C₆-alkyl group, as defined above, attached via a sulfonyl [S(O)₂] group. The term "C₁-C₁₀-alkylsulfonyl" is a C₁-C₁₀-alkyl group, as defined above, attached via a sulfonyl [S(O)₂] group. C₁-C₂-Alkylsulfonyl is methylsulfonyl or ethylsulfonyl. C₁-C₃-Alkylsulfonyl is additionally, for example, n-propylsulfonyl or 1-methylethylsulfonyl (isopropylsulfonyl). C₁-C₄-Alkylsulfonyl is additionally, for example, butylsulfonyl, 1-methylpropylsulfonyl (sec-butylsulfonyl), 2-methylpropylsulfonyl (isobutylsulfonyl) or 1,1-dimethylethylsulfonyl (tert-butylsulfonyl). C₁-C₆-Alkylsulfonyl is additionally, for example, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl. C₁-C₈-Alkylsulfonyl is additionally, for example, heptylsulfonyl, octylsulfonyl, 2-ethylhexylsulfonyl and positional isomers thereof. C₁-C₁₀-Alkylsulfonyl is additionally, for example, nonylsulfonyl, decylsulfonyl and positional isomers thereof.

The term "C₁-C₂-haloalkylsulfonyl" is a C₁-C₂-haloalkyl group, as defined above, attached via a sulfonyl [S(O)₂] group. The term "C₁-C₃-haloalkylsulfonyl" is a C₁-C₃-haloalkyl group, as defined above, attached via a sulfonyl [S(O)₂] group. The term "C₁-C₄-haloalkylsulfonyl" is a C₁-C₄-haloalkyl group, as defined above, attached via a sulfonyl [S(O)₂] group. The term "C₁-C₆-haloalkylsulfonyl" is a C₁-C₆-haloalkyl group, as defined above, attached via a sulfonyl [S(O)₂] group. The term "C₁-C₁₀-haloalkylsulfonyl" is a C₁-C₁₀-haloalkyl group, as defined above, attached via a sulfonyl [S(O)₂] group. C₁-C₂-Haloalkylsulfonyl is, for example, S(O)₂CH₂F, S(O)₂CHF₂, S(O)₂CF₃, S(O)₂CH₂Cl, S(O)₂CHCl₂, S(O)₂CCl₃, chlorofluoromethylsulfonyl, dichlorofluoromethylsulfonyl, chlorodifluoromethylsulfonyl, 2-fluoroethylsulfonyl, 2-chloroethylsulfonyl, 2-bromoethylsulfonyl, 2-iodoethylsulfonyl, 2,2-difluoroethylsulfonyl, 2,2,2-trifluoroethylsulfonyl, 2-chloro-2-fluoroethylsulfonyl, 2-chloro-2,2-difluoroethylsulfonyl, 2,2-dichloro-2-fluoroethylsulfonyl, 2,2,2-trichloroethylsulfonyl or S(O)₂C₂F₅. C₁-C₃-Haloalkylsulfonyl is additionally, for example, 2-fluoropropylsulfonyl, 3-fluoropropylsulfonyl, 2,2-difluoropropylsulfonyl, 2,3-difluoropropylsulfonyl, 2-chloropropylsulfonyl, 3-chloropropylsulfonyl, 2,3-dichloropropylsulfonyl, 2-bromopropylsulfonyl, 3-bromopropylsulfonyl, 3,3,3-trifluoropropylsulfonyl, 3,3,3-trichloropropylsulfonyl, S(O)₂CH₂-C₂F₅, S(O)₂CF₂-C₂F₅, 1-(CH₂F)-2-fluoroethylsulfonyl, 1-(CH₂Cl)-2-chloroethylsulfonyl or 1-(CH₂Br)-2-bromoethylsulfonyl. C₁-C₄-Haloalkylsulfonyl is additionally, for example, 4-fluorobutylsulfonyl, 4-chlorobutylsulfonyl, 4-bromobutylsulfonyl or nonafluorobutylsulfonyl. C₁-C₆-Haloalkylsulfonyl is additionally, for example, 5-fluoropentylsulfonyl, 5-chloropentylsulfonyl, 5-brompentylsulfonyl, 5-iodopentylsulfonyl, undecafluoropentylsulfonyl, 6-fluorohexylsulfonyl, 6-chlorohexylsulfonyl, 6-bromohexylsulfonyl, 6-iodohexylsulfonyl or dodecafluorohexylsulfonyl.

The substituent "oxo" (or =O) replaces a CH₂ group by a C(=O) group.

The substituent =S replaces a CH₂ group by a C(=S) group.

The term "alkylcarbonyl" is a C₁-C₆-alkyl ("C₁-C₆-alkylcarbonyl"), preferably a C₁-C₄-alkyl ("C₁-C₄-alkylcarbonyl") group, as defined above, attached via a carbonyl [C(=O)] group. Examples are acetyl (methylcarbonyl), propionyl (ethylcarbonyl), propylcarbonyl, isopropylcarbonyl, n-butylcarbonyl and the like.

The term "haloalkylcarbonyl" is a C₁-C₆-haloalkyl ("C₁-C₆-haloalkylcarbonyl"), preferably a C₁-C₄-haloalkyl ("C₁-C₄-haloalkylcarbonyl") group, as defined above, attached via a carbonyl [C(=O)] group. Examples are trifluoromethylcarbonyl, 2,2,2-trifluoroethylcarbonyl and the like.

The term "alkoxycarbonyl" is a C₁-C₆-alkoxy ("C₁-C₆-alkoxycarbonyl"), preferably a C₁-C₄-alkoxy ("C₁-C₄-alkoxycarbonyl") group, as defined above, attached via a carbonyl [C(=O)] group. Examples are methoxycarbonyl), ethoxycarbonyl, propoxycarbonyl, isopropoxycarbonyl, n-butoxycarbonyl and the like.

The term "haloalkoxycarbonyl" is a C₁-C₆-haloalkoxy ("C₁-C₆-haloalkoxycarbonyl"), preferably a C₁-C₄-haloalkoxy ("C₁-C₄-haloalkoxycarbonyl") group, as defined above, attached via a carbonyl [C(=O)] group. Examples are trifluoromethoxycarbonyl, 2,2,2-trifluoroethoxycarbonyl and the like.

The term "C₁-C₆-alkylamino" is a group -N(H)C₁-C₆-alkyl. Examples are methylamino, ethylamino, propylamino, isopropylamino, butylamino and the like.

The term "di-(C₁-C₆-alkyl)amino" is a group -N(C₁-C₆-alkyl)₂. Examples are dimethylamino, diethylamino, ethylmethylamino, dipropylamino, diisopropylamino, methylpropylamino, methylisopropylamino, ethylpropylamino, ethylisopropylamino, dibutylamino and the like.

The term "aminocarbonyl" is a group -C(O)-NH₂.

The term "C₁-C₆-alkylaminocarbonyl" is a group -C(O)-N(H)C₁-C₆-alkyl. Examples are methylaminocarbonyl, ethylaminocarbonyl, propylaminocarbonyl, isopropylaminocarbonyl, butylaminocarbonyl and the like.

The term "di-(C₁-C₆-alkyl)aminocarbonyl" is a group -C(O)-N(C₁-C₆-alkyl)₂. Examples are dimethylaminocarbonyl, diethylaminocarbonyl, ethylmethylaminocarbonyl, dipropylaminocarbonyl, diisopropylaminocarbonyl, methylpropylaminocarbonyl, methylisopropylaminocarbonyl, ethylpropylaminocarbonyl, ethylisopropylaminocarbonyl, dibutylaminocarbonyl and the like.

The term "3-, 4-, 5-, 6-, 7-, 8-, 9- or 10-membered saturated, partially unsaturated or maximally unsaturated heterocyclic ring (or heteromonocyclic or heterobicyclic ring) containing 1, 2 or 3 (or 4) heteroatoms or heteroatom groups selected from N, O, S, NO, SO and SO₂, as ring members" denotes a 3-, 4-, 5-, 6-, 7-, 8-, 9- or 10-membered, preferably a 3-, 4-, 5-, 6- or 7-membered saturated, partially unsaturated or maximum unsaturated heteromonocyclic ring or a 7-, 8-, 9- or 10-membered saturated, partially unsaturated or maximally unsaturated heterobicyclic ring containing 1, 2 or 3 (or 4) heteroatoms or heteroatom groups selected from N, O, S, NO, SO and SO₂, as ring members.

Unsaturated rings contain at least one C-C and/or C-N and/or N-N double bond(s). Maximally unsaturated rings contain as many conjugated C-C and/or C-N and/or N-N double bonds as allowed by the ring size. Maximally unsaturated 5- or 6-membered heterocyclic rings are aromatic. Partially unsaturated rings contain less than the maximum number of C-C and/or C-N and/or N-N double bond(s) allowed by the ring size. The heterocyclic ring may be attached to the remainder of the molecule via a carbon ring member or via a nitrogen ring member. As a matter of course, the heterocyclic ring contains at least one carbon ring atom. If the ring contains more than one O ring atom, these are not adjacent.

The term "3-, 4-, 5-, 6- or 7-membered saturated, partially unsaturated or maximum unsaturated heterocyclic ring containing 1, 2 or 3 (or 4) heteroatoms or heteroatom groups selected from N, O, S, NO, SO and SO₂, as ring members" [wherein "maximum unsaturated" includes also "aromatic"] as used herein denotes monocyclic radicals, the monocyclic radicals being saturated, partially unsaturated or maximum unsaturated (including aromatic). The term "3-, 4-, 5-, 6-, 7- or 8-membered saturated, partially unsaturated or maximum unsaturated heterocyclic ring containing 1, 2 or 3 (or 4) heteroatoms or heteroatom groups selected from N, O, S, NO, SO and SO₂, as ring members" [wherein "maximum unsaturated" includes also "aromatic"] as used herein further also encompasses 8-membered heteromonocyclic radicals containing 1, 2 or 3 (or 4) heteroatoms or heteroatom groups selected from N, O, S, NO, SO and SO₂, as ring members, the monocyclic radicals being saturated, partially unsaturated or maximum unsaturated (including aromatic). Unsaturated rings contain at least one C-C and/or C-N and/or N-N double bond(s). Maximum unsaturated rings contain as many conjugated C-C and/or C-N and/or N-N double bonds as allowed by the ring size. Maximum unsaturated 5- or 6-membered heterocyclic rings are aromatic. 7- and 8-membered rings cannot be aromatic. They are homoaromatic (7-membered ring, 3 double bonds) or have 4 double bonds (8-membered ring). The heterocyclic ring may be attached to the remainder of the molecule via a carbon ring member or via a nitrogen ring member. As a matter of course, the heterocyclic ring contains at least one carbon ring atom. If the ring contains more than one O ring atom, these are not adjacent.

Examples of a 3-, 4-, 5-, 6- or 7-membered saturated heterocyclic ring include: Oxiranyl, thiiranyl, aziridinyl, oxetanyl, thietanyl, azetidinyl, tetrahydrofuran-2-yl, tetrahydrofuran-3-yl, tetrahydrothien-2-yl, tetrahydrothien-3-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-1-yl, 1,3,4-triazolidin-2-yl, 2-tetrahydropyranyl, 4-tetrahydropyranyl, 1,3-dioxan-5-yl, 1,4-dioxan-2-yl, piperidin-1-yl, piperidin-2-yl, piperidin-3-yl, piperidin-4-yl, hexahydropyridazin-3-yl, hexahydropyridazin-4-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl and 1,2,4-hexahydrotriazin-3-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl, azepan-1-, -2-, -3- or-4-yl, oxepan-2-, -3-, -4- or -5-yl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl and the like.

Examples of a 3-, 4-, 5-, 6- or 7-membered partially unsaturated heterocyclic ring include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6- or -7-yl, 3,4,5,6-tetrahydro[2H]azepin-2-, -3-, -4-, -5-, -6- or -7-yl, 2,3,4,7-tetrahydro[1H]azepin-1-, -2-, -3-, -4-, -5-, -6- or -7-yl, 2,3,6,7-tetrahydro[1H]azepin-1-, -2-, -3-, -4-, -5-, -6- or -7-yl, tetrahydrooxepinyl, such as 2,3,4,5-tetrahydro[1H]oxepin-2-, -3-, -4-, -5-, -6- or -7-yl, 2,3,4,7-tetrahydro[1H]oxepin-2-, -3-, -4-, -5-, -6- or -7-yl, 2,3,6,7-tetrahydro[1H]oxepin-2-, -3-, -4-, -5-, -6- or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl and tetrahydro-1,4-dioxepinyl.

Examples for a 3-, 4-, 5-, 6- or 7-membered maximally unsaturated (including aromatic) heterocyclic ring are 5- or 6-membered heteroaromatic rings, such as 2-furyl, 3-furyl, 2-thienyl, 3-thienyl, 1-pyrrolyl, 2-pyrrolyl, 3-pyrrolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,3,4-triazol-1-yl, 1,3,4-triazol-2-yl, 2-pyridinyl, 3-pyridinyl, 4-pyridinyl, 1-oxopyridin-2-yl, 1-oxopyridin-3-yl, 1-oxopyridin-4-yl, 3-pyridazinyl, 4-pyridazinyl, 2-pyrimidinyl, 4-pyrimidinyl, 5-pyrimidinyl and 2-pyrazinyl, and also homoaromatic radicals, such as 1H-azepine, 1H-[1,3]-diazepine and 1H-[1,4]-diazepine.

In the present invention, the "heterobicyclic rings" contain two rings which have at least one ring atom in common. At least one of the two rings contains a heteroatom or heteroatom group selected from N, O, S, NO, SO and SO₂ as ring member. The term comprises condensed (fused) ring systems, in which the two rings have two neighboring ring atoms in common, as well as spiro systems, in which the rings have only one ring atom in common, and bridged systems with at least three ring atoms in common.

### Examples for fused systems:

Examples for a 7-, 8-, 9- or 10-membered saturated heterobicyclic ring containing 1, 2 or 3 (or 4) heteroatoms or heteroatom groups selected from N, O, S, NO, SO and SO₂, as ring members are:

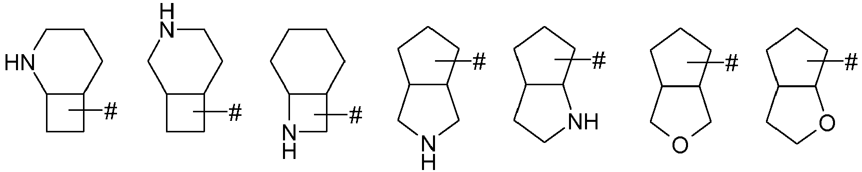

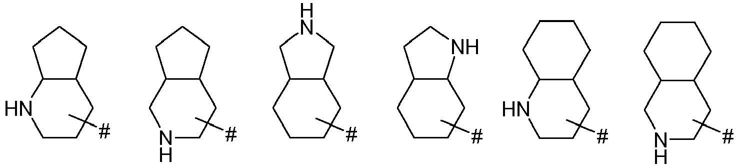

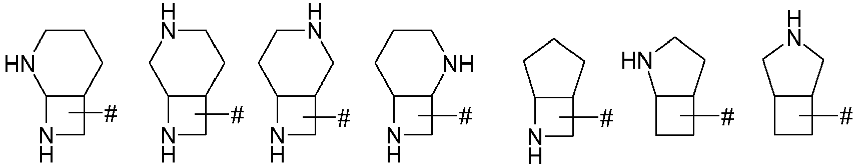

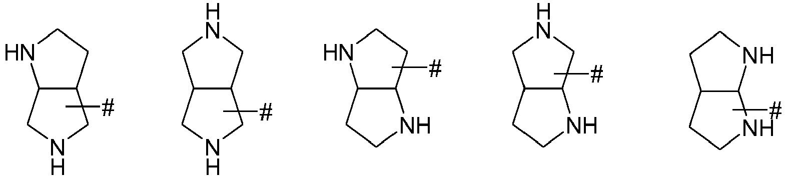

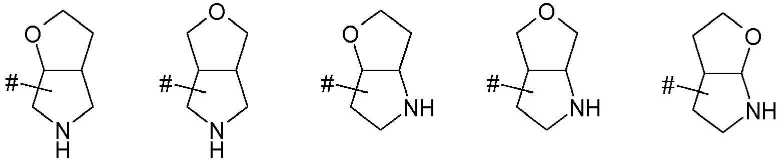

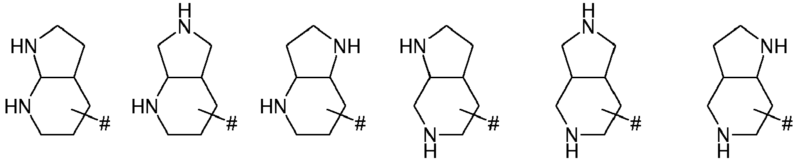

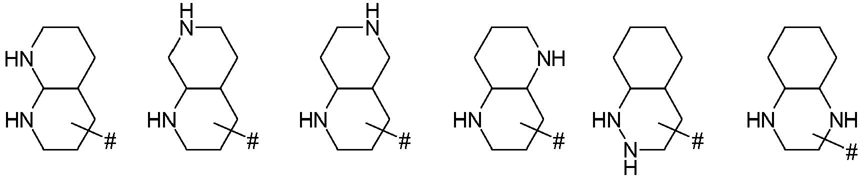

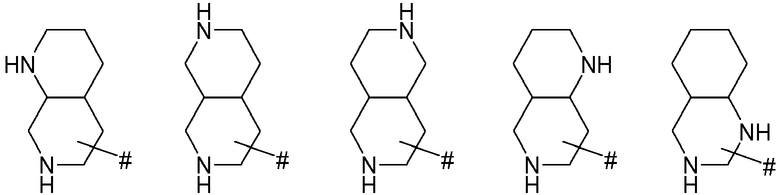

Examples for a 8-, 9- or 10-membered partially unsaturated heterobicyclic ring containing 1, 2 or 3 (or 4) heteroatoms or heteroatom groups selected from N, O, S, NO, SO and SO₂, as ring members are:

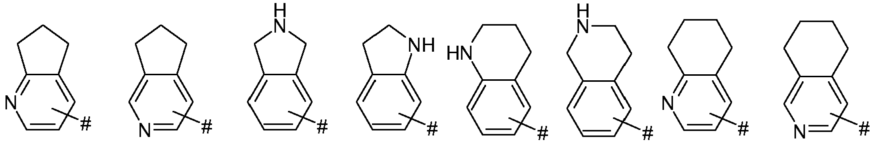

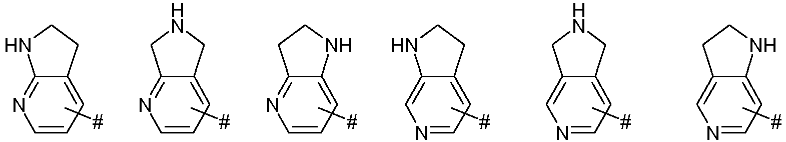

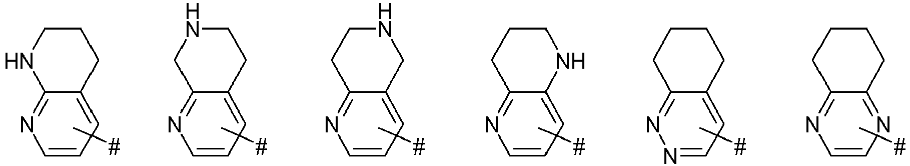

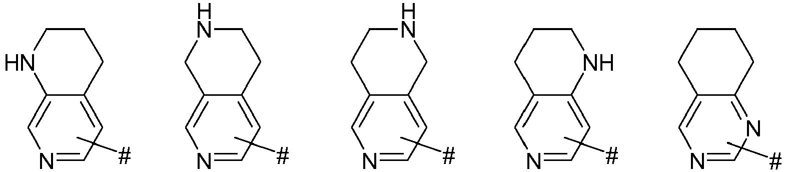

Examples for a 8-, 9- or 10-membered maximally unsaturated heterobicyclic ring containing 1, 2 or 3 (or 4) heteroatoms or heteroatom groups selected from N, O, S, NO, SO and SO₂, as ring members are:

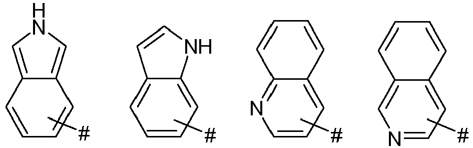

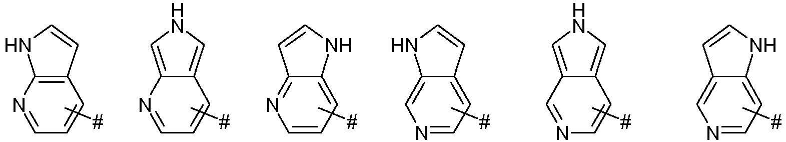

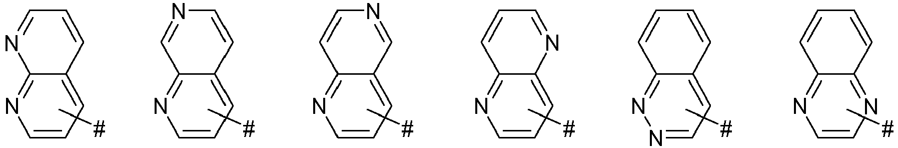

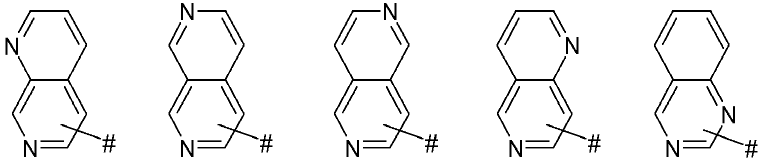

Examples for spiro-bound 7-, 8-, 9- or 10-membered heterobicyclic rings containing 1, 2 or 3 (or 4) heteroatoms or heteroatom groups selected from N, O, S, NO, SO and SO₂, as ring members are

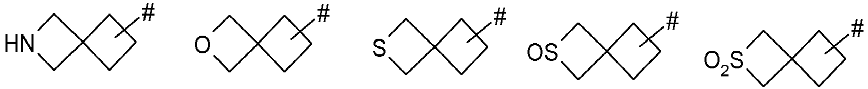

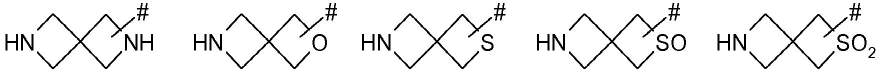

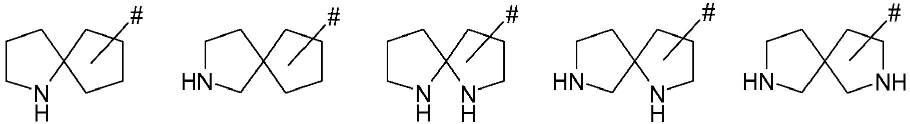

Examples for bridged 7-, 8-, 9- or 10-membered heterobicyclic rings containing 1, 2 or 3 (or 4) heteroatoms or heteroatom groups selected from N, O, S, NO, SO and SO₂, as ring members are 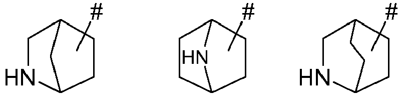 and the like.

In the above structures # denotes the attachment point to the remainder of the molecule. The attachment point is not restricted to the ring on which is shown, but can be on either of the fused rings, and may be on a carbon or on a nitrogen ring atom. If the rings carry one or more substituents, these may be bound to carbon and/or to nitrogen ring atoms (if the latter are not part of a double bond).

If the above-defined heteromonocyclic or heterobicyclic rings are those formed by R⁵ and R⁶ together with the nitrogen atom they are bound to, only those of the above-defined rings are suitable which contain at least one secondary nitrogen atom (i.e. NH) as a ring member, and in which the attachment point to the group C=W is on this secondary nitrogen atom (see also below embodiments of R⁵ and R⁶).

The remarks made below concerning preferred embodiments of the variables of the compounds of formula I, especially with respect to their substituents B¹, B², B³, G¹, G², G³, G⁴, R¹, R², R^{3a}, R^{3b}, R^{3c}, R^{3d}, R⁴, R⁵, R⁶, R⁷, R⁸, R⁹, R^{10a}, R^{10b}, R¹¹, R¹², R¹³, R¹⁴, R^{14a}, R^{14b}, R¹⁵, R¹⁶, m and n, the features of the use and method according to the invention and of the composition of the invention are valid both on their own and, in particular, in every possible combination with each other.

In a preferred embodiment of the description, the heteromonocyclic ring formed by R⁵ and R⁶ together with the nitrogen atom to which they are bound mandatorily carries at least one substituent R⁷, while the heterobicyclic ring formed by R⁵ and R⁶ together with the nitrogen atom to which they are bound optionally carries at least one substituent R⁷. Thus, preferably, R⁵ and R⁶, together with the nitrogen atom to which they are bound, form a 3-, 4-, 5-, 6-, 7-, 8-, 9- or 10-membered saturated, partially unsaturated or maximally unsaturated heteromonocyclic or heterobicyclic ring, where the ring may further contain 1, 2, 3 or 4 heteroatoms or heteroatom-containing groups selected from O, S, N, NH, SO, and SO₂, as ring members, wherein the heteromonocyclic ring is (mandatorily) substituted with 1, 2, 3, 4, 5, 6, 7 or 8 substituents R⁷, and where the heterobicyclic ring may be substituted with 1, 2, 3, 4, 5, 6, 7 or 8 substituents R⁷; where R⁷ has one of the above general or, in particular, one of the below preferred meanings.

In the invention, the heterocyclic ring formed by R⁵ and R⁶ together with the nitrogen atom to which they are bound is monocyclic. Thus, R⁵ and R⁶, together with the nitrogen atom to which they are bound, form a 3-, 4-, 5- or 6-membered saturated, heteromonocyclic ring, where the ring may further contain 1 or 2 heteroatoms or heteroatom-containing groups selected from O, S, NH, SO, and SO₂, as ring members, wherein the heteromonocyclic ring may be (and preferably is) substituted with 1, 2, 3, 4, 5 or 6 substituents R⁷; where R⁷ has one of the above general or, in particular, one of the below preferred meanings.

More preferably, R⁵ and R⁶, together with the nitrogen atom to which they are bound, form a 3-, 4-, 5- or 6-membered saturated heteromonocyclic ring, where the ring may further contain 1 or 2, preferably 1, heteroatoms or heteroatom-containing groups selected from O and NH as ring members, wherein the heterocyclic ring may carry (preferably carries) 1, 2, 3, 4, 5 or 6 substituents R⁷, where R⁷ has one of the above general or, in particular, one of the below preferred meanings.

Especially, R⁵ and R⁶, together with the nitrogen atom to which they are bound, form a 3-, 4-, 5- or 6-membered saturated heteromonocyclic ring, where the ring may further contain 1 or 2 heteroatom-containing groups NH as ring members, wherein the heterocyclic ring may carry (preferably carries) 1, 2, 3, 4, 5 or 6 substituents R⁷, where R⁷ has one of the above general or, in particular, one of the below preferred meanings.

Suitable heteromonocyclic rings formed by R⁵ and R⁶ together with the nitrogen atom they are bound to are for example: aziridin-1-yl, diaziridin-1-yl, azetidin-1-yl, 1,2-diazetidin-1-yl, 1,3-diazetidin-1-yl, pyrrolidin-1-yl, pyrazolidin-1-yl, imidazolidin-1-yl, 1,2,3-triazolidin-1-yl, 1,2,3-triazolidin-2-yl, 1,2,4-triazolidin-1-yl, 1,2,3-triazolidin-4-yl, piperidin-1-yl, hexahydropyrimidin-1-yl, piperazin-1-yl, azepan-1-yl, 1,4-diazepan-1-yl, isothiazolidin-2-yl, 1-oxo-isothiazolidin-2-yl, 1,1-dioxo-isothiazolidin-2-yl, thiazolidin-3-yl, isoxazolidin-2-yl, oxazolidin-3-yl, morpholin-4-yl (also termed morpholino), thiomorpholin-4-yl (also termed thiomorpholino), 1-oxothiomorpholin-4-yl, 1,1-dioxothiomorpholin-4-yl, 1,4-thiazinan-4-yl, 1-oxo-1,4-thiazinan-4-yl and 1,1-dioxo-1,4-thiazinan-4-yl. Among the above monocyclic rings, preference is given to aziridin-1-yl, azetidin-1-yl, 1,2-diazetidin-1-yl, 1,3-diazetidin-1-yl, pyrrolidin-1-yl, imidazolidin-1-yl, [1,2,4]-triazolidin-1-yl, piperidin-1-yl, piperazin-1-yl, hexahydropyrimidin-1-yl, morpholin-4-yl, thiomorpholin-4-yl, 1-oxo-1,4-thiazinan-4-yl and 1,1-dioxo-1,4-thiazinan-4-yl. Even more preference is given to azetidin-1-yl, pyrrolidin-1-yl and piperidin-1-yl.

Preferably these heteromonocyclic rings carry 1, 2, 3, 4, 5, 6, 7 or 8 substituents R⁷. According to the invention, these heteromonocyclic rings carry 1, 2, 3, 4, 5 or 6 substituents R⁷. In these heteromonocyclic rings, R⁷ has independently of each other one of the above general, or in particular, one of the below preferred meanings.

In another more preferred embodiment of the description, R⁵ and R⁶, together with the nitrogen atom to which they are bound, form a 7-, 8-, 9- or 10-membered saturated, partially unsaturated or maximally unsaturated heterobicyclic ring, where the ring may further contain 1, 2, 3 or 4 heteroatoms or heteroatom-containing groups selected from O, S, N, NH, SO, and SO₂, as ring members, wherein the heterobicyclic ring may be substituted with 1, 2, 3, 4, 5, 6, 7 or 8 substituents R⁷; where R⁷ has one of the above general or, in particular, one of the below preferred meanings.

In this aspect of the description, R⁵ and R⁶, together with the nitrogen atom to which they are bound, preferably form a 7-, 8-, 9- or 10-membered saturated, partially unsaturated or maximally unsaturated heterobicyclic ring, where the ring may further contain 1 or 2 heteroatoms or heteroatom-containing groups selected from O, S, SO, SO₂, N and NH as ring members, wherein the heterocyclic ring may be substituted with 1, 2, 3 or 4 substituents R⁷, where R⁷ has one of the above general or, in particular, one of the below preferred meanings.

Even more preferably, in the description, R⁵ and R⁶, together with the nitrogen atom to which they are bound, form a 7-, 8-, 9- or 10-membered saturated or partially unsaturated heterobicyclic ring, where the ring may further contain 1 or 2 heteroatoms or heteroatom-containing groups selected from O, S, N and NH as ring members, wherein the heterocyclic ring is unsubstituted or substituted with 1, 2, 3 or 4 substituents R⁷, where R⁷ has one of the above general or, in particular, one of the below preferred meanings.

In an alternative even more preferred aspect of the description, R⁵ and R⁶, together with the nitrogen atom to which they are bound, form a 7-, 8-, 9- or 10-membered saturated heterobicyclic ring, where the ring may further contain 1 or 2, preferably 1, heteroatoms or heteroatom-containing groups selected from O, S, SO, SO₂, and NH as ring members, wherein the heterobicyclic ring may be substituted with 1, 2, 3 or 4, 5 or 6 substituents R⁷, where R⁷ has one of the above general or, in particular, one of the below preferred meanings.

Suitable heterobicyclic rings of the description formed by N, R⁵ and R⁶ are for example the following: 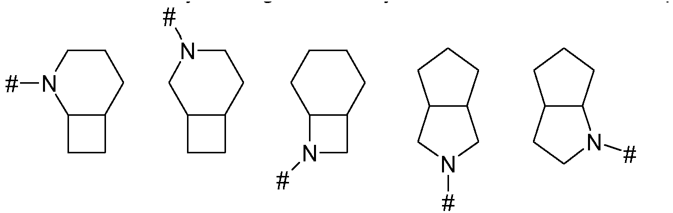 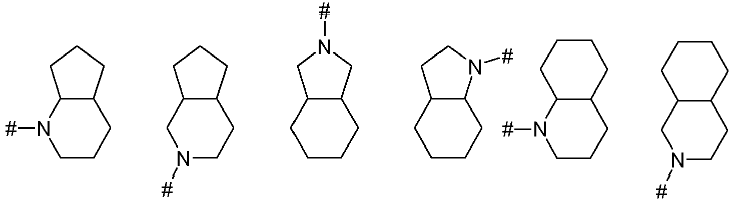 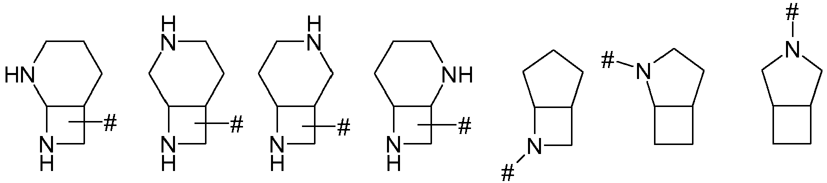 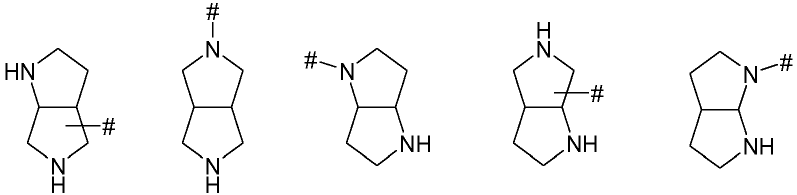 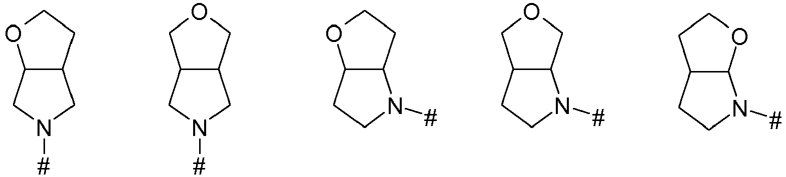 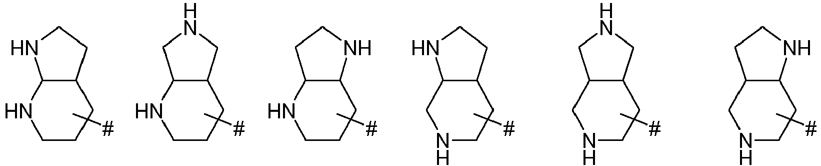 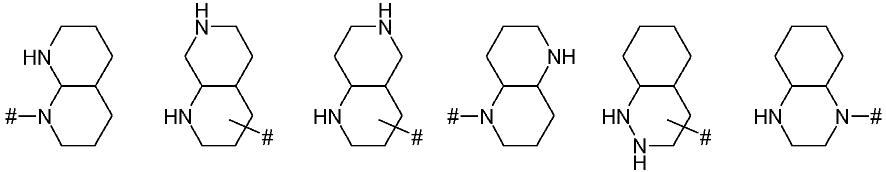 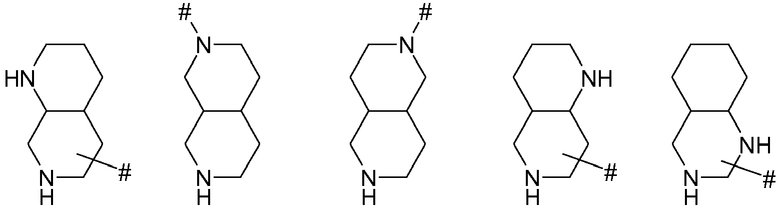 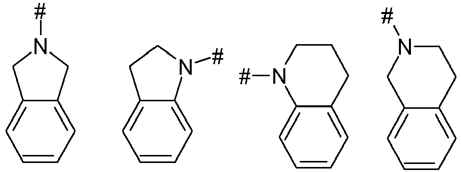 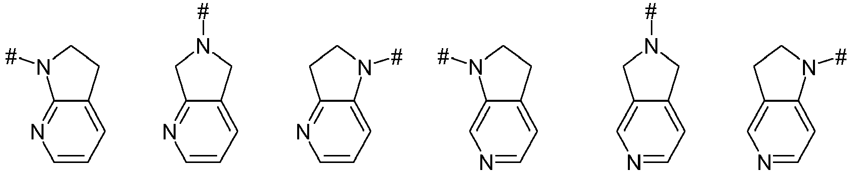 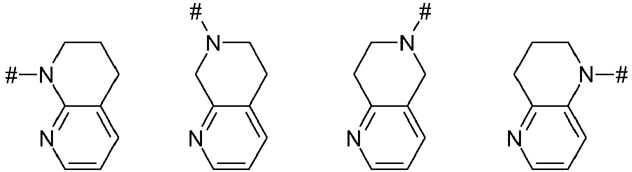 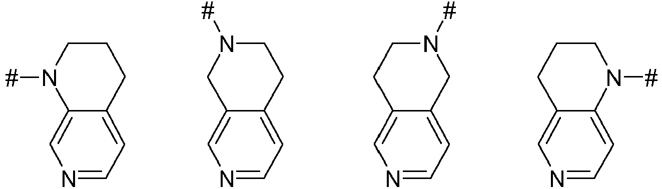 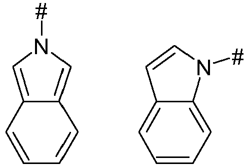 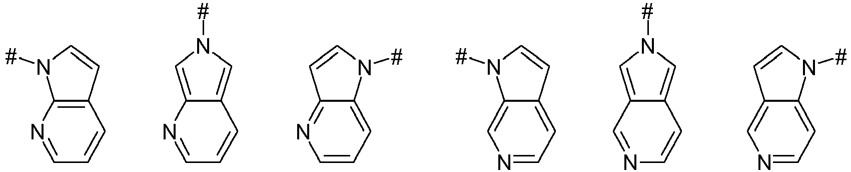 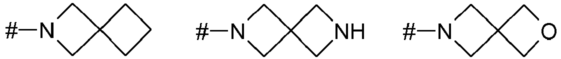 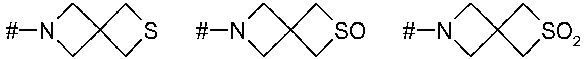 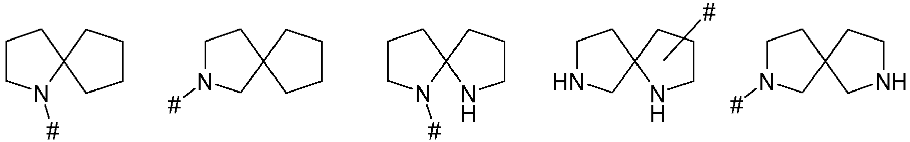 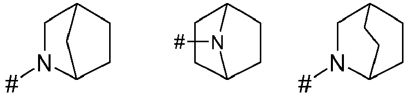 wherein # denotes the attachment point to C(=W). Some of the rings containing two secondary nitrogen ring atoms show the attachment point unlocalized. In this case, the attachment point is localized on either one of the two nitrogen ring atoms.

Among the above heterobicyclic rings of the description, preference is given to the following: 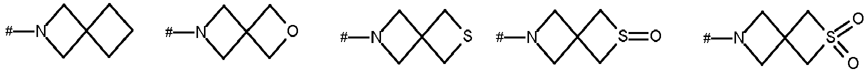 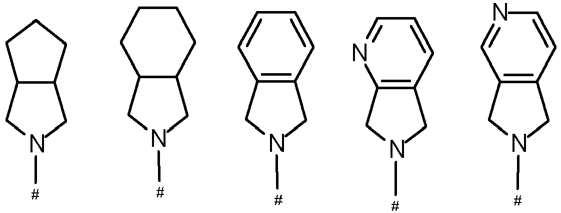 wherein # denotes the attachment point to C(=W).

These heterobicyclic rings of the description may carry 1, 2, 3, 4 or 5, preferably 1, 2 or 3, more preferably 1 or 2, substituents R⁷, wherein each R⁷ has independently one of the above general, or in particular, one of the below preferred meanings.

The radical R⁷ can be a substituent on a carbon ring atom as well as on a nitrogen ring atom. In case that it is a substituent on a nitrogen atom, it is not selected from halogen, azido, nitro, -SCN, -SF₅, -Si(R¹²)₃, -OR⁹ and -OSO₂R⁹, and preferably not from -S(O)ₙR⁹ and -N(R^{10a})R^{10b}, either.

Thus, as a nitrogen substituent, R⁷ is selected from cyano, C₁-C₆-alkyl, C₃-C₈-cycloalkyl, C₂-C₆-alkenyl, C₂-C₆-alkynyl, wherein the four last-mentioned aliphatic and cycloaliphatic radicals may be partially or fully halogenated and/or may be substituted by one or more radicals R⁸;
-OR⁹, -S(O)ₙR⁹, -N(R^{10a})R^{10b}, -C(=O)N(R^{10a})R^{10b}, -C(=S)N(R^{10a})R^{10b}, -C(=O)OR⁹, -C(=O)R⁸,
phenyl which may be substituted with 1, 2, 3, 4 or 5 substituents R¹¹; and a 3-, 4-, 5-, 6-, 7-, 8-, 9- or 10-membered saturated, partially unsaturated or maximally unsaturated heteromonocyclic or heterobicyclic ring containing 1, 2, 3 or 4 heteroatoms or heteroatom groups selected from N, O, S, NO, SO and SO₂, as ring members, where the heteromonocyclic or heterobicyclic ring may be substituted by one or more radicals R¹¹;
and preferably from cyano, C₁-C₆-alkyl, C₃-C₈-cycloalkyl, C₂-C₆-alkenyl, C₂-C₆-alkynyl, wherein the four last-mentioned aliphatic and cycloaliphatic radicals may be partially or fully halogenated and/or may be substituted by one or more radicals R⁸; -C(=O)N(R^{10a})R^{10b}, -C(=S)N(R^{10a})R^{10b}, -C(=O)OR⁹, -C(=O)R⁸,
phenyl which may be substituted with 1, 2, 3, 4 or 5 substituents R¹¹; and a 3-, 4-, 5-, 6-, 7-, 8-, 9- or 10-membered saturated, partially unsaturated or maximally unsaturated heteromonocyclic or heterobicyclic ring containing 1, 2, 3 or 4 heteroatoms or heteroatom groups selected from N, O, S, NO, SO and SO₂, as ring members, where the heteromonocyclic or heterobicyclic ring may be substituted by one or more radicals R¹¹.

These provisos apply to the below preferred embodiments of R⁷ accordingly.

Preferably, each R⁷ is independently selected from halogen, cyano, oxo, C₁-C₆-alkyl, C₃-C₈-cycloalkyl, C₂-C₆-alkenyl, C₂-C₆-alkynyl, wherein the four last-mentioned aliphatic and cycloaliphatic radicals may be partially or fully halogenated and/or may be substituted by one or more radicals R⁸; -OR⁹, -S(O)ₙR⁹, -N(R^{10a})R^{10b},-C(=O)N(R^{10a})R^{10b}, -C(=S)N(R^{10a})R^{10b}, -C(=O)OR⁹, -C(=O)R⁸, phenyl which may be substituted with 1, 2, 3, 4 or 5 substituents R¹¹; and a 3-, 4-, 5- or 6-membered saturated, partially unsaturated or maximally unsaturated heteromonocyclic ring containing 1, 2 or 3 heteroatoms or heteroatom groups selected from N, O, S, NO, SO and SO₂, as ring members, where the heteromonocyclic ring may be substituted by one or more radicals R¹¹, wherein n, R⁸, R⁹, R^{10a}, R^{10b} and R¹¹ have one of the above general, or in particular, one of the below preferred meanings.

It is self-evident that if R⁷ has the meaning oxo, 2 radicals R⁷ bound to the same carbon atom form a radical =O. It is also self-evident that if R⁷ has the meaning =S, 2 radicals R⁷ bound to the same carbon atom form a radical =S. According to the invention, each R⁷ is independently selected from halogen, cyano, oxo, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, wherein the 8 last-mentioned substituents may carry a radical R⁸; C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, -N(R^{10a})R^{10b}, -C(=O)N(R^{10a})R^{10b}, -C(=S)N(R^{10a})R^{10b}, and -C(=O)R⁸, wherein R⁸, R^{10a} and R^{10b} have one of the above general, or in particular, one of the below preferred meanings.

In R⁷, the radical R⁸ as a substituent on an aliphatic or cycloaliphatic group, such as a C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl or C₂-C₆-haloalkynyl group, is preferably selected from cyano; C₃-C₈-cycloalkyl which may be substituted by 1 or 2 substituents selected from CN, methyl, and oxo; C₃-C₈-halocycloalkyl; -OR⁹; -S(O)ₙR⁹; -N(R^{10a})R^{10b}; -C(=O)N(R^{10a})R^{10b}; -C(=O)OR⁹; phenyl, optionally substituted with 1, 2, 3, 4 or 5 substituents R¹⁶; and a 3-, 4-, 5-, 6- or 7- membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶, wherein n, R⁹, R^{10a}, R^{10b} and R¹⁶ have one of the above general, or in particular, one of the below preferred meanings.

According to the invention, R⁸ is selected from cyano, C₃-C₆-cycloalkyl which may be substituted by 1 or 2 substituents selected from CN, methyl and oxo; C₃-C₆-halocycloalkyl, and -OR⁹.

In the group -C(=O)R⁸ as a preferred meaning for R⁷, the radical R⁸ is preferably selected from hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b}; wherein R⁹, R^{10a}, R^{10b} and R¹⁶ have one of the above general, or in particular, one of the below preferred meanings.

According to the invention, in this embodiment, R⁸ is more preferably selected from hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl, C₃-C₈-halocycloalkyl-C₁-C₄-alkyl, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b}; wherein R⁹, R^{10a}, R^{10b} and R¹⁶ have one of the above general, or in particular, one of the below preferred meanings. Even more preferably, R⁸ is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₂-alkyl-, especially C₃-C₈-cycloalkyl-CH₂-, C₃-C₈-halocycloalkyl-C₁-C₂-alkyl-, especially C₃-C₈-halocycloalkyl-CH₂-, where the cycloalkyl moieties in the 6 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b}, wherein R⁹, R^{10a} and R^{10b} have one of the above general, or in particular, one of the below preferred meanings. In particular, R⁸ is selected from C₁-C₆-alkyl, C₁-C₆-haloalkyl, -OR⁹ and -N(R^{10a})R^{10b}, wherein R⁹, R^{10a} and R^{10b} have one of the above general, or in particular, one of the below preferred meanings.

In -OR⁹, -S(O)ₙR⁹, or -C(=O)OR⁹ as a preferred meaning for R⁷, the radical R⁹ is preferably selected from hydrogen, C₁-C₆-alkyl, C₁-C₆-haloalkyl, C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, C₂-C₆-alkenyl, C₂-C₆-haloalkenyl, C₂-C₆-alkynyl, C₂-C₆-haloalkynyl, phenyl, optionally substituted with 1, 2, 3, 4 or 5 substituents R¹⁶, and a 3-, 4-, 5-, 6- or 7- membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶; and preferably from hydrogen, C₁-C₆-alkyl and C₁-C₆-haloalkyl; wherein each R¹⁶ is independently selected from the group consisting of halogen, cyano, nitro, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-C₆-cycloalkyl, C₃-C₆-halocycloalkyl, C₂-C₄-alkenyl, C₂-C₄-haloalkenyl, C₂-C₄-alkynyl and C₂-C₄-haloalkynyl; or two R¹⁶ present on the same carbon atom of a saturated ring may form together =O or =S.

According to the invention, R⁹ is selected from hydrogen, C₁-C₆-alkyl and C₁-C₆-haloalkyl, and is specifically C₁-C₆-alkyl.

In -NR^{10a}R^{10b}, --C(=O)N(R^{10a})R^{10b} or in --C(=S)N(R^{10a})R^{10b} as a preferred meaning of R⁷, the radicals R^{10a} and R^{10b}, independently of each other, are preferably selected from hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group;
C₂-C₆-alkenyl, C₂-C₆-haloalkenyl, C₂-C₆-alkynyl, C₂-C₆-haloalkynyl, -C(=O)R¹³, -C(=O)N(R^{14a})R^{14b},
phenyl, optionally substituted with 1, 2, 3, 4 or 5 substituents R¹⁶, and a 3-, 4-, 5-, 6- or 7- membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶,
where R¹³, R^{14a}, R^{14b} and R¹⁶ have one of the above general, or in particular, one of the preferred meanings.

More preferably:
- R^{10a}: is hydrogen or methyl; and
- R^{10b}: is selected from hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -C(=O)R¹³, -C(=O)N(R^{14a})R^{14b}, phenyl, optionally substituted with 1, 2, 3, 4 or 5 substituents R¹⁶; and a 3-, 4-, 5-, 6- or 7- membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more substituents R¹⁶,
wherein R¹³, R^{14a}, R^{14b} and R¹⁶ have one of the above general meanings, or, in particular, R¹⁶ has one of the above preferred meanings and R¹³, R^{14a} and R^{14b} have one of the below preferred meanings.

Even more preferably:
- R^{10a}: is hydrogen; and
- R^{10b}: is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl C₂-C₆-haloalkynyl, -C(=O)R¹³ and -C(=O)N(R^{14a})R^{14b}; wherein R¹³, R^{14a} and R^{14b} have one of the above general meanings, or, in particular, one of the below preferred meanings

Particularly:
- R^{10a}: is hydrogen; and
- R^{10b}: is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl and C₂-C₆-haloalkynyl.

In particular,
R^{10a} is hydrogen; and
R^{10b} is selected from -C(=O)R¹³ and -C(=O)N(R^{14a})R^{14b};
wherein R¹³, R^{14a} and R^{14b} have one of the above general meanings, or, in particular, one of the below preferred meanings. According to the invention:
- R^{10a}: is hydrogen or methyl, preferably hydrogen; and
- R^{10b}: is selected from hydrogen, C₁-C₆-alkyl, C₁-C₆-haloalkyl, -C(=O)R¹³ and -C(=O)N(R^{14a})R^{14b};
wherein R¹³, R^{14a} and R^{14b} have one of the above general meanings, or, in particular, one of the below preferred meanings.

In the above mentioned radical R^{10a} and R^{10b}
R¹³ preferably is selected from hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₃-C₆-cycloalkyl, C₃-C₆-halocycloalkyl, C₃-C₆-cycloalkyl-C₁-C₄-alkyl-, where the cycloalkyl moieties in the three last-mentioned groups may be substituted by a cyano group; C₁-C₆-alkyl substituted with a CN group, C₁-C₄-alkoxy and C₁-C₄-haloalkoxy.
Specifically, in the above mentioned radical R^{10a} and R^{10b}
R¹³ is selected from hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₃-C₆-cycloalkyl, C₃-C₆-halocycloalkyl, C₃-C₆-cycloalkyl-C₁-C₄-alkyl-, where the cycloalkyl moieties in the three last-mentioned groups may be substituted by a cyano group; and C₁-C₆-alkyl substituted with a CN group.

In the invention, in the above mentioned radical R^{10a} and R^{10b} R¹³ is selected from C₁-C₆-alkyl, C₁-C₆-haloalkyl, C₃-C₆-cycloalkyl, C₃-C₆-halocycloalkyl, where the cycloalkyl moieties in the two last-mentioned groups may be substituted by a cyano group; C₁-C₄-alkoxy-C₁-C₄-alkyl, C₁-C₄-alkoxy and C₁-C₄-haloalkoxy.

In the above radicals R^{10a} and R^{10b}
- R^{14a}: is preferably selected from hydrogen and C₁-C₆-alkyl; and
- R^{14b}: is preferably selected from hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₃-C₆-cycloalkyl, C₃-C₆-halocycloalkyl, C₃-C₆-cycloalkyl-C₁-C₄-alkyl-, where the cycloalkyl moieties in the three last-mentioned groups may be substituted by a cyano group; and C₁-C₆-alkyl substituted with a CN group.

In the invention, in the above radicals R^{10a} and R^{10b}
- R^{14a}: is preferably selected from hydrogen and C₁-C₆-alkyl; and
- R^{14b}: is preferably selected from hydrogen, C₁-C₆-alkyl and C₁-C₆-haloalkyl.

More preferably, R⁷ is selected from halogen, cyano, oxo, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group, C₂-C₆-alkenyl, C₂-C₆-haloalkenyl, C₂-C₆-alkynyl, C₂-C₆-haloalkynyl, -NR^{10a}NR^{10b}, -C(=O)N(R^{10a})R^{10b}, and -C(=O)R⁸, wherein R⁸, R^{10b} and R^{10b} have one of the above general meanings, or, in particular, have one of the above preferred meanings. Even more preferably, R⁷ is selected from halogen, cyano, oxo, C₁-C₆-alkyl, C₁-C₆-haloalkyl, -N(R^{10a})R^{10b}, -C(=O)N(R^{10a})R^{10b}, and -C(=O)R⁸, wherein R⁸, R^{10b} and R^{10b} have one of the above general meanings, or, in particular, have one of the above preferred meanings.

In particular, R⁷ is selected from fluorine, cyano, oxo, methyl, ethyl, n-propyl, isopropyl, butyl, isobutyl, CF₃, CH₂CHF₂, CH₂CHF₂, CH₂CF₃, C₁-C₄-alkyl substituted by a cyano group; cyclopropyl, cyclobutyl, cyclopropyl substituted by a cyano group; cyclobutyl substituted by a cyano group, C₃-C₆-halocycloalkyl, C₃-C₆-cycloalkyl-C₁-C₂-alkyl-, C₃-C₆-halocycloalkyl-C₁-C₆-alkyl-, where the cycloalkyl moiety in the 3 last-mentioned radicals may carry a CN group; allyl, propargyl, -NHC(=O)R¹³, -NHC(=O)N(R^{14a})R^{14b}, -C(=O)NHR^{10b} and -C(=O)R⁸, wherein
- R⁸: is hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₂-alkyl-, C₃-C₈-halocycloalkyl-C₁-C₂-alkyl-, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b}, where R⁹, R^{10a} and R^{10b} have one of the preferred meanings;
- R¹³: is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₃-C₆-cycloalkyl, C₃-C₆-halocycloalkyl, C₃-C₆-cycloalkyl-C₁-C₄-alkyl-, where the cycloalkyl moieties in the three last-mentioned groups may be substituted by a cyano group; C₁-C₆-alkyl substituted with a CN group, C₁-C₄-alkoxy and C₁-C₄-haloalkoxy; and
- R^{10b}: is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl and C₂-C₆-haloalkynyl.

Especially, R⁷ is selected from fluorine, cyano, oxo, methyl, ethyl, n-propyl, isopropyl, butyl, isobutyl, CF₃, CH₂CHF₂, CH₂CHF₂, CH₂CF₃, C₁-C₄-alkyl substituted by a cyano group; cyclopropyl, cyclobutyl, cyclopropyl substituted by a cyano group; cyclobutyl substituted by a cyano group, C₃-C₆-halocycloalkyl, C₃-C₆-cycloalkyl-C₁-C₂-alkyl-, C₃-C₆-halocycloalkyl-C₁-C₆-alkyl-, where the cycloalkyl moiety in the 3 last-mentioned radicals may carry a CN group; allyl, propargyl, -NHC(=O)R¹³, -C(=O)NHR^{10b} and -C(=O)R⁸, wherein
- R⁸: is hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₂-alkyl-, C₃-C₈-halocycloalkyl-C₁-C₂-alkyl-, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b}, where R⁹, R^{10a} and R^{10b} have one of the preferred meanings;
- R¹³: is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl, C₃-C₆-cycloalkyl, C₃-C₆-halocycloalkyl, C₃-C₆-cycloalkyl-C₁-C₄-alkyl-, where the cycloalkyl moieties in the three last-mentioned groups may be substituted by a cyano group; and C₁-C₆-alkyl substituted with a CN group and
- R^{10b}: is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl and C₂-C₆-haloalkynyl.

In an alternative specific embodiment, R⁷ is selected from fluorine, cyano, oxo, methyl, ethyl, n-propyl, isopropyl, butyl, isobutyl, CF₃, CH₂CHF₂, CH₂CHF₂, CH₂CF₃, -NHC(=O)R¹³, -NHC(=O)N(R^{14a})R^{14b}, -C(=O)NHR^{10b} and -C(=O)R⁸, wherein
- R⁸: is C₁-C₆-alkyl, C₁-C₆-haloalkyl, or -OR⁹ and -N(R^{10a})R^{10b}, where R⁹ has one of the above preferred meanings and is in particular C₁-C₆-alkyl or C₁-C₆-haloalkyl;
- R¹³: is C₁-C₆-alkyl, C₁-C₆-haloalkyl, C₃-C₆-cycloalkyl, C₃-C₆-halocycloalkyl, where the cycloalkyl moieties in the two last-mentioned groups may be substituted by a cyano group; C₁-C₄-alkoxy-C₁-C₄-alkyl, C₁-C₄-alkoxy and C₁-C₄-haloalkoxy;
- R^{10b}: is selected from C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl-, C₃-C₈-halocycloalkyl-C₁-C₄-alkyl-, where the cycloalkyl moieties in the 4 last-mentioned radicals may carry a CN group; C₂-C₆-alkenyl, C₂-C₆-haloalkenyl, C₂-C₆-alkynyl and C₂-C₆-haloalkynyl;
- R^{14a}: is selected from hydrogen and C₁-C₆-alkyl; and
- R^{14b}: is selected from hydrogen, C₁-C₆-alkyl and C₁-C₆-haloalkyl.

In the invention, W is O. In the invention, B¹, B² and B³ are CR². More preferably, B¹ and B³ are CR², where R² is not hydrogen, and B² is CR², where R² has one of the meanings given above.

Preferably, R² is selected from hydrogen, halogen, cyano, azido, nitro, -SCN, -SF₅, C₁-C₆-alkyl, C₃-C₈-cycloalkyl, C₂-C₆-alkenyl, C₂-C₆-alkynyl, wherein the four last mentioned aliphatic and cycloaliphatic radicals may be partially or fully halogenated and/or may be substituted by one or more radicals R⁸; -OR⁹, -S(O)ₙR⁹ and -NR^{10a}R^{10b}, wherein n, R⁸, R⁹, R^{10a} and R^{10b} have one of the above general meanings, or, in particular, one of the below preferred meanings. According to the invention, R² is selected from hydrogen, halogen and C₁-C₂-haloalkyl, preferably from hydrogen, F, Cl, Br and CF₃.

In a particular embodiment, B¹ and B³ are C-Cl and B² is C-F. In a further particular embodiment, B¹ and B³ are C-CF₃ and B² is C-H. In a further particular embodiment B¹ and B³ are C-Br and B² is C-F. In a further particular embodiment, B¹, B² and B³ are C-Cl. In a further particular embodiment, B¹ is C-Cl, B² is C-H and B³ is C-CF₃. In a further particular embodiment, B¹ is C-Br, B² is C-H and B³ is C-CF₃. Specifically, B¹ and B³ are C-CI and B² is C-F.

Preferably, G¹, G², G³ and G⁴ are CR⁴, where R⁴ has one of the meanings given above or below. Likewise preferably, G¹, G³ and G⁴ are CR⁴ and G² is N, where R⁴ has one of the meanings given above or below. Likewise preferably, G², G³ and G⁴ are CR⁴ and G¹ is N, where R⁴ has one of the meanings given above or below.

According to the invention, G¹, G³ and G⁴ are CH and G² is CR⁴, where R⁴ has one of the above general or, in particular, one of the below preferred meanings.

Preferably, R⁴ is selected from hydrogen, halogen, cyano, C₁-C₄-alkyl, C₁-C₄-haloalkyl, C₃-C₈-cycloalkyl, C₃-C₅-halocycloalkyl, C₂-C₄-alkenyl, C₂-C₄-haloalkenyl, C₂-C₄-alkynyl, C₂-C₄-haloalkynyl, C₁-C₄-alkoxy, C₁-C₄-haloalkoxy, C₁-C₄-alkylthio and C₁-C₄-haloalkylthio; and in the invention, R⁴ is selected from hydrogen, halogen and methyl.

Preferably, R¹ is selected from C₁-C₄-alkyl, C₁-C₄-haloalkyl and -C(=O)OR¹⁵, and particularly preferably from C₁-C₄-haloalkyl and -C(=O)OR¹⁵, wherein R¹⁵ is preferably C₁-C₄-alkyl. In particular, R¹ is C₁-C₄-haloalkyl, specifically C₁-C₂-haloalkyl or -C(=O)OR¹⁵, wherein R¹⁵ is C₁-C₄-alkyl. More specifically R¹ is halomethyl, in particular fluoromethyl, such as fluoromethyl, difluoromethyl and trifluoromethyl.

In the invention, R¹ is trifluoromethyl.

Preferably, R^{3a} and R^{3b} are selected, independently of each other, from hydrogen, halogen, hydroxyl, C₁-C₃-alkyl, C₂-C₃-alkenyl, C₂-C₃-alkynyl, C₁-C₃-haloalkyl, C₁-C₃-alkoxy, C₁-C₃-alkylthio and C₁-C₃-alkylsulfonyl.

According to the invention, R^{3a} and R^{3b} are selected, independently of each other, from hydrogen and halogen, preferably hydrogen and fluorine and are in particular hydrogen.

If not specified otherwise above, R⁸, R⁹, R^{10a}, R^{10b}, R¹¹, R¹², R¹³, R¹⁵ and R¹⁶ have following preferred meanings:
In case R⁸ is a substituent on an alkyl, alkenyl or alkynyl group, it is preferably selected from the group consisting of cyano, C₃-C₈-cycloalkyl, C₃-C₈-halocycloalkyl, -OR⁹, -SR⁹, -C(=O)N(R^{10a})R^{10b}, -C(=S)N(R^{10a})R^{10b}, -C(=O)OR⁹, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R⁹, R^{10a}, R^{10b} and R¹⁶ have one of the meanings given above or in particular one of the preferred meanings given below.

In case R⁸ is a substituent on an alkyl, alkenyl or alkynyl group, it is even more preferably selected from the group consisting of cyano, C₃-C₆-cycloalkyl, C₃-C₆-halocycloalkyl, C₁-C₄-alkoxy, C₁-C₄-haloalkoxy, C₁-C₄-alkylthio, C₁-C₄-haloalkylthio,-C(=O)N(R^{10a})R^{10b}, -C(=S)N(R^{10a})R^{10b}, -C(=O)OR⁹, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R⁹, R^{10a}, R^{10b} and R¹⁶ have one of the meanings given above or in particular one of the preferred meanings given below. In particular it is selected from the group consisting of cyano, C₃-C₆-cycloalkyl, C₃-C₆-halocycloalkyl, -C(=O)N(R^{10a})R^{10b}, -C(=S)N(R^{10a})R^{10b},-C(=O)OR⁹, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R⁹, R^{10a}, R^{10b} and R¹⁶ have one of the meanings given above or in particular one of the preferred meanings given below.

In case R⁸ is a substituent on a cycloalkyl group, it is preferably selected from the group consisting of cyano, C₁-C₆-alkyl, C₁-C₆-haloalkyl, C₁-C₆-alkoxy-C₁-C₆-alkyl, -OR⁹, -OSO₂R⁹, -SR⁹, -N(R^{10a})R^{10b}, -C(=O)N(R^{10a})R^{10b}, -C(=S)N(R^{10a})R^{10b}, -C(=O)OR⁹, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R⁹, R^{10a}, R^{10b} and R¹⁶ have one of the meanings given above or in particular one of the preferred meanings given below.

In case R⁸ is a substituent on a cycloalkyl group, it is even more preferably selected from the group consisting of cyano, C₁-C₄-alkyl, C₁-C₃-haloalkyl, C₁-C₄-alkoxy and C₁-C₃-haloalkoxy. In particular, R⁸ as a substituent on a cycloalkyl group is selected from cyano, C₁-C₄-alkyl and C₁-C₃-haloalkyl.

In case of R⁸ in a group -C(=O)R⁸, =C(R⁸)₂ or -C(=NR⁶)R⁸, R⁸ is preferably selected from the group consisting of hydrogen, C₁-C₆-alkyl, C₁-C₆-haloalkyl, C₁-C₆-alkoxy-C₁-C₆-alkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, -OR⁹, -SR⁹, -N(R^{10a})R^{10b}, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R⁹, R^{10a}, R^{10b} and R¹⁶ have one of the meanings given above or in particular one of the preferred meanings given below.

In case of R⁸ in a group -C(=O)R⁸, =C(R⁸)₂ or -C(=NR⁶)R⁸, R⁸ is more preferably selected from the group consisting of C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, -N(R^{10a})R^{10b}, phenyl which may be substituted by 1, 2, 3, 4 or 5 radicals R¹⁶, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁶; where R^{10a}, R^{10b} and R¹⁶ have has one of the meanings given above or in particular one of the preferred meanings given below.

Preferably, each R⁹ is independently selected from the group consisting of hydrogen, C₁-C₆-alkyl, C₁-C₆-haloalkyl, C₃-C₈-cycloalkyl, C₃-C₈-halocycloalkyl, C₃-C₈-cycloalkyl-C₁-C₄-alkyl, phenyl which may be substituted by 1, 2, 3, 4 or 5 radicals R¹⁶; and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more, e.g. 1, 2, 3 or 4, preferably 1 or 2, more preferably 1, radicals R¹⁶, where R¹⁶ has one of the meanings given above or in particular one of the preferred meanings given below.

More preferably, each R⁹ is independently selected from the group consisting of hydrogen, C₁-C₆-alkyl, C₁-C₆-haloalkyl, phenyl which may be substituted by 1, 2, 3, 4 or 5 radicals R¹⁶; and a 5- or 6-membered heteroaromatic ring containing 1, 2 or 3 heteroatoms selected from N, O and S, as ring members, where the heteroaromatic ring may be substituted by one or more radicals R¹⁶; where R¹⁶ has one of the meanings given above or in particular one of the preferred meanings given below.

R^{10a} and R^{10b} are, independently of each other, preferably selected from hydrogen, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkylcarbonyl, C₁-C₄-haloalkylcarbonyl, C₁-C₄-alkylaminocarbonyl, C₁-C₄-haloalkylaminocarbonyl, C₃-C₆-cycloalkylaminocarbonyl, C₃-C₆-halocycloalkylaminocarbonyl, and a 3-, 4-, 5-, 6- or 7-membered saturated, partially unsaturated or maximally unsaturated heterocyclic ring comprising 1, 2 or 3 heteroatoms or heteroatom groups selected from N, O, S, NO, SO and SO₂, as ring members, where the heterocyclic ring is optionally substituted with one or more, preferably 1, 2 or 3, in particular 1, substituents selected from halogen, CN, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkoxy, C₁-C₄-haloalkoxy, C₁-C₄-alkylthio and C₁-C₄-haloalkylthio;
or, R^{10a} and R^{10b}, together with the nitrogen atom to which they are bound, form a 5- or 6-membered saturated, partially unsaturated or aromatic heterocyclic ring, which additionally may contain 1 or 2 further heteroatoms or heteroatom groups selected from N, O, S, NO, SO and SO₂, as ring members, where the heterocyclic ring may carry 1 or 2, in particular 1, substituents selected from halogen, CN, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, C₁-C₄-alkoxy, C₁-C₄-haloalkoxy, C₁-C₄-alkylthio and C₁-C₄-haloalkylthio.

More preferably, R^{10a} and R^{10b} are, independently of each other, selected from hydrogen, C₁-C₄-alkyl, C₁-C₄-haloalkyl, C₃-C₆-cycloalkyl, C₃-C₆-halocycloalkyl, and a 3-or 4-membered saturated heterocyclic ring comprising 1 heteroatom or heteroatom group selected from N, O, S, NO, SO and SO₂, as ring member, where the heterocyclic ring is optionally substituted with one or more, preferably 1, 2 or 3, in particular 1, substituents selected from halogen, CN, C₁-C₄-alkyl, C₁-C₄-haloalkyl, C₁-C₄-alkoxy and C₁-C₄-haloalkoxy; and are specifically, independently of each other, selected from hydrogen, C₁-C₄-alkyl and C₁-C₄-haloalkyl.

Each R¹¹ and each R¹⁶ are independently of each occurrence and independently of each other preferably selected from halogen, CN, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl and C₁-C₄-haloalkylsulfonyl, and more preferably from halogen, CN, C₁-C₄-alkyl, C₁-C₄-haloalkyl, C₁-C₄-alkoxy and C₁-C₄-haloalkoxy.

Each R¹² is preferably selected from C₁-C₄-alkyl and is in particular methyl.

In case R¹³ is a substituent on an alkyl, alkenyl or alkynyl group, it is preferably selected from the group consisting of cyano, C₃-C₈-cycloalkyl, C₃-C₈-halocycloalkyl,-OH, -SH, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl, C₁-C₄-haloalkylsulfonyl and phenyl which may be substituted by 1, 2 or 3 radicals selected from halogen, C₁-C₄-alkyl, C₁-C₄-haloalkyl, C₁-C₄-alkoxy and C₁-C₄-haloalkoxy.

In case R¹³ is a substituent on a cycloalkyl group, it is preferably selected from the group consisting of cyano, C₁-C₆-alkyl, C₁-C₆-haloalkyl, C₃-C₈-cycloalkyl, C₃-C₈-halocycloalkyl, -OH, -SH, C₁-C₄-alkoxy, C₁-C₄-haloalkoxy, C₁-C₄-alkylthio, C₁-C₄-haloalkylthio, C₁-C₄-alkylsulfinyl, C₁-C₄-haloalkylsulfinyl, C₁-C₄-alkylsulfonyl, C₁-C₄-haloalkylsulfonyl and phenyl which may be substituted by 1, 2 or 3 radicals selected from halogen, C₁-C₄-alkyl, C₁-C₄-haloalkyl, C₁-C₄-alkoxy and C₁-C₄-haloalkoxy.

In case R¹³ is a substituent on a cycloalkyl group, it is even more preferably selected from the group consisting of halogen, C₁-C₄-alkyl, C₁-C₃-haloalkyl, C₁-C₄-alkoxy and C₁-C₃-haloalkoxy. In particular, R¹³ as a substituent on a cycloalkyl group is selected from halogen, C₁-C₄-alkyl and C₁-C₃-haloalkyl.

In case of R¹³ in a group -C(=O)R¹³, -C(=S)R¹³, =C(R¹³)₂ or -C(=NR¹⁴)R¹³, R⁸ is preferably selected from the group consisting of hydrogen, C₁-C₆-alkyl, C₁-C₆-haloalkyl, C₃-C₈-cycloalkyl, C₃-C₈-halocycloalkyl, -OH, -SH, C₁-C₆-alkoxy, C₁-C₆-haloalkoxy and phenyl which may be substituted by 1, 2 or 3 radicals selected from halogen, C₁-C₄-alkyl, C₁-C₄-haloalkyl, C₁-C₄-alkoxy and C₁-C₄-haloalkoxy.

R¹⁴, R^{14a} and R^{14b} are, independently of each other, preferably selected from hydrogen, C₁-C₄-alkyl, C₁-C₄-haloalkyl, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl and benzyl, where the phenyl ring in benzyl is optionally substituted 1, 2 or 3, in particular 1, substituents selected from halogen, C₁-C₄-alkyl, C₁-C₄-haloalkyl, C₁-C₄-alkoxy and C₁-C₄-haloalkoxy;
or, R^{14a} and R^{14b}, together with the nitrogen atom to which they are bound, form a 5- or 6-membered saturated, partially unsaturated or aromatic heterocyclic ring, which additionally may contain 1 or 2 further heteroatoms or heteroatom groups selected from N, O, S, NO, SO and SO₂, as ring members, where the heterocyclic ring may carry 1 or 2, in particular 1, substituents selected from halogen, C₁-C₄-alkyl, C₁-C₄-haloalkyl, C₁-C₄-alkoxy and C₁-C₄-haloalkoxy.

More preferably, R¹⁴, R^{14a} and R^{14b} are, independently of each other, selected from hydrogen, C₁-C₄-alkyl, C₁-C₄-haloalkyl, C₃-C₆-cycloalkyl, C₃-C₆-halocycloalkyl and benzyl, where the phenyl ring in benzyl is optionally substituted 1, 2 or 3, in particular 1, substituents selected from halogen, C₁-C₄-alkyl, C₁-C₄-haloalkyl, C₁-C₄-alkoxy and C₁-C₄-haloalkoxy;
or,
R^{14a} and R^{14b}, together with the nitrogen atom to which they are bound, form a 5- or 6-membered saturated, partially unsaturated or aromatic heterocyclic ring, which additionally may contain 1 or 2 further heteroatoms or heteroatom groups selected from N, O, S, NO, SO and SO₂, as ring members, where the heterocyclic ring may carry 1 or 2, in particular 1, substituents selected from halogen, C₁-C₄-alkyl, C₁-C₄-haloalkyl, C₁-C₄-alkoxy and C₁-C₄-haloalkoxy.

Each R¹⁵ is preferably selected from hydrogen, C₁-C₆-alkyl, C₁-C₆-haloalkyl, phenyl, benzyl, pyridyl and phenoxy, wherein the four last-mentioned radicals may be unsubstituted and/or carry 1, 2 or 3 substituents selected from C₁-C₆-alkyl, C₁-C₆-haloalkyl, C₁-C₆-alkoxy and C₁-C₆-haloalkoxy.

In a particular aspect of the description, compound I is a compound of formula IA, i.e. a compound of formula I, wherein B¹ is CR^{2a}, B² is CR^{2b}, B³ is CR^{2c}, R¹ is CF₃, R^{3a} and R^{3b} are each hydrogen, G¹ is C-H, G³ is C-H, G⁴ is C-H and G² is C-R⁴, 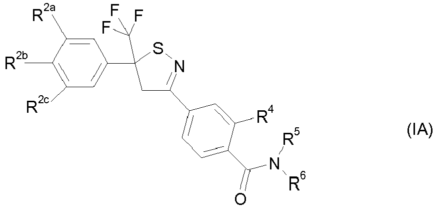 where R⁴, R⁵ and R⁶ have one of the above-given general or, in particular, one of the above-given preferred meanings, and R^{2a}, R^{2b} and R^{2c}, independently of each other, have one of the general or, in particular, one of the preferred meanings given above for R², except for compounds IA wherein R^{2a} and R^{2c} are Cl, and simultaneously R^{2b} is H, R⁴ is F, Cl, CH₃ or SCH₃, and R⁵ and R⁶, together with the nitrogen atom they are bound to, form a ring selected from unsubstituted aziridin-1-yl, unsubstituted azetidin-1-yl, unsubstituted pyrrolidin-1-yl, unsubstituted piperidin-1-yl, unsubstituted thiazolidin-3-yl, unsubstituted morpholin-4-yl, unsubstituted thiomorpholin-4-yl, unsubstituted 1-oxo-1,4-thiazinan-4-yl and unsubstituted 1,1-dioxo-1,4-thiazinan-4-yl.

Examples of preferred compounds of the description are compounds of the following formulae Ia.1 to Ia.52, where the variables have one of the general or preferred meanings given above.

Examples of preferred compounds of the description are the individual compounds compiled in the tables 1 to 265 below. Moreover, the meanings mentioned below for the individual variables in the tables are per se, independently of the combination in which they are mentioned, a particularly preferred embodiment of the substituents in question.

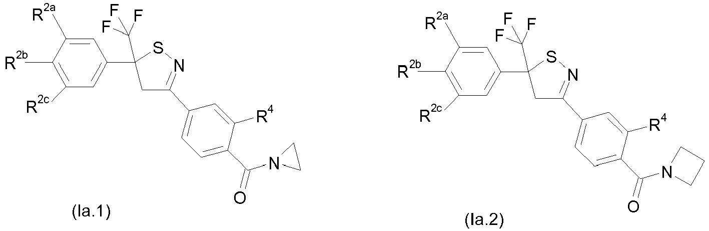

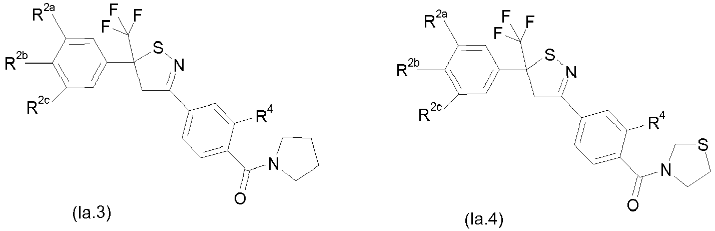

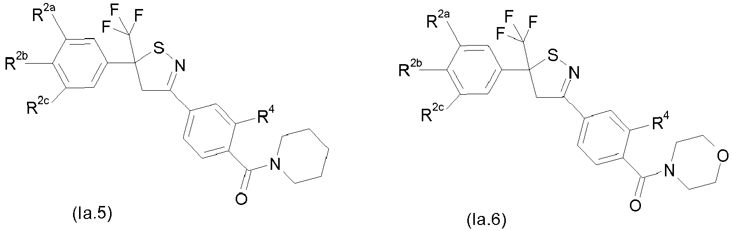

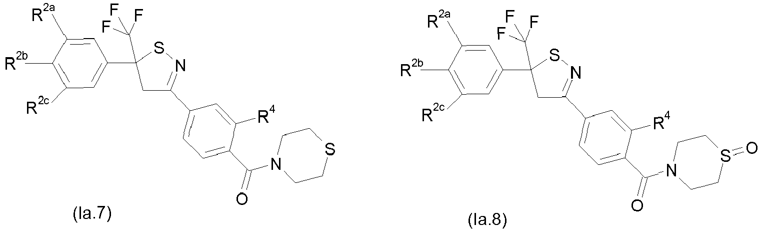

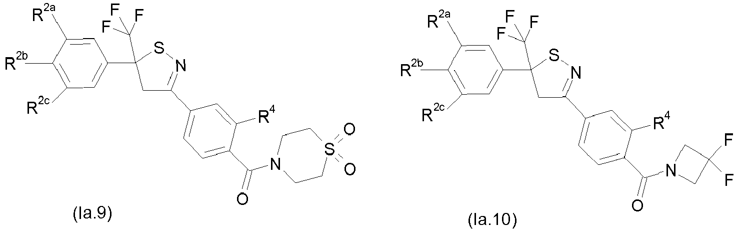

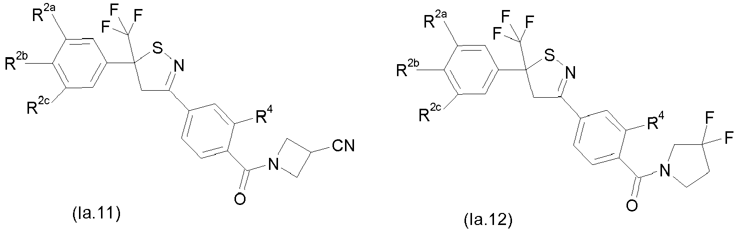

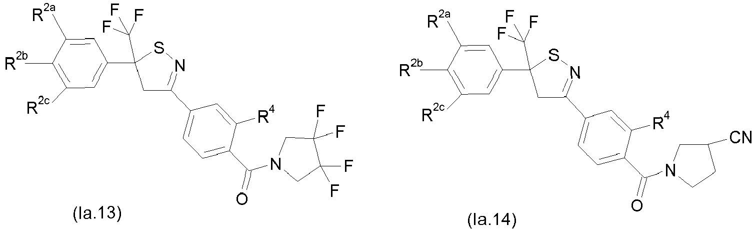

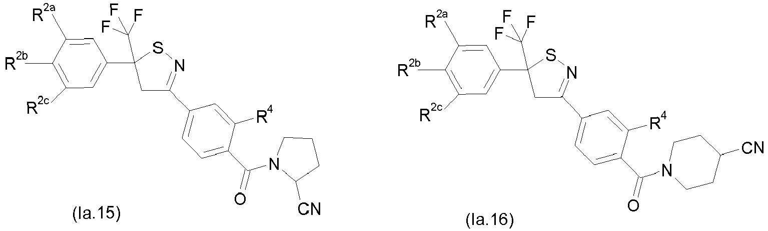

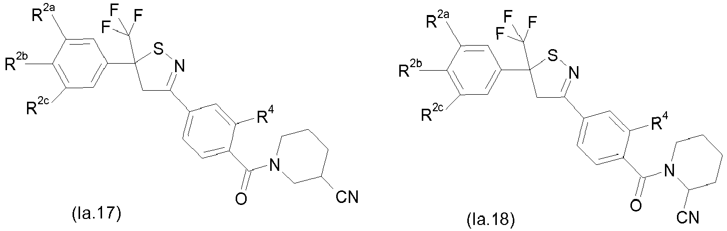

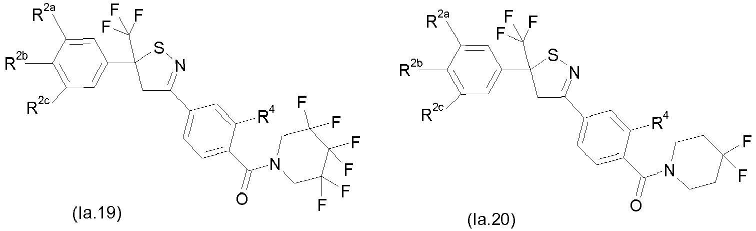

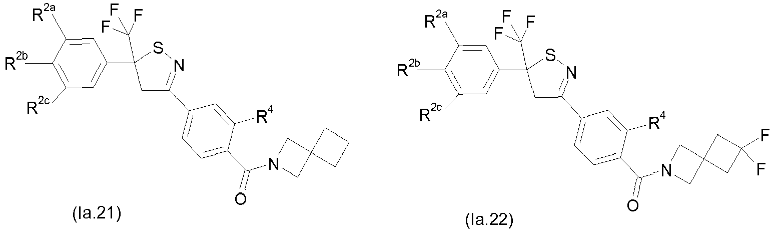

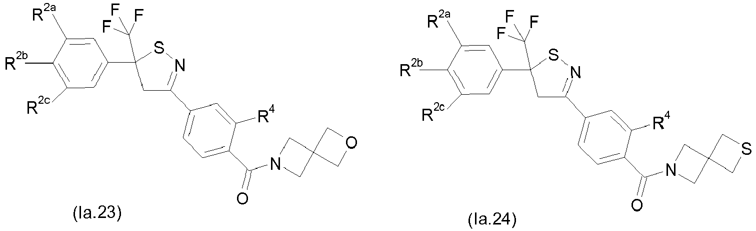

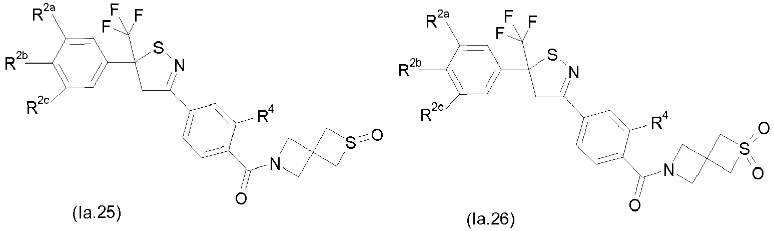

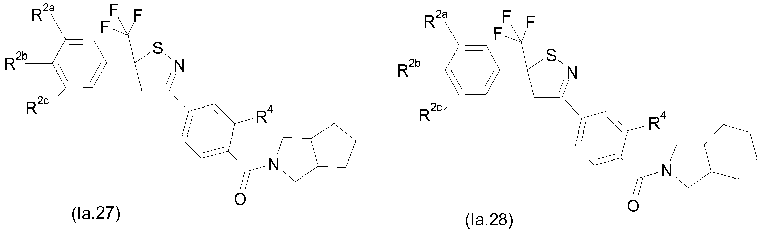

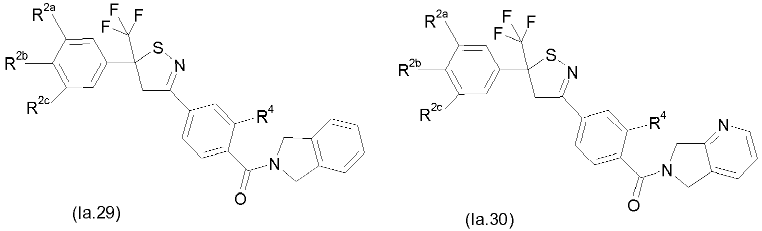

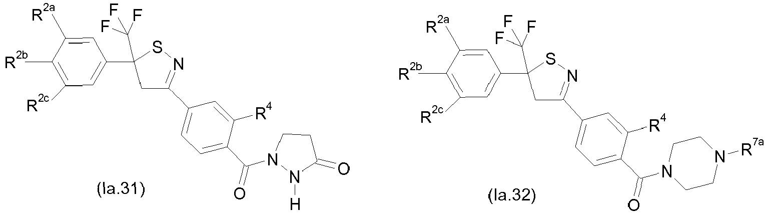

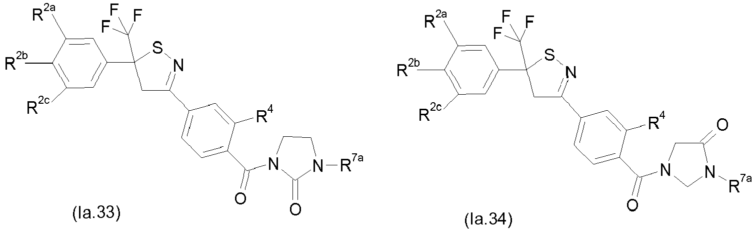

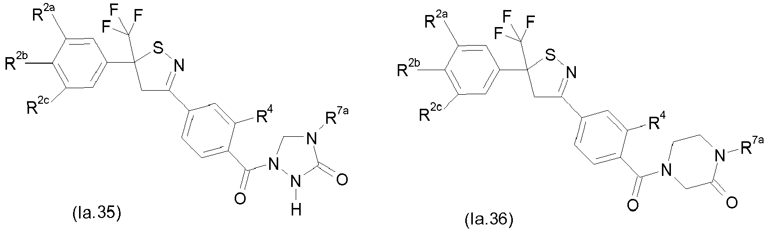

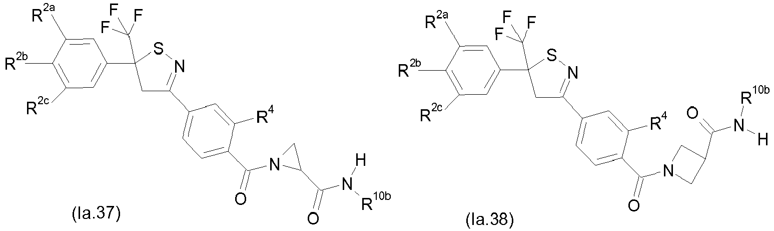

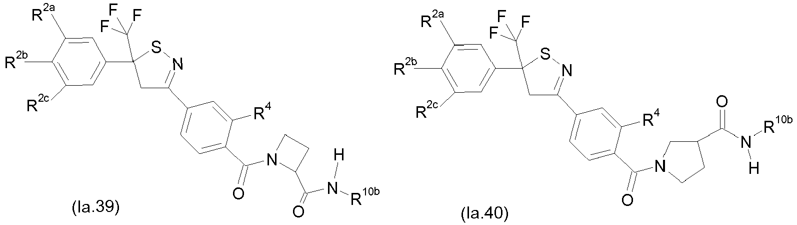

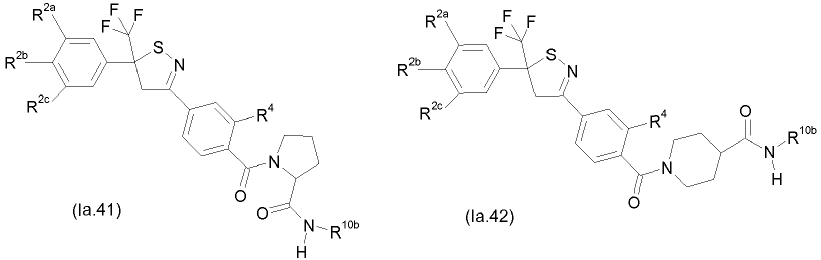

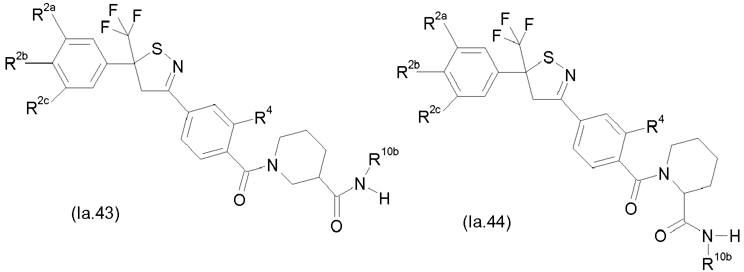

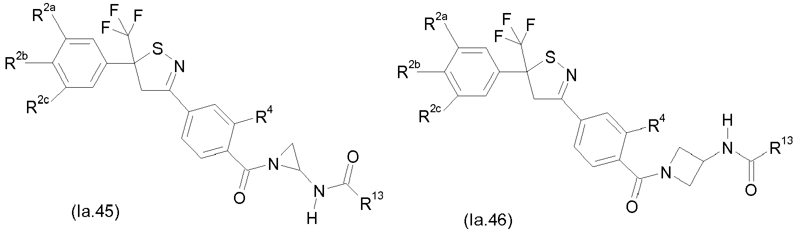

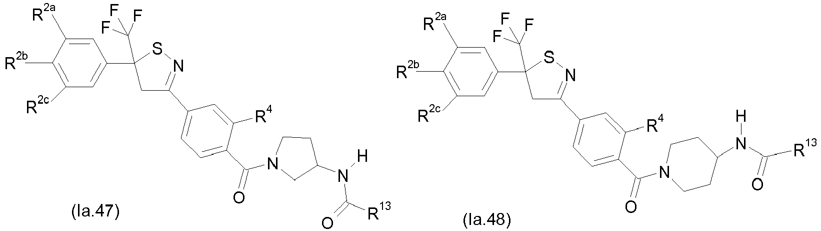

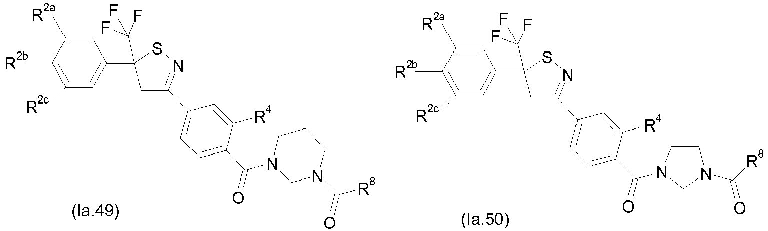

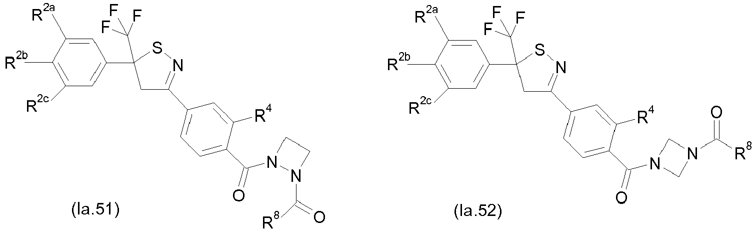

### Table 1

Compounds of the formula Ia.1, in which the combination of R^{2a}, R^{2b}, R^{2c} and R⁴ for a compound corresponds in each case to one row of Table A, except of rows A-37, A-307, A-337 and A-667.

### Table 2

Compounds of the formula la.2 in which the combination of R^{2a}, R^{2b}, R^{2c} and R⁴ for a compound corresponds in each case to one row of Table A, except of rows except of rows A-37, A-307, A-337 and A-667.

### Table 3

Compounds of the formula Ia.3 in which the combination of R^{2a}, R^{2b}, R^{2c} and R⁴ for a compound corresponds in each case to one row of Table A, except of rows except of rows A-37, A-307, A-337 and A-667.

### Table 4

Compounds of the formula Ia.4 in which the combination of R^{2a}, R^{2b}, R^{2c} and R⁴ for a compound corresponds in each case to one row of Table A, except of rows except of rows A-37, A-307, A-337 and A-667.

### Table 5

Compounds of the formula Ia.5 in which the combination of R^{2a}, R^{2b}, R^{2c} and R⁴ for a compound corresponds in each case to one row of Table A, except of rows except of rows A-37, A-307, A-337 and A-667.

### Table 6

Compounds of the formula Ia.6 in which the combination of R^{2a}, R^{2b}, R^{2c} and R⁴ for a compound corresponds in each case to one row of Table A, except of rows except of rows A-37, A-307, A-337 and A-667.

### Table 7

Compounds of the formula Ia.7 in which the combination of R^{2a}, R^{2b}, R^{2c} and R⁴ for a compound corresponds in each case to one row of Table A, except of rows except of rows A-37, A-307, A-337 and A-667.

### Table 8

Compounds of the formula Ia.8 in which the combination of R^{2a}, R^{2b}, R^{2c} and R⁴ for a compound corresponds in each case to one row of Table A, except of rows except of rows except of rows A-37, A-307, A-337 and A-667.

### Table 9

Compounds of the formula Ia.9 in which the combination of R^{2a}, R^{2b}, R^{2c} and R⁴ for a compound corresponds in each case to one row of Table A, except of rows except of rows except of rows A-37, A-307, A-337 and A-667.

### Table 10

Compounds of the formula Ia.10 in which the combination of R^{2a}, R^{2b}, R^{2c} and R⁴ for a compound corresponds in each case to one row of Table A.

### Table 11

Compounds of the formula Ia.11 in which the combination of R^{2a}, R^{2b}, R^{2c} and R⁴ for a compound corresponds in each case to one row of Table A.

### Table 12

Compounds of the formula Ia.12 in which the combination of R^{2a}, R^{2b}, R^{2c} and R⁴ for a compound corresponds in each case to one row of Table A.

### Table 13

Compounds of the formula Ia.13 in which the combination of R^{2a}, R^{2b}, R^{2c} and R⁴ for a compound corresponds in each case to one row of Table A.

### Table 14

Compounds of the formula Ia.14 in which the combination of R^{2a}, R^{2b}, R^{2c} and R⁴ for a compound corresponds in each case to one row of Table A.

### Table 15

Compounds of the formula Ia.15 in which the combination of R^{2a}, R^{2b}, R^{2c} and R⁴ for a compound corresponds in each case to one row of Table A.

### Table 16

Compounds of the formula Ia.16 in which the combination of R^{2a}, R^{2b}, R^{2c} and R⁴ for a compound corresponds in each case to one row of Table A.

### Table 17

Compounds of the formula Ia.17 in which the combination of R^{2a}, R^{2b}, R^{2c} and R⁴ for a compound corresponds in each case to one row of Table A.

### Table 18

Compounds of the formula Ia.18 in which the combination of R^{2a}, R^{2b}, R^{2c} and R⁴ for a compound corresponds in each case to one row of Table A.

### Table 19

Compounds of the formula la.19 in which the combination of R^{2a}, R^{2b}, R^{2c} and R⁴ for a compound corresponds in each case to one row of Table A.

### Table 20

Compounds of the formula Ia.20 in which the combination of R^{2a}, R^{2b}, R^{2c} and R⁴ for a compound corresponds in each case to one row of Table A.

### Table 21

Compounds of the formula Ia.21 in which the combination of R^{2a}, R^{2b}, R^{2c} and R⁴ for a compound corresponds in each case to one row of Table A.

### Table 22

Compounds of the formula Ia.22 in which the combination of R^{2a}, R^{2b}, R^{2c} and R⁴ for a compound corresponds in each case to one row of Table A.

### Table 23

Compounds of the formula Ia.23 in which the combination of R^{2a}, R^{2b}, R^{2c} and R⁴ for a compound corresponds in each case to one row of Table A.

### Table 24

Compounds of the formula Ia.24 in which the combination of R^{2a}, R^{2b}, R^{2c} and R⁴ for a compound corresponds in each case to one row of Table A.

### Table 25

Compounds of the formula Ia.25 in which the combination of R^{2a}, R^{2b}, R^{2c} and R⁴ for a compound corresponds in each case to one row of Table A.

### Table 26

Compounds of the formula Ia.26 in which the combination of R^{2a}, R^{2b}, R^{2c} and R⁴ for a compound corresponds in each case to one row of Table A.

### Table 27

Compounds of the formula Ia.27 in which the combination of R^{2a}, R^{2b}, R^{2c} and R⁴ for a compound corresponds in each case to one row of Table A.

### Table 28

Compounds of the formula Ia.28 in which the combination of R^{2a}, R^{2b}, R^{2c} and R⁴ for a compound corresponds in each case to one row of Table A.

### Table 29

Compounds of the formula Ia.29 in which the combination of R^{2a}, R^{2b}, R^{2c} and R⁴ for a compound corresponds in each case to one row of Table A.

### Table 30

Compounds of the formula Ia.30 in which the combination of R^{2a}, R^{2b}, R^{2c} and R⁴ for a compound corresponds in each case to one row of Table A.

### Table 31

Compounds of the formula Ia.31 in which the combination of R^{2a}, R^{2b}, R^{2c} and R⁴ for a compound corresponds in each case to one row of Table A.

### Table 32

Compounds of the formula Ia.32 in which R^{7a} is hydrogen and the combination of R^{2a}, R^{2b}, R^{2c} and R⁴ for a compound corresponds in each case to one row of Table A.

### Table 33

Compounds of the formula Ia.32 in which R^{7a} is methyl and the combination of R^{2a}, R^{2b}, R^{2c} and R⁴ for a compound corresponds in each case to one row of Table A.

### Table 34

Compounds of the formula Ia.32 in which R^{7a} is ethyl and the combination of R^{2a}, R^{2b}, R^{2c} and R⁴ for a compound corresponds in each case to one row of Table A.

### Table 35

Compounds of the formula Ia.32 in which R^{7a} is isopropyl (-CH(CH₃)₂) and the combination of R^{2a}, R^{2b}, R^{2c} and R⁴ for a compound corresponds in each case to one row of Table A.

### Table 36

Compounds of the formula Ia.32 in which R^{7a} is 2,2-difluoroethyl and the combination of R^{2a}, R^{2b}, R^{2c} and R⁴ for a compound corresponds in each case to one row of Table A.

### Table 37

Compounds of the formula Ia.32 in which R^{7a} is 2,2,2-trifluoroethyl and the combination of R^{2a}, R^{2b}, R^{2c} and R⁴ for a compound corresponds in each case to one row of Table A.

### Table 38

Compounds of the formula Ia.32 in which R^{7a} is cyclopropyl and the combination of R^{2a}, R^{2b}, R^{2c} and R⁴ for a compound corresponds in each case to one row of Table A.

### Table 39

Compounds of the formula Ia.32 in which R^{7a} is cyclopropylmethyl- and the combination of R^{2a}, R^{2b}, R^{2c} and R⁴ for a compound corresponds in each case to one row of Table A.

### Table 40

Compounds of the formula Ia.32 in which R^{7a} is allyl and the combination of R^{2a}, R^{2b}, R^{2c} and R⁴ for a compound corresponds in each case to one row of Table A.

### Table 41

Compounds of the formula Ia.32 in which R^{7a} is prop-2-ynyl (propargyl) and the combination of R^{2a}, R^{2b}, R^{2c} and R⁴ for a compound corresponds in each case to one row of Table A.

### Tables 42 to 51

Compounds of the formula Ia.33 in which R^{7a} is as defined in any of tables 32 to 41 and the combination of R^{2a}, R^{2b}, R^{2c} and R⁴ for a compound corresponds in each case to one row of Table A.

### Tables 52 to 61

Compounds of the formula Ia.34 in which R^{7a} is as defined in any of tables 32 to 41 and the combination of R^{2a}, R^{2b}, R^{2c} and R⁴ for a compound corresponds in each case to one row of Table A.

### Tables 62 to 71

Compounds of the formula Ia.35 in which R^{7a} is as defined in any of tables 32 to 41 and the combination of R^{2a}, R^{2b}, R^{2c} and R⁴ for a compound corresponds in each case to one row of Table A.

### Tables 72 to 81

Compounds of the formula Ia.36 in which R^{7a} is as defined in any of tables 32 to 41 and the combination of R^{2a}, R^{2b}, R^{2c} and R⁴ for a compound corresponds in each case to one row of Table A.

### Tables 82 to 91

Compounds of the formula Ia.37 in which R^{10b} is as defined for R^{7a} in any of tables 32 to 41 and the combination of R^{2a}, R^{2b}, R^{2c} and R⁴ for a compound corresponds in each case to one row of Table A.

### Tables 92 to 101

Compounds of the formula Ia.38 in which R^{10b} is as defined for R^{7a} in any of tables 32 to 41 and the combination of R^{2a}, R^{2b}, R^{2c} and R⁴ for a compound corresponds in each case to one row of Table A.

### Tables 102 to 111

Compounds of the formula Ia.39 in which R^{10b} is as defined for R^{7a} in any of tables 32 to 41 and the combination of R^{2a}, R^{2b}, R^{2c} and R⁴ for a compound corresponds in each case to one row of Table A.

### Tables 112 to 121

Compounds of the formula Ia.40 in which R^{10b} is as defined for R^{7a} in any of tables 32 to 41 and the combination of R^{2a}, R^{2b}, R^{2c} and R⁴ for a compound corresponds in each case to one row of Table A.

### Tables 122 to 131

Compounds of the formula Ia.41 in which R^{10b} is as defined for R^{7a} in any of tables 32 to 41 and the combination of R^{2a}, R^{2b}, R^{2c} and R⁴ for a compound corresponds in each case to one row of Table A.

### Tables 132 to 141

Compounds of the formula Ia.42 in which R^{10b} is as defined for R^{7a} in any of tables 32 to 41 and the combination of R^{2a}, R^{2b}, R^{2c} and R⁴ for a compound corresponds in each case to one row of Table A.

### Tables 142 to 151

Compounds of the formula Ia.43 in which R^{10b} is as defined for R^{7a} in any of tables 32 to 41 and the combination of R^{2a}, R^{2b}, R^{2c} and R⁴ for a compound corresponds in each case to one row of Table A.

### Tables 152 to 161

Compounds of the formula Ia.44 in which R^{10b} is as defined for R^{7a} in any of tables 32 to 41 and the combination of R^{2a}, R^{2b}, R^{2c} and R⁴ for a compound corresponds in each case to one row of Table A.

### Tables 162 to 171

Compounds of the formula Ia.45 in which R¹³ is as defined for R^{7a} in any of tables 32 to 41 and the combination of R^{2a}, R^{2b}, R^{2c} and R⁴ for a compound corresponds in each case to one row of Table A.

### Table 172

Compounds of the formula Ia.45 in which R¹³ is CF₃ and the combination of R^{2a}, R^{2b}, R^{2c} and R⁴ for a compound corresponds in each case to one row of Table A.

### Table 173

Compounds of the formula Ia.45 in which R¹³ is vinyl and the combination of R^{2a}, R^{2b}, R^{2c} and R⁴ for a compound corresponds in each case to one row of Table A.

### Table 174

Compounds of the formula Ia.45 in which R¹³ is ethynyl and the combination of R^{2a}, R^{2b}, R^{2c} and R⁴ for a compound corresponds in each case to one row of Table A.

### Table 175

Compounds of the formula Ia.45 in which R¹³ is -NH(CH₃) and the combination of R^{2a}, R^{2b}, R^{2c} and R⁴ for a compound corresponds in each case to one row of Table A.

### Table 176

Compounds of the formula la.45 in which R¹³ is -NH(CH₂CH₃) and the combination of R^{2a}, R^{2b}, R^{2c} and R⁴ for a compound corresponds in each case to one row of Table A.

### Table 177

Compounds of the formula Ia.45 in which R¹³ is -NH(CH₂CF₃) and the combination of R^{2a}, R^{2b}, R^{2c} and R⁴ for a compound corresponds in each case to one row of Table A.

### Tables 178 to 193

Compounds of the formula la.46 in which R¹³ is as defined in any of tables 162 to 177 and the combination of R^{2a}, R^{2b}, R^{2c} and R⁴ for a compound corresponds in each case to one row of Table A.

### Tables 194 to 209

Compounds of the formula Ia.47 in which R¹³ is as defined in any of tables 162 to 177 and the combination of R^{2a}, R^{2b}, R^{2c} and R⁴ for a compound corresponds in each case to one row of Table A.

### Tables 210 to 225

Compounds of the formula Ia.48 in which R¹³ is as defined in any of tables 162 to 177 and the combination of R^{2a}, R^{2b}, R^{2c} and R⁴ for a compound corresponds in each case to one row of Table A.

### Tables 226 to 235

Compounds of the formula Ia.49 in which R⁸ is as defined for R^{7a} in any of tables 32 to 41 and the combination of R^{2a}, R^{2b}, R^{2c} and R⁴ for a compound corresponds in each case to one row of Table A.

### Tables 236 to 245

Compounds of the formula Ia.50 in which R⁸ is as defined for R^{7a} in any of tables 32 to 41 and the combination of R^{2a}, R^{2b}, R^{2c} and R⁴ for a compound corresponds in each case to one row of Table A.

### Tables 246 to 255

Compounds of the formula Ia.51 in which R⁸ is as defined for R^{7a} in any of tables 32 to 41 and the combination of R^{2a}, R^{2b}, R^{2c} and R⁴ for a compound corresponds in each case to one row of Table A.

### Tables 256 to 265

Compounds of the formula Ia.52 in which R⁸ is as defined for R^{7a} in any of tables 32 to 41 and the combination of R^{2a}, R^{2b}, R^{2c} and R⁴ for a compound corresponds in each case to one row of Table A.

**Table A**

| No. | R^{2a} | R^{2b} | R^{2c} | R⁴ |
|---|---|---|---|---|
| A-1 | F | H | F | H |
| A-2 | F | F | F | H |
| A-3 | F | Cl | F | H |
| A-4 | F | Br | F | H |
| A-5 | F | H | Cl | H |
| A-6 | F | H | Br | H |
| A-7 | Cl | H | Cl | H |
| A-8 | Cl | Cl | Cl | H |
| A-9 | Cl | F | Cl | H |
| A-10 | Cl | Br | Cl | H |
| A-11 | Cl | H | Br | H |
| A-12 | Br | H | Br | H |
| A-13 | Br | F | Br | H |
| A-14 | Br | Cl | Br | H |
| A-15 | CF₃ | H | F | H |
| A-16 | CF₃ | H | Cl | H |
| A-17 | CF₃ | H | Br | H |
| A-18 | CF₃ | H | CF₃ | H |
| A-19 | CF₃ | F | F | H |
| A-20 | CF₃ | Cl | Cl | H |
| A-21 | CF₃ | Br | Br | H |
| A-22 | SF₅ | H | F | H |
| A-23 | SF₅ | H | Cl | H |
| A-24 | SF₅ | H | Br | H |
| A-25 | SF₅ | H | CF₃ | H |
| A-26 | SF₅ | H | H | H |
| A-27 | CF₃ | H | H | H |
| A-28 | Br | H | H | H |
| A-29 | Cl | H | H | H |
| A-30 | F | H | H | H |
| A-31 | F | H | F | CH₃ |
| A-32 | F | F | F | CH₃ |
| A-33 | F | Cl | F | CH₃ |
| A-34 | F | Br | F | CH₃ |
| A-35 | F | H | Cl | CH₃ |
| A-36 | F | H | Br | CH₃ |
| A-37 | Cl | H | Cl | CH₃ |
| A-38 | Cl | Cl | Cl | CH₃ |
| A-39 | Cl | F | Cl | CH₃ |
| A-40 | Cl | Br | Cl | CH₃ |
| A-41 | Cl | H | Br | CH₃ |
| A-42 | Br | H | Br | CH₃ |
| A-43 | Br | F | Br | CH₃ |
| A-44 | Br | Cl | Br | CH₃ |
| A-45 | CF₃ | H | F | CH₃ |
| A-46 | CF₃ | H | Cl | CH₃ |
| A-47 | CF₃ | H | Br | CH₃ |
| A-48 | CF₃ | H | CF₃ | CH₃ |
| A-49 | CF₃ | F | F | CH₃ |
| A-50 | CF₃ | Cl | Cl | CH₃ |
| A-51 | CF₃ | Br | Br | CH₃ |
| A-52 | SF₅ | H | F | CH₃ |
| A-53 | SF₅ | H | Cl | CH₃ |
| A-54 | SF₅ | H | Br | CH₃ |
| A-55 | SF₅ | H | CF₃ | CH₃ |
| A-56 | SF₅ | H | H | CH₃ |
| A-57 | CF₃ | H | H | CH₃ |
| A-58 | Br | H | H | CH₃ |
| A-59 | Cl | H | H | CH₃ |
| A-60 | F | H | H | CH₃ |
| A-61 | F | H | F | CH₂CH₃ |
| A-62 | F | F | F | CH₂CH₃ |
| A-63 | F | Cl | F | CH₂CH₃ |
| A-64 | F | Br | F | CH₂CH₃ |
| A-65 | F | H | Cl | CH₂CH₃ |
| A-66 | F | H | Br | CH₂CH₃ |
| A-67 | Cl | H | Cl | CH₂CH₃ |
| A-68 | Cl | Cl | Cl | CH₂CH₃ |
| A-69 | Cl | F | Cl | CH₂CH₃ |
| A-70 | Cl | Br | Cl | CH₂CH₃ |
| A-71 | Cl | H | Br | CH₂CH₃ |
| A-72 | Br | H | Br | CH₂CH₃ |
| A-73 | Br | F | Br | CH₂CH₃ |
| A-74 | Br | Cl | Br | CH₂CH₃ |
| A-75 | CF₃ | H | F | CH₂CH₃ |
| A-76 | CF₃ | H | Cl | CH₂CH₃ |
| A-77 | CF₃ | H | Br | CH₂CH₃ |
| A-78 | CF₃ | H | CF₃ | CH₂CH₃ |
| A-79 | CF₃ | F | F | CH₂CH₃ |
| A-80 | CF₃ | Cl | Cl | CH₂CH₃ |
| A-81 | CF₃ | Br | Br | CH₂CH₃ |
| A-82 | SF₅ | H | F | CH₂CH₃ |
| A-83 | SF₅ | H | Cl | CH₂CH₃ |
| A-84 | SF₅ | H | Br | CH₂CH₃ |
| A-85 | SF₅ | H | CF₃ | CH₂CH₃ |
| A-86 | SF₅ | H | H | CH₂CH₃ |
| A-87 | CF₃ | H | H | CH₂CH₃ |
| A-88 | Br | H | H | CH₂CH₃ |
| A-89 | Cl | H | H | CH₂CH₃ |
| A-90 | F | H | H | CH₂CH₃ |
| A-91 | F | H | F | CH(CH₃)₂ |
| A-92 | F | F | F | CH(CH₃)₂ |
| A-93 | F | Cl | F | CH(CH₃)₂ |
| A-94 | F | Br | F | CH(CH₃)₂ |
| A-95 | F | H | Cl | CH(CH₃)₂ |
| A-96 | F | H | Br | CH(CH₃)₂ |
| A-97 | Cl | H | Cl | CH(CH₃)₂ |
| A-98 | Cl | Cl | Cl | CH(CH₃)₂ |
| A-99 | Cl | F | Cl | CH(CH₃)₂ |
| A-100 | Cl | Br | Cl | CH(CH₃)₂ |
| A-101 | Cl | H | Br | CH(CH₃)₂ |
| A-102 | Br | H | Br | CH(CH₃)₂ |
| A-103 | Br | F | Br | CH(CH₃)₂ |
| A-104 | Br | Cl | Br | CH(CH₃)₂ |
| A-105 | CF₃ | H | F | CH(CH₃)₂ |
| A-106 | CF₃ | H | Cl | CH(CH₃)₂ |
| A-107 | CF₃ | H | Br | CH(CH₃)₂ |
| A-108 | CF₃ | H | CF₃ | CH(CH₃)₂ |
| A-109 | CF₃ | F | F | CH(CH₃)₂ |
| A-110 | CF₃ | Cl | Cl | CH(CH₃)₂ |
| A-111 | CF₃ | Br | Br | CH(CH₃)₂ |
| A-112 | SF₅ | H | F | CH(CH₃)₂ |
| A-113 | SF₅ | H | Cl | CH(CH₃)₂ |
| A-114 | SF₅ | H | Br | CH(CH₃)₂ |
| A-115 | SF₅ | H | CF₃ | CH(CH₃)₂ |
| A-116 | SF₅ | H | H | CH(CH₃)₂ |
| A-117 | CF₃ | H | H | CH(CH₃)₂ |
| A-118 | Br | H | H | CH(CH₃)₂ |
| A-119 | Cl | H | H | CH(CH₃)₂ |
| A-120 | F | H | H | CH(CH₃)₂ |
| A-121 | F | H | F | CHF₂ |
| A-122 | F | F | F | CHF₂ |
| A-123 | F | Cl | F | CHF₂ |
| A-124 | F | Br | F | CHF₂ |
| A-125 | F | H | Cl | CHF₂ |
| A-126 | F | H | Br | CHF₂ |
| A-127 | Cl | H | Cl | CHF₂ |
| A-128 | Cl | Cl | Cl | CHF₂ |
| A-129 | Cl | F | Cl | CHF₂ |
| A-130 | Cl | Br | Cl | CHF₂ |
| A-131 | Cl | H | Br | CHF₂ |
| A-132 | Br | H | Br | CHF₂ |
| A-133 | Br | F | Br | CHF₂ |
| A-134 | Br | Cl | Br | CHF₂ |
| A-135 | CF₃ | H | F | CHF₂ |
| A-136 | CF₃ | H | Cl | CHF₂ |
| A-137 | CF₃ | H | Br | CHF₂ |
| A-138 | CF₃ | H | CF₃ | CHF₂ |
| A-139 | CF₃ | F | F | CHF₂ |
| A-140 | CF₃ | Cl | Cl | CHF₂ |
| A-141 | CF₃ | Br | Br | CHF₂ |
| A-142 | SF₅ | H | F | CHF₂ |
| A-143 | SF₅ | H | Cl | CHF₂ |
| A-144 | SF₅ | H | Br | CHF₂ |
| A-145 | SF₅ | H | CF₃ | CHF₂ |
| A-146 | SF₅ | H | H | CHF₂ |
| A-147 | CF₃ | H | H | CHF₂ |
| A-148 | Br | H | H | CHF₂ |
| A-149 | Cl | H | H | CHF₂ |
| A-150 | F | H | H | CHF₂ |
| A-151 | F | H | F | CF₃ |
| A-152 | F | F | F | CF₃ |
| A-153 | F | Cl | F | CF₃ |
| A-154 | F | Br | F | CF₃ |
| A-155 | F | H | Cl | CF₃ |
| A-156 | F | H | Br | CF₃ |
| A-157 | Cl | H | Cl | CF₃ |
| A-158 | Cl | Cl | Cl | CF₃ |
| A-159 | Cl | F | Cl | CF₃ |
| A-160 | Cl | Br | Cl | CF₃ |
| A-161 | Cl | H | Br | CF₃ |
| A-162 | Br | H | Br | CF₃ |
| A-163 | Br | F | Br | CF₃ |
| A-164 | Br | Cl | Br | CF₃ |
| A-165 | CF₃ | H | F | CF₃ |
| A-166 | CF₃ | H | Cl | CF₃ |
| A-167 | CF₃ | H | Br | CF₃ |
| A-168 | CF₃ | H | CF₃ | CF₃ |
| A-169 | CF₃ | F | F | CF₃ |
| A-170 | CF₃ | Cl | Cl | CF₃ |
| A-171 | CF₃ | Br | Br | CF₃ |
| A-172 | SF₅ | H | F | CF₃ |
| A-173 | SF₅ | H | Cl | CF₃ |
| A-174 | SF₅ | H | Br | CF₃ |
| A-175 | SF₃ | H | CF₃ | CF₃ |
| A-176 | SF₅ | H | H | CF₃ |
| A-177 | CF₃ | H | H | CF₃ |
| A-178 | Br | H | H | CF₃ |
| A-179 | Cl | H | H | CF₃ |
| A-180 | F | H | H | CF₃ |
| A-181 | F | H | F | CH₂-CH=CH₂ |
| A-182 | F | F | F | CH₂-CH=CH₂ |
| A-183 | F | Cl | F | CH₂-CH=CH₂ |
| A-184 | F | Br | F | CH₂-CH=CH₂ |
| A-185 | F | H | Cl | CH₂-CH=CH₂ |
| A-186 | F | H | Br | CH₂-CH=CH₂ |
| A-187 | Cl | H | Cl | CH₂-CH=CH₂ |
| A-188 | Cl | Cl | Cl | CH₂-CH=CH₂ |
| A-189 | Cl | F | Cl | CH₂-CH=CH₂ |
| A-190 | Cl | Br | Cl | CH₂-CH=CH₂ |
| A-191 | Cl | H | Br | CH₂-CH=CH₂ |
| A-192 | Br | H | Br | CH₂-CH=CH₂ |
| A-193 | Br | F | Br | CH₂-CH=CH₂ |
| A-194 | Br | Cl | Br | CH₂-CH=CH₂ |
| A-195 | CF₃ | H | F | CH₂-CH=CH₂ |
| A-196 | CF₃ | H | Cl | CH₂-CH=CH₂ |
| A-197 | CF₃ | H | Br | CH₂-CH=CH₂ |
| A-198 | CF₃ | H | CF₃ | CH₂-CH=CH₂ |
| A-199 | CF₃ | F | F | CH₂-CH=CH₂ |
| A-200 | CF₃ | Cl | Cl | CH₂-CH=CH₂ |
| A-201 | CF₃ | Br | Br | CH₂-CH=CH₂ |
| A-202 | SF₅ | H | F | CH₂-CH=CH₂ |
| A-203 | SF₅ | H | Cl | CH₂-CH=CH₂ |
| A-204 | SF₅ | H | Br | CH₂-CH=CH₂ |
| A-205 | SF₅ | H | CF₃ | CH₂-CH=CH₂ |
| A-206 | SF₅ | H | H | CH₂-CH=CH₂ |
| A-207 | CF₃ | H | H | CH₂-CH=CH₂ |
| A-208 | Br | H | H | CH₂-CH=CH₂ |
| A-209 | Cl | H | H | CH₂-CH=CH₂ |
| A-210 | F | H | H | CH₂-CH=CH₂ |
| A-211 | F | H | F | CH=CH₂ |
| A-212 | F | F | F | CH=CH₂ |
| A-213 | F | Cl | F | CH=CH₂ |
| A-214 | F | Br | F | CH=CH₂ |
| A-215 | F | H | Cl | CH=CH₂ |
| A-216 | F | H | Br | CH=CH₂ |
| A-217 | Cl | H | Cl | CH=CH₂ |
| A-218 | Cl | Cl | Cl | CH=CH₂ |
| A-219 | Cl | F | Cl | CH=CH₂ |
| A-220 | Cl | Br | Cl | CH=CH₂ |
| A-221 | Cl | H | Br | CH=CH₂ |
| A-222 | Br | H | Br | CH=CH₂ |
| A-223 | Br | F | Br | CH=CH₂ |
| A-224 | Br | Cl | Br | CH=CH₂ |
| A-225 | CF₃ | H | F | CH=CH₂ |
| A-226 | CF₃ | H | Cl | CH=CH₂ |
| A-227 | CF₃ | H | Br | CH=CH₂ |
| A-228 | CF₃ | H | CF₃ | CH=CH₂ |
| A-229 | CF₃ | F | F | CH=CH₂ |
| A-230 | CF₃ | Cl | Cl | CH=CH₂ |
| A-231 | CF₃ | Br | Br | CH=CH₂ |
| A-232 | SF₅ | H | F | CH=CH₂ |
| A-233 | SF₅ | H | Cl | CH=CH₂ |
| A-234 | SF₅ | H | Br | CH=CH₂ |
| A-235 | SF₅ | H | CF₃ | CH=CH₂ |
| A-236 | SF₅ | H | H | CH=CH₂ |
| A-237 | CF₃ | H | H | CH=CH₂ |
| A-238 | Br | H | H | CH=CH₂ |
| A-239 | Cl | H | H | CH=CH₂ |
| A-240 | F | H | H | CH=CH₂ |
| A-241 | F | H | F | C≡CH |
| A-242 | F | F | F | C≡CH |
| A-243 | F | Cl | F | C≡CH |
| A-244 | F | Br | F | C≡CH |
| A-245 | F | H | Cl | C≡CH |
| A-246 | F | H | Br | C≡CH |
| A-247 | Cl | H | Cl | C≡CH |
| A-248 | Cl | Cl | Cl | C≡CH |
| A-249 | Cl | F | Cl | C≡CH |
| A-250 | Cl | Br | Cl | C≡CH |
| A-251 | Cl | H | Br | C≡CH |
| A-252 | Br | H | Br | C≡CH |
| A-253 | Br | F | Br | C≡CH |
| A-254 | Br | Cl | Br | C≡CH |
| A-255 | CF₃ | H | F | C≡CH |
| A-256 | CF₃ | H | Cl | C≡CH |
| A-257 | CF₃ | H | Br | C≡CH |
| A-258 | CF₃ | H | CF₃ | C≡CH |
| A-259 | CF₃ | F | F | C≡CH |
| A-260 | CF₃ | Cl | Cl | C≡CH |
| A-261 | CF₃ | Br | Br | C≡CH |
| A-262 | SF₅ | H | F | C≡CH |
| A-263 | SF₅ | H | Cl | C≡CH |
| A-264 | SF₅ | H | Br | C≡CH |
| A-265 | SF₅ | H | CF₃ | C≡CH |
| A-266 | SF₅ | H | H | C≡CH |
| A-267 | CF₃ | H | H | C≡CH |
| A-268 | Br | H | H | C≡CH |
| A-269 | Cl | H | H | C≡CH |
| A-270 | F | H | H | C≡CH |
| A-271 | F | H | F | *^{c}*C₃H₅* |
| A-272 | F | F | F | *^{c}*C₃H₅* |
| A-273 | F | Cl | F | *^{c}*C₃H₅* |
| A-274 | F | Br | F | *^{c}*C₃H₅* |
| A-275 | F | H | Cl | *^{c}*C₃H₅* |
| A-276 | F | H | Br | *^{c}*C₃H₅* |
| A-277 | Cl | H | Cl | *^{c}*C₃H₅* |
| A-278 | Cl | Cl | Cl | *^{c}*C₃H₅* |
| A-279 | Cl | F | Cl | *^{c}*C₃H₅* |
| A-280 | Cl | Br | Cl | *^{c}*C₃H₅* |
| A-281 | Cl | H | Br | *^{c}*C₃H₅* |
| A-282 | Br | H | Br | *^{c}*C₃H₅* |
| A-283 | Br | F | Br | *^{c}*C₃H₅* |
| A-284 | Br | Cl | Br | *^{c}*C₃H₅* |
| A-285 | CF₃ | H | F | *^{c}*C₃H₅* |
| A-286 | CF₃ | H | Cl | *^{c}*C₃H₅* |
| A-287 | CF₃ | H | Br | *^{c}*C₃H₅* |
| A-288 | CF₃ | H | CF₃ | *^{c}*C₃H₅* |
| A-289 | CF₃ | F | F | *^{c}*C₃H₅* |
| A-290 | CF₃ | Cl | Cl | *^{c}*C₃H₅* |
| A-291 | CF₃ | Br | Br | *^{c}*C₃H₅* |
| A-292 | SF₅ | H | F | *^{c}*C₃H₅* |
| A-293 | SF₅ | H | Cl | *^{c}*C₃H₅* |
| A-294 | SF₅ | H | Br | *^{c}*C₃H₅* |
| A-295 | SF₅ | H | CF₃ | *^{c}*C₃H₅* |
| A-296 | SF₅ | H | H | *^{c}*C₃H₅* |
| A-297 | CF₃ | H | H | *^{c}*C₃H₅* |
| A-298 | Br | H | H | *^{c}*C₃H₅* |
| A-299 | Cl | H | H | *^{c}*C₃H₅* |
| A-300 | F | H | H | *^{c}*C₃H₅* |
| A-301 | F | H | F | F |
| A-302 | F | F | F | F |
| A-303 | F | Cl | F | F |
| A-304 | F | Br | F | F |
| A-305 | F | H | Cl | F |
| A-306 | F | H | Br | F |
| A-307 | Cl | H | Cl | F |
| A-308 | Cl | Cl | Cl | F |
| A-309 | Cl | F | Cl | F |
| A-310 | Cl | Br | Cl | F |
| A-311 | Cl | H | Br | F |
| A-312 | Br | H | Br | F |
| A-313 | Br | F | Br | F |
| A-314 | Br | Cl | Br | F |
| A-315 | CF₃ | H | F | F |
| A-316 | CF₃ | H | Cl | F |
| A-317 | CF₃ | H | Br | F |
| A-318 | CF₃ | H | CF₃ | F |
| A-319 | CF₃ | F | F | F |
| A-320 | CF₃ | Cl | Cl | F |
| A-321 | CF₃ | Br | Br | F |
| A-322 | SF₅ | H | F | F |
| A-323 | SF₅ | H | Cl | F |
| A-324 | SF₅ | H | Br | F |
| A-325 | SF₅ | H | CF₃ | F |
| A-326 | SF₅ | H | H | F |
| A-327 | CF₃ | H | H | F |
| A-328 | Br | H | H | F |
| A-329 | Cl | H | H | F |
| A-330 | F | H | H | F |
| A-331 | F | H | F | Cl |
| A-332 | F | F | F | Cl |
| A-333 | F | Cl | F | Cl |
| A-334 | F | Br | F | Cl |
| A-335 | F | H | Cl | Cl |
| A-336 | F | H | Br | Cl |
| A-337 | Cl | H | Cl | Cl |
| A-338 | Cl | Cl | Cl | Cl |
| A-339 | Cl | F | Cl | Cl |
| A-340 | Cl | Br | Cl | Cl |
| A-341 | Cl | H | Br | Cl |
| A-342 | Br | H | Br | Cl |
| A-343 | Br | F | Br | Cl |
| A-344 | Br | Cl | Br | Cl |
| A-345 | CF₃ | H | F | Cl |
| A-346 | CF₃ | H | Cl | Cl |
| A-347 | CF₃ | H | Br | Cl |
| A-348 | CF₃ | H | CF₃ | Cl |
| A-349 | CF₃ | F | F | Cl |
| A-350 | CF₃ | Cl | Cl | Cl |
| A-351 | CF₃ | Br | Br | Cl |
| A-352 | SF₅ | H | F | Cl |
| A-353 | SF₅ | H | Cl | Cl |
| A-354 | SF₅ | H | Br | Cl |
| A-355 | SF₅ | H | CF₃ | Cl |
| A-356 | SF₅ | H | H | Cl |
| A-357 | CF₃ | H | H | Cl |
| A-358 | Br | H | H | Cl |
| A-359 | Cl | H | H | Cl |
| A-360 | F | H | H | Cl |
| A-361 | F | H | F | Br |
| A-362 | F | F | F | Br |
| A-363 | F | Cl | F | Br |
| A-364 | F | Br | F | Br |
| A-365 | F | H | Cl | Br |
| A-366 | F | H | Br | Br |
| A-367 | Cl | H | Cl | Br |
| A-368 | Cl | Cl | Cl | Br |
| A-369 | Cl | F | Cl | Br |
| A-370 | Cl | Br | Cl | Br |
| A-371 | Cl | H | Br | Br |
| A-372 | Br | H | Br | Br |
| A-373 | Br | F | Br | Br |
| A-374 | Br | Cl | Br | Br |
| A-375 | CF₃ | H | F | Br |
| A-376 | CF₃ | H | Cl | Br |
| A-377 | CF₃ | H | Br | Br |
| A-378 | CF₃ | H | CF₃ | Br |
| A-379 | CF₃ | F | F | Br |
| A-380 | CF₃ | Cl | Cl | Br |
| A-381 | CF₃ | Br | Br | Br |
| A-382 | SF₅ | H | F | Br |
| A-383 | SF₅ | H | Cl | Br |
| A-384 | SF₅ | H | Br | Br |
| A-385 | SF₅ | H | CF₃ | Br |
| A-386 | SF₅ | H | H | Br |
| A-387 | CF₃ | H | H | Br |
| A-388 | Br | H | H | Br |
| A-389 | Cl | H | H | Br |
| A-390 | F | H | H | Br |
| A-391 | F | H | F | CN |
| A-392 | F | F | F | CN |
| A-393 | F | Cl | F | CN |
| A-394 | F | Br | F | CN |
| A-395 | F | H | Cl | CN |
| A-396 | F | H | Br | CN |
| A-397 | Cl | H | Cl | CN |
| A-398 | Cl | Cl | Cl | CN |
| A-399 | Cl | F | Cl | CN |
| A-400 | Cl | Br | Cl | CN |
| A-401 | Cl | H | Br | CN |
| A-402 | Br | H | Br | CN |
| A-403 | Br | F | Br | CN |
| A-404 | Br | Cl | Br | CN |
| A-405 | CF₃ | H | F | CN |
| A-406 | CF₃ | H | Cl | CN |
| A-407 | CF₃ | H | Br | CN |
| A-408 | CF₃ | H | CF₃ | CN |
| A-409 | CF₃ | F | F | CN |
| A-410 | CF₃ | Cl | Cl | CN |
| A-411 | CF₃ | Br | Br | CN |
| A-412 | SF₅ | H | F | CN |
| A-413 | SF₅ | H | Cl | CN |
| A-414 | SF₅ | H | Br | CN |
| A-415 | SF₅ | H | CF₃ | CN |
| A-416 | SF₅ | H | H | CN |
| A-417 | CF₃ | H | H | CN |
| A-418 | Br | H | H | CN |
| A-419 | Cl | H | H | CN |
| A-420 | F | H | H | CN |
| A-421 | F | H | F | OCH₃ |
| A-422 | F | F | F | OCH₃ |
| A-423 | F | Cl | F | OCH₃ |
| A-424 | F | Br | F | OCH₃ |
| A-425 | F | H | Cl | OCH₃ |
| A-426 | F | H | Br | OCH₃ |
| A-427 | Cl | H | Cl | OCH₃ |
| A-428 | Cl | Cl | Cl | OCH₃ |
| A-429 | Cl | F | Cl | OCH₃ |
| A-430 | Cl | Br | Cl | OCH₃ |
| A-431 | Cl | H | Br | OCH₃ |
| A-432 | Br | H | Br | OCH₃ |
| A-433 | Br | F | Br | OCH₃ |
| A-434 | Br | Cl | Br | OCH₃ |
| A-435 | CF₃ | H | F | OCH₃ |
| A-436 | CF₃ | H | Cl | OCH₃ |
| A-437 | CF₃ | H | Br | OCH₃ |
| A-438 | CF₃ | H | CF₃ | OCH₃ |
| A-439 | CF₃ | F | F | OCH₃ |
| A-440 | CF₃ | Cl | Cl | OCH₃ |
| A-441 | CF₃ | Br | Br | OCH₃ |
| A-442 | SF₅ | H | F | OCH₃ |
| A-443 | SF₅ | H | Cl | OCH₃ |
| A-444 | SF₅ | H | Br | OCH₃ |
| A-445 | SF₅ | H | CF₃ | OCH₃ |
| A-446 | SF₅ | H | H | OCH₃ |
| A-447 | CF₃ | H | H | OCH₃ |
| A-448 | Br | H | H | OCH₃ |
| A-449 | Cl | H | H | OCH₃ |
| A-450 | F | H | H | OCH₃ |
| A-451 | F | H | F | OCH₂CH₃ |
| A-452 | F | F | F | OCH₂CH₃ |
| A-453 | F | Cl | F | OCH₂CH₃ |
| A-454 | F | Br | F | OCH₂CH₃ |
| A-455 | F | H | Cl | OCH₂CH₃ |
| A-456 | F | H | Br | OCH₂CH₃ |
| A-457 | Cl | H | Cl | OCH₂CH₃ |
| A-458 | Cl | Cl | Cl | OCH₂CH₃ |
| A-459 | Cl | F | Cl | OCH₂CH₃ |
| A-460 | Cl | Br | Cl | OCH₂CH₃ |
| A-461 | Cl | H | Br | OCH₂CH₃ |
| A-462 | Br | H | Br | OCH₂CH₃ |
| A-463 | Br | F | Br | OCH₂CH₃ |
| A-464 | Br | Cl | Br | OCH₂CH₃ |
| A-465 | CF₃ | H | F | OCH₂CH₃ |
| A-466 | CF₃ | H | Cl | OCH₂CH₃ |
| A-467 | CF₃ | H | Br | OCH₂CH₃ |
| A-468 | CF₃ | H | CF₃ | OCH₂CH₃ |
| A-469 | CF₃ | F | F | OCH₂CH₃ |
| A-470 | CF₃ | Cl | Cl | OCH₂CH₃ |
| A-471 | CF₃ | Br | Br | OCH₂CH₃ |
| A-472 | SF₅ | H | F | OCH₂CH₃ |
| A-473 | SF₅ | H | Cl | OCH₂CH₃ |
| A-474 | SF₅ | H | Br | OCH₂CH₃ |
| A-475 | SF₅ | H | CF₃ | OCH₂CH₃ |
| A-476 | SF₅ | H | H | OCH₂CH₃ |
| A-477 | CF₃ | H | H | OCH₂CH₃ |
| A-478 | Br | H | H | OCH₂CH₃ |
| A-479 | Cl | H | H | OCH₂CH₃ |
| A-480 | F | H | H | OCH₂CH₃ |
| A-481 | F | H | F | OCH(CH₃)₂ |
| A-482 | F | F | F | OCH(CH₃)₂ |
| A-483 | F | Cl | F | OCH(CH₃)₂ |
| A-484 | F | Br | F | OCH(CH₃)₂ |
| A-485 | F | H | Cl | OCH(CH₃)₂ |
| A-486 | F | H | Br | OCH(CH₃)₂ |
| A-487 | Cl | H | Cl | OCH(CH₃)₂ |
| A-488 | Cl | Cl | Cl | OCH(CH₃)₂ |
| A-489 | Cl | F | Cl | OCH(CH₃)₂ |
| A-490 | Cl | Br | Cl | OCH(CH₃)₂ |
| A-491 | Cl | H | Br | OCH(CH₃)₂ |
| A-492 | Br | H | Br | OCH(CH₃)₂ |
| A-493 | Br | F | Br | OCH(CH₃)₂ |
| A-494 | Br | Cl | Br | OCH(CH₃)₂ |
| A-495 | CF₃ | H | F | OCH(CH₃)₂ |
| A-496 | CF₃ | H | Cl | OCH(CH₃)₂ |
| A-497 | CF₃ | H | Br | OCH(CH₃)₂ |
| A-498 | CF₃ | H | CF₃ | OCH(CH₃)₂ |
| A-499 | CF₃ | F | F | OCH(CH₃)₂ |
| A-500 | CF₃ | Cl | Cl | OCH(CH₃)₂ |
| A-501 | CF₃ | Br | Br | OCH(CH₃)₂ |
| A-502 | SF₅ | H | F | OCH(CH₃)₂ |
| A-503 | SF₅ | H | Cl | OCH(CH₃)₂ |
| A-504 | SF₅ | H | Br | OCH(CH₃)₂ |
| A-505 | SF₅ | H | CF₃ | OCH(CH₃)₂ |
| A-506 | SF₅ | H | H | OCH(CH₃)₂ |
| A-507 | CF₃ | H | H | OCH(CH₃)₂ |
| A-508 | Br | H | H | OCH(CH₃)₂ |
| A-509 | Cl | H | H | OCH(CH₃)₂ |
| A-510 | F | H | H | OCH(CH₃)₂ |
| A-511 | F | H | F | OCH₂CH=CH₂ |
| A-512 | F | F | F | OCH₂CH=CH₂ |
| A-513 | F | Cl | F | OCH₂CH=CH₂ |
| A-514 | F | Br | F | OCH₂CH=CH₂ |
| A-515 | F | H | Cl | OCH₂CH=CH₂ |
| A-516 | F | H | Br | OCH₂CH=CH₂ |
| A-517 | Cl | H | Cl | OCH₂CH=CH₂ |
| A-518 | Cl | Cl | Cl | OCH₂CH=CH₂ |
| A-519 | Cl | F | Cl | OCH₂CH=CH₂ |
| A-520 | Cl | Br | Cl | OCH₂CH=CH₂ |
| A-521 | Cl | H | Br | OCH₂CH=CH₂ |
| A-522 | Br | H | Br | OCH₂CH=CH₂ |
| A-523 | Br | F | Br | OCH₂CH=CH₂ |
| A-524 | Br | Cl | Br | OCH₂CH=CH₂ |
| A-525 | CF₃ | H | F | OCH₂CH=CH₂ |
| A-526 | CF₃ | H | Cl | OCH₂CH=CH₂ |
| A-527 | CF₃ | H | Br | OCH₂CH=CH₂ |
| A-528 | CF₃ | H | CF₃ | OCH₂CH=CH₂ |
| A-529 | CF₃ | F | F | OCH₂CH=CH₂ |
| A-530 | CF₃ | Cl | Cl | OCH₂CH=CH₂ |
| A-531 | CF₃ | Br | Br | OCH₂CH=CH₂ |
| A-532 | SF₅ | H | F | OCH₂CH=CH₂ |
| A-533 | SF₅ | H | Cl | OCH₂CH=CH₂ |
| A-534 | SF₅ | H | Br | OCH₂CH=CH₂ |
| A-535 | SF₅ | H | CF₃ | OCH₂CH=CH₂ |
| A-536 | SF₅ | H | H | OCH₂CH=CH₂ |
| A-537 | CF₃ | H | H | OCH₂CH=CH₂ |
| A-538 | Br | H | H | OCH₂CH=CH₂ |
| A-539 | Cl | H | H | OCH₂CH=CH₂ |
| A-540 | F | H | H | OCH₂CH=CH₂ |
| A-541 | F | H | F | O-*^{c}*C₃H₅* |
| A-542 | F | F | F | O-*^{c}*C₃H₅* |
| A-543 | F | Cl | F | O-*^{c}*C₃H₅* |
| A-544 | F | Br | F | O-*^{c}*C₃H₅* |
| A-545 | F | H | Cl | O-*^{c}*C₃H₅* |
| A-546 | F | H | Br | O-*^{c}*C₃H₅* |
| A-547 | Cl | H | Cl | O-*^{c}*C₃H₅* |
| A-548 | Cl | Cl | Cl | O-*^{c}*C₃H₅* |
| A-549 | Cl | F | Cl | O-*^{c}*C₃H₅* |
| A-550 | Cl | Br | Cl | O-*^{c}*C₃H₅* |
| A-551 | Cl | H | Br | O-*^{c}*C₃H₅* |
| A-552 | Br | H | Br | O-*^{c}*C₃H₅* |
| A-553 | Br | F | Br | O-*^{c}*C₃H₅* |
| A-554 | Br | Cl | Br | O-*^{c}*C₃H₅* |
| A-555 | CF₃ | H | F | O-*^{c}*C₃H₅* |
| A-556 | CF₃ | H | Cl | O-*^{c}*C₃H₅* |
| A-557 | CF₃ | H | Br | O-*^{c}*C₃H₅* |
| A-558 | CF₃ | H | CF₃ | O-*^{c}*C₃H₅* |
| A-559 | CF₃ | F | F | O-*^{c}*C₃H₅* |
| A-560 | CF₃ | Cl | Cl | O-*^{c}*C₃H₅* |
| A-561 | CF₃ | Br | Br | O-*^{c}*C₃H₅* |
| A-562 | SF₅ | H | F | O-*^{c}*C₃H₅* |
| A-563 | SF₅ | H | Cl | O-*^{c}*C₃H₅* |
| A-564 | SF₅ | H | Br | O-*^{c}*C₃H₅* |
| A-565 | SF₅ | H | CF₃ | O-*^{c}*C₃H₅* |
| A-566 | SF₅ | H | H | O-*^{c}*C₃H₅* |
| A-567 | CF₃ | H | H | O-*^{c}*C₃H₅* |
| A-568 | Br | H | H | O-*^{c}*C₃H₅* |
| A-569 | Cl | H | H | O-*^{c}*C₃H₅* |
| A-570 | F | H | H | O-*^{c}*C₃H₅* |
| A-571 | F | H | F | OCHF₂ |
| A-572 | F | F | F | OCHF₂ |
| A-573 | F | Cl | F | OCHF₂ |
| A-574 | F | Br | F | OCHF₂ |
| A-575 | F | H | Cl | OCHF₂ |
| A-576 | F | H | Br | OCHF₂ |
| A-577 | Cl | H | Cl | OCHF₂ |
| A-578 | Cl | Cl | Cl | OCHF₂ |
| A-579 | Cl | F | Cl | OCHF₂ |
| A-580 | Cl | Br | Cl | OCHF₂ |
| A-581 | Cl | H | Br | OCHF₂ |
| A-582 | Br | H | Br | OCHF₂ |
| A-583 | Br | F | Br | OCHF₂ |
| A-584 | Br | Cl | Br | OCHF₂ |
| A-585 | CF₃ | H | F | OCHF₂ |
| A-586 | CF₃ | H | Cl | OCHF₂ |
| A-587 | CF₃ | H | Br | OCHF₂ |
| A-588 | CF₃ | H | CF₃ | OCHF₂ |
| A-589 | CF₃ | F | F | OCHF₂ |
| A-590 | CF₃ | Cl | Cl | OCHF₂ |
| A-591 | CF₃ | Br | Br | OCHF₂ |
| A-592 | SF₅ | H | F | OCHF₂ |
| A-593 | SF₅ | H | Cl | OCHF₂ |
| A-594 | SF₅ | H | Br | OCHF₂ |
| A-595 | SF₅ | H | CF₃ | OCHF₂ |
| A-596 | SF₅ | H | H | OCHF₂ |
| A-597 | CF₃ | H | H | OCHF₂ |
| A-598 | Br | H | H | OCHF₂ |
| A-599 | Cl | H | H | OCHF₂ |
| A-600 | F | H | H | OCHF₂ |
| A-601 | F | H | F | OCF₃ |
| A-602 | F | F | F | OCF₃ |
| A-603 | F | Cl | F | OCF₃ |
| A-604 | F | Br | F | OCF₃ |
| A-605 | F | H | Cl | OCF₃ |
| A-606 | F | H | Br | OCF₃ |
| A-607 | Cl | H | Cl | OCF₃ |
| A-608 | Cl | Cl | Cl | OCF₃ |
| A-609 | Cl | F | Cl | OCF₃ |
| A-610 | Cl | Br | Cl | OCF₃ |
| A-611 | Cl | H | Br | OCF₃ |
| A-612 | Br | H | Br | OCF₃ |
| A-613 | Br | F | Br | OCF₃ |
| A-614 | Br | Cl | Br | OCF₃ |
| A-615 | CF₃ | H | F | OCF₃ |
| A-616 | CF₃ | H | Cl | OCF₃ |
| A-617 | CF₃ | H | Br | OCF₃ |
| A-618 | CF₃ | H | CF₃ | OCF₃ |
| A-619 | CF₃ | F | F | OCF₃ |
| A-620 | CF₃ | Cl | Cl | OCF₃ |
| A-621 | CF₃ | Br | Br | OCF₃ |
| A-622 | SF₅ | H | F | OCF₃ |
| A-623 | SF₅ | H | Cl | OCF₃ |
| A-624 | SF₅ | H | Br | OCF₃ |
| A-625 | SF₅ | H | CF₃ | OCF₃ |
| A-626 | SF₅ | H | H | OCF₃ |
| A-627 | CF₃ | H | H | OCF₃ |
| A-628 | Br | H | H | OCF₃ |
| A-629 | Cl | H | H | OCF₃ |
| A-630 | F | H | H | OCF₃ |
| A-631 | F | H | F | OCH₂CF₃ |
| A-632 | F | F | F | OCH₂CF₃ |
| A-633 | F | Cl | F | OCH₂CF₃ |
| A-634 | F | Br | F | OCH₂CF₃ |
| A-635 | F | H | Cl | OCH₂CF₃ |
| A-636 | F | H | Br | OCH₂CF₃ |
| A-637 | Cl | H | Cl | OCH₂CF₃ |
| A-638 | Cl | Cl | Cl | OCH₂CF₃ |
| A-639 | Cl | F | Cl | OCH₂CF₃ |
| A-640 | Cl | Br | Cl | OCH₂CF₃ |
| A-641 | Cl | H | Br | OCH₂CF₃ |
| A-642 | Br | H | Br | OCH₂CF₃ |
| A-643 | Br | F | Br | OCH₂CF₃ |
| A-644 | Br | Cl | Br | OCH₂CF₃ |
| A-645 | CF₃ | H | F | OCH₂CF₃ |
| A-646 | CF₃ | H | Cl | OCH₂CF₃ |
| A-647 | CF₃ | H | Br | OCH₂CF₃ |
| A-648 | CF₃ | H | CF₃ | OCH₂CF₃ |
| A-649 | CF₃ | F | F | OCH₂CF₃ |
| A-650 | CF₃ | Cl | Cl | OCH₂CF₃ |
| A-651 | CF₃ | Br | Br | OCH₂CF₃ |
| A-652 | SF₅ | H | F | OCH₂CF₃ |
| A-653 | SF₅ | H | Cl | OCH₂CF₃ |
| A-654 | SF₅ | H | Br | OCH₂CF₃ |
| A-655 | SF₅ | H | CF₃ | OCH₂CF₃ |
| A-656 | SF₅ | H | H | OCH₂CF₃ |
| A-657 | CF₃ | H | H | OCH₂CF₃ |
| A-658 | Br | H | H | OCH₂CF₃ |
| A-659 | Cl | H | H | OCH₂CF₃ |
| A-660 | F | H | H | OCH₂CF₃ |
| A-661 | F | H | F | SCH₃ |
| A-662 | F | F | F | SCH₃ |
| A-663 | F | Cl | F | SCH₃ |
| A-664 | F | Br | F | SCH₃ |
| A-665 | F | H | Cl | SCH₃ |
| A-666 | F | H | Br | SCH₃ |
| A-667 | Cl | H | Cl | SCH₃ |
| A-668 | Cl | Cl | Cl | SCH₃ |
| A-669 | Cl | F | Cl | SCH₃ |
| A-670 | Cl | Br | Cl | SCH₃ |
| A-671 | Cl | H | Br | SCH₃ |
| A-672 | Br | H | Br | SCH₃ |
| A-673 | Br | F | Br | SCH₃ |
| A-674 | Br | Cl | Br | SCH₃ |
| A-675 | CF₃ | H | F | SCH₃ |
| A-676 | CF₃ | H | Cl | SCH₃ |
| A-677 | CF₃ | H | Br | SCH₃ |
| A-678 | CF₃ | H | CF₃ | SCH₃ |
| A-679 | CF₃ | F | F | SCH₃ |
| A-680 | CF₃ | Cl | Cl | SCH₃ |
| A-681 | CF₃ | Br | Br | SCH₃ |
| A-682 | SF₅ | H | F | SCH₃ |
| A-683 | SF₅ | H | Cl | SCH₃ |
| A-684 | SF₅ | H | Br | SCH₃ |
| A-685 | SF₅ | H | CF₃ | SCH₃ |
| A-686 | SF₅ | H | H | SCH₃ |
| A-687 | CF₃ | H | H | SCH₃ |
| A-688 | Br | H | H | SCH₃ |
| A-689 | Cl | H | H | SCH₃ |
| A-690 | F | H | H | SCH₃ |
| A-691 | F | H | F | SCH₂CH₃ |
| A-692 | F | F | F | SCH₂CH₃ |
| A-693 | F | Cl | F | SCH₂CH₃ |
| A-694 | F | Br | F | SCH₂CH₃ |
| A-695 | F | H | Cl | SCH₂CH₃ |
| A-696 | F | H | Br | SCH₂CH₃ |
| A-697 | Cl | H | Cl | SCH₂CH₃ |
| A-698 | Cl | Cl | Cl | SCH₂CH₃ |
| A-699 | Cl | F | Cl | SCH₂CH₃ |
| A-700 | Cl | Br | Cl | SCH₂CH₃ |
| A-701 | Cl | H | Br | SCH₂CH₃ |
| A-702 | Br | H | Br | SCH₂CH₃ |
| A-703 | Br | F | Br | SCH₂CH₃ |
| A-704 | Br | Cl | Br | SCH₂CH₃ |
| A-705 | CF₃ | H | F | SCH₂CH₃ |
| A-706 | CF₃ | H | Cl | SCH₂CH₃ |
| A-707 | CF₃ | H | Br | SCH₂CH₃ |
| A-708 | CF₃ | H | CF₃ | SCH₂CH₃ |
| A-709 | CF₃ | F | F | SCH₂CH₃ |
| A-710 | CF₃ | Cl | Cl | SCH₂CH₃ |
| A-711 | CF₃ | Br | Br | SCH₂CH₃ |
| A-712 | SF₅ | H | F | SCH₂CH₃ |
| A-713 | SF₅ | H | Cl | SCH₂CH₃ |
| A-714 | SF₅ | H | Br | SCH₂CH₃ |
| A-715 | SF₅ | H | CF₃ | SCH₂CH₃ |
| A-716 | SF₅ | H | H | SCH₂CH₃ |
| A-717 | CF₃ | H | H | SCH₂CH₃ |
| A-718 | Br | H | H | SCH₂CH₃ |
| A-719 | Cl | H | H | SCH₂CH₃ |
| A-720 | F | H | H | SCH₂CH₃ |
| A-721 | F | H | F | SCH(CH₃)₂ |
| A-722 | F | F | F | SCH(CH₃)₂ |
| A-723 | F | Cl | F | SCH(CH₃)₂ |
| A-724 | F | Br | F | SCH(CH₃)₂ |
| A-725 | F | H | Cl | SCH(CH₃)₂ |
| A-726 | F | H | Br | SCH(CH₃)₂ |
| A-727 | Cl | H | Cl | SCH(CH₃)₂ |
| A-728 | Cl | Cl | Cl | SCH(CH₃)₂ |
| A-729 | Cl | F | Cl | SCH(CH₃)₂ |
| A-730 | Cl | Br | Cl | SCH(CH₃)₂ |
| A-731 | Cl | H | Br | SCH(CH₃)₂ |
| A-732 | Br | H | Br | SCH(CH₃)₂ |
| A-733 | Br | F | Br | SCH(CH₃)₂ |
| A-734 | Br | Cl | Br | SCH(CH₃)₂ |
| A-735 | CF₃ | H | F | SCH(CH₃)₂ |
| A-736 | CF₃ | H | Cl | SCH(CH₃)₂ |
| A-737 | CF₃ | H | Br | SCH(CH₃)₂ |
| A-738 | CF₃ | H | CF₃ | SCH(CH₃)₂ |
| A-739 | CF₃ | F | F | SCH(CH₃)₂ |
| A-740 | CF₃ | Cl | Cl | SCH(CH₃)₂ |
| A-741 | CF₃ | Br | Br | SCH(CH₃)₂ |
| A-742 | SF₅ | H | F | SCH(CH₃)₂ |
| A-743 | SF₅ | H | Cl | SCH(CH₃)₂ |
| A-744 | SF₅ | H | Br | SCH(CH₃)₂ |
| A-745 | SF₅ | H | CF₃ | SCH(CH₃)₂ |
| A-746 | SF₅ | H | H | SCH(CH₃)₂ |
| A-747 | CF₃ | H | H | SCH(CH₃)₂ |
| A-748 | Br | H | H | SCH(CH₃)₂ |
| A-749 | Cl | H | H | SCH(CH₃)₂ |
| A-750 | F | H | H | SCH(CH₃)₂ |
| A-751 | F | H | F | SCH₂CH=CH₂ |
| A-752 | F | F | F | SCH₂CH=CH₂ |
| A-753 | F | Cl | F | SCH₂CH=CH₂ |
| A-754 | F | Br | F | SCH₂CH=CH₂ |
| A-755 | F | H | Cl | SCH₂CH=CH₂ |
| A-756 | F | H | Br | SCH₂CH=CH₂ |
| A-757 | Cl | H | Cl | SCH₂CH=CH₂ |
| A-758 | Cl | Cl | Cl | SCH₂CH=CH₂ |
| A-759 | Cl | F | Cl | SCH₂CH=CH₂ |
| A-760 | Cl | Br | Cl | SCH₂CH=CH₂ |
| A-761 | Cl | H | Br | SCH₂CH=CH₂ |
| A-762 | Br | H | Br | SCH₂CH=CH₂ |
| A-763 | Br | F | Br | SCH₂CH=CH₂ |
| A-764 | Br | Cl | Br | SCH₂CH=CH₂ |
| A-765 | CF₃ | H | F | SCH₂CH=CH₂ |
| A-766 | CF₃ | H | Cl | SCH₂CH=CH₂ |
| A-767 | CF₃ | H | Br | SCH₂CH=CH₂ |
| A-768 | CF₃ | H | CF₃ | SCH₂CH=CH₂ |
| A-769 | CF₃ | F | F | SCH₂CH=CH₂ |
| A-770 | CF₃ | Cl | Cl | SCH₂CH=CH₂ |
| A-771 | CF₃ | Br | Br | SCH₂CH=CH₂ |
| A-772 | SF₅ | H | F | SCH₂CH=CH₂ |
| A-773 | SF₅ | H | Cl | SCH₂CH=CH₂ |
| A-774 | SF₅ | H | Br | SCH₂CH=CH₂ |
| A-775 | SF₅ | H | CF₃ | SCH₂CH=CH₂ |
| A-776 | SF₅ | H | H | SCH₂CH=CH₂ |
| A-777 | CF₃ | H | H | SCH₂CH=CH₂ |
| A-778 | Br | H | H | SCH₂CH=CH₂ |
| A-779 | Cl | H | H | SCH₂CH=CH₂ |
| A-780 | F | H | H | SCH₂CH=CH₂ |
| A-781 | F | H | F | S-*^{c}*C₃H₅* |
| A-782 | F | F | F | S-*^{c}*C₃H₅* |
| A-783 | F | Cl | F | S-*^{c}*C₃H₅* |
| A-784 | F | Br | F | S-*^{c}*C₃H₅* |
| A-785 | F | H | Cl | S-*^{c}*C₃H₅* |
| A-786 | F | H | Br | S-*^{c}*C₃H₅* |
| A-787 | Cl | H | Cl | S-*^{c}*C₃H₅* |
| A-788 | Cl | Cl | Cl | S-*^{c}*C₃H₅* |
| A-789 | Cl | F | Cl | S-*^{c}*C₃H₅* |
| A-790 | Cl | Br | Cl | S-*^{c}*C₃H₅* |
| A-791 | Cl | H | Br | S-*^{c}*C₃H₅* |
| A-792 | Br | H | Br | S-*^{c}*C₃H₅* |
| A-793 | Br | F | Br | S-*^{c}*C₃H₅* |
| A-794 | Br | Cl | Br | S-*^{c}*C₃H₅* |
| A-795 | CF₃ | H | F | S-*^{c}*C₃H₅* |
| A-796 | CF₃ | H | Cl | S-*^{c}*C₃H₅* |
| A-797 | CF₃ | H | Br | S-*^{c}*C₃H₅* |
| A-798 | CF₃ | H | CF₃ | S-*^{c}*C₃H₅* |
| A-799 | CF₃ | F | F | S-*^{c}*C₃H₅* |
| A-800 | CF₃ | Cl | Cl | S-*^{c}*C₃H₅* |
| A-801 | CF₃ | Br | Br | S-*^{c}*C₃H₅* |
| A-802 | SF₅ | H | F | S-*^{c}*C₃H₅* |
| A-803 | SF₅ | H | Cl | S-*^{c}*C₃H₅* |
| A-804 | SF₅ | H | Br | S-*^{c}*C₃H₅* |
| A-805 | SF₅ | H | CF₃ | S-*^{c}*C₃H₅* |
| A-806 | SF₅ | H | H | S-*^{c}*C₃H₅* |
| A-807 | CF₃ | H | H | S-*^{c}*C₃H₅* |
| A-808 | Br | H | H | S-*^{c}*C₃H₅* |
| A-809 | Cl | H | H | S-*^{c}*C₃H₅* |
| A-810 | F | H | H | S-*^{c}*C₃H₅* |
| A-811 | F | H | F | SCF₃ |
| A-812 | F | F | F | SCF₃ |
| A-813 | F | Cl | F | SCF₃ |
| A-814 | F | Br | F | SCF₃ |
| A-815 | F | H | Cl | SCF₃ |
| A-816 | F | H | Br | SCF₃ |
| A-817 | Cl | H | Cl | SCF₃ |
| A-818 | Cl | Cl | Cl | SCF₃ |
| A-819 | Cl | F | Cl | SCF₃ |
| A-820 | Cl | Br | Cl | SCF₃ |
| A-821 | Cl | H | Br | SCF₃ |
| A-822 | Br | H | Br | SCF₃ |
| A-823 | Br | F | Br | SCF₃ |
| A-824 | Br | Cl | Br | SCF₃ |
| A-825 | CF₃ | H | F | SCF₃ |
| A-826 | CF₃ | H | Cl | SCF₃ |
| A-827 | CF₃ | H | Br | SCF₃ |
| A-828 | CF₃ | H | CF₃ | SCF₃ |
| A-829 | CF₃ | F | F | SCF₃ |
| A-830 | CF₃ | Cl | Cl | SCF₃ |
| A-831 | CF₃ | Br | Br | SCF₃ |
| A-832 | SF₅ | H | F | SCF₃ |
| A-833 | SF₅ | H | Cl | SCF₃ |
| A-834 | SF₅ | H | Br | SCF₃ |
| A-835 | SF₅ | H | CF₃ | SCF₃ |
| A-836 | SF₅ | H | H | SCF₃ |
| A-837 | CF₃ | H | H | SCF₃ |
| A-838 | Br | H | H | SCF₃ |
| A-839 | Cl | H | H | SCF₃ |
| A-840 | F | H | H | SCF₃ |
| A-841 | F | H | F | SCH₂CF₃ |
| A-842 | F | F | F | SCH₂CF₃ |
| A-843 | F | Cl | F | SCH₂CF₃ |
| A-844 | F | Br | F | SCH₂CF₃ |
| A-845 | F | H | Cl | SCH₂CF₃ |
| A-846 | F | H | Br | SCH₂CF₃ |
| A-847 | Cl | H | Cl | SCH₂CF₃ |
| A-848 | Cl | Cl | Cl | SCH₂CF₃ |
| A-849 | Cl | F | Cl | SCH₂CF₃ |
| A-850 | Cl | Br | Cl | SCH₂CF₃ |
| A-851 | Cl | H | Br | SCH₂CF₃ |
| A-852 | Br | H | Br | SCH₂CF₃ |
| A-853 | Br | F | Br | SCH₂CF₃ |
| A-854 | Br | Cl | Br | SCH₂CF₃ |
| A-855 | CF₃ | H | F | SCH₂CF₃ |
| A-856 | CF₃ | H | Cl | SCH₂CF₃ |
| A-857 | CF₃ | H | Br | SCH₂CF₃ |
| A-858 | CF₃ | H | CF₃ | SCH₂CF₃ |
| A-859 | CF₃ | F | F | SCH₂CF₃ |
| A-860 | CF₃ | Cl | Cl | SCH₂CF₃ |
| A-861 | CF₃ | Br | Br | SCH₂CF₃ |
| A-862 | SF₅ | H | F | SCH₂CF₃ |
| A-863 | SF₅ | H | Cl | SCH₂CF₃ |
| A-864 | SF₅ | H | Br | SCH₂CF₃ |
| A-865 | SF₅ | H | CF₃ | SCH₂CF₃ |
| A-866 | SF₅ | H | H | SCH₂CF₃ |
| A-867 | CF₃ | H | H | SCH₂CF₃ |
| A-868 | Br | H | H | SCH₂CF₃ |
| A-869 | Cl | H | H | SCH₂CF₃ |
| A-870 | F | H | H | SCH₂CF₃ |

| | | | | |
|---|---|---|---|---|
| * *^{c}*C₃H₅ = cyclopropyl | | | | |

Among the above compounds, preference is given to compounds of formula Ia.2, Ia.3, Ia.10, Ia.11, Ia.12, Ia.13 and Ia.14.

The compounds of the formula I can be prepared by the methods as described in the below schemes or synthesis descriptions or in the synthesis descriptions of the working examples or by standard methods of organic chemistry. The substituents, variables and indices are as defined above for formula I, if not otherwise specified.

Compounds of formula I can be prepared by dehydrating a compound of formula **1** as shown in scheme 1 below. A' is A or a precursor of A, wherein A is a group C(=W)N(R⁵R⁶) and W, R⁵ und R⁶ are as defined above. Typical precursors of A are a halogen atom, CN, carboxy, tert-butoxycarbonyl, an acetale group, a protected aldehyde group or -OSO₂-R^{z1}, where R^{z1} is C₁-C₄-alkyl, C₁-C₄-haloalkyl or phenyl which may be substituted by 1, 2 or 3 radicals selected from C₁-C₄-alkyl, C₁-C₄-haloalkyl C₁-C₄-alkoxy or C₁-C₄-haloalkoxy. Compounds I' correspond to compounds I when A' is A. Dehydration either occurs spontaneously or with the help of dehydrating agents, such as molecular sieves, acid-washed molecular sieves, magnesium sulfate, sodium sulfate, silica gel, SOCl₂, POCl₃, Burgess reagent, trifluoroacetic anhydride, p-toluene sulfonic acid, anhydrous HCl or sulfuric acid. Preferably, p-toluene sulfonic acid or acid-washed molecular sieves are used. The water formed may alternatively be removed, e.g. by azeotropic distillation, e.g. with benzene/toluene as entrainer, e.g. using a Dean Stark trap. If necessary (i.e. if A' is a precursor of A), A' is then converted into a group A.

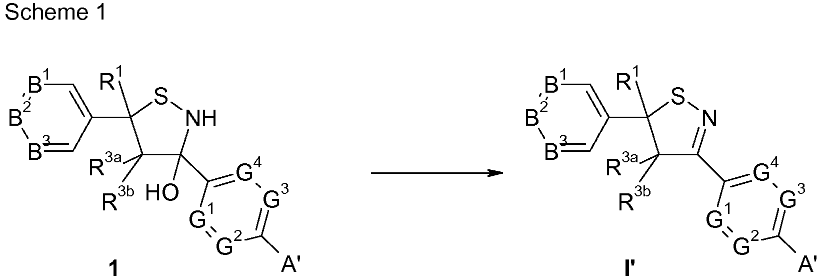

Compounds **1** wherein R^{3b} is hydrogen can be prepared by reacting a compound **3** with an amination agent to give a compound of formula **2,** which reacts spontaneously to the compound **1,** as shown in scheme 2. Depending on the amination agents used, amination can the carried out in a one step reaction, wherein compound **3** reacts directly to compound **2,** or as a two step reaction, wherein the SH group of compound **3** is first oxidized to a S-CI group, which then further reacts to a S-NH₂ group, thus giving compound **2.**

Suitable amination agents for the one step reaction are for example HOSA (hydroxylamine-O-sulfonic acid), which is generally used in the presence of a base (suitable bases being for example sodium hydrogen phosphate, potassium hydrogen phosphate, sodium hydroxide, potassium hydroxide, sodium carbonate, potassium carbonate, sodium methanolate, triethylamine and the like), O-(diphenylphosphoryl)hydroxyl amine, which is generally also used in the presence of a base (suitable bases being for example sodium hydrogen phosphate, potassium hydrogen phosphate, sodium hydroxide, potassium hydroxide, sodium carbonate, potassium carbonate, sodium methanolate, triethylamine and the like), 2,4-dinitrophenylhydroxyl amine, O-mesitylensulfonylhydroxylamine and 2-oxa-1-azaspiro[2.5]octane, among which HOSA and O-(diphenylphosphoryl)hydroxyl amine are preferred. The amination is generally carried out in a solvent, suitable solvents being for example chlorinated alkanes, such as methylene chloride or chloroform, aromatic solvents, such as benzene, toluene, the xylenes, chlorobenzene or dichlorobenzene, and ethers, such as diethylether, dipropylether, methyl tert-butylether, methyl isobutylether, ethylenegylcol dimethylether, tetrahydrofuran (THF) or dioxane and the like. The reaction is suitably carried out low temperature, e.g. at from -100 to 0°C or -78 to 0°C. Generally, the compound **3** is dispersed in a solvent and cooled to the desired temperature and the base is added followed by the amination agent, or the amination agent is added followed by the base, or base and amination agent are added simultaneously. HOSA is suitably used in combination with an amine base, such as triethylamine. In this case, it is preferred to cool compound **3** to -30 to 0°C, preferably - 20 to -10°C, to add the amine base at this temperature and then HOSA and keep the reaction at approximately -10 to 0°C.

Alternatively, O-(diphenylphosphoryl)hydroxyl amine can be used in combination with a base, e.g. with an inorganic base, such as sodium hydrogen phosphate, potassium hydrogen phosphate, sodium hydroxide, potassium hydroxide, sodium carbonate or potassium carbonate and specifically sodium hydrogen phosphate. In this case, it is suitable to cool compound **3** to -80 to -30°C, especially -80 to -70°C, to add the base at this temperature and then O-(diphenylphosphoryl)hydroxyl amine and keep the reaction at approximately 0°C to room temperature.

In the two step reaction, the compound **3** is first reacted with a chlorination agent which converts the SH group into an S-CI group. Suitable chlorination agents are for example sulfurylchloride, N-chloro succinimide (NCS), sodium hypochlorite, monochloroamine (NH₂Cl) or chlorine, which is preferably used in the presence of FeCl₃. The chlorination can be carried out in analogy to the method described in Synthesis 1987, 1987, 683-688, Tetrahedron 66(36), 2010, 7279-7287, J. Org. Chem. 59(4), 1994, 914-921, J. Org. Chem. 63, 1998, 4878-4888 or J. Chem Soc. 1938, 2114-2117. The chlorination is generally carried out in a solvent. Suitable solvents are for example ethers, such as diethylether, dipropylether, methyl tert-butylether, methyl isobutylether, ethylenegylcol dimethylether, tetrahydrofuran or dioxane. The reaction temperature can vary over wide ranges and is generally from 0°C to the boiling point of the reaction mixture (if a solvent is used). The chlorinated compound is then reacted without isolation with ammonia or ammonium hydroxide. If anhydrous ammonia is used, the reaction is generally carried out at from -78 to -33°C. If aqueous ammonia or ammonium hydroxide is used, the reaction can also be carried out at higher temperatures, such as 0 to 25°C. The reaction is generally carried out in a solvent. Suitable solvents are for example the above-listed ethers, among which the water-miscible ethers, such as THF and dioxane, are preferred. In general, the chlorinated compound is dissolved in a solvent to which ammonia or ammonium hydroxide is added. The reaction can be carried out as described, for example, in Synthesis, 1987, 8, 683-688. The chlorination/amination can also be carried as a one pot reaction. For example, the thiol **3** is reacted simultaneously with a chlorinating agent (such as NCS or aqueous sodium hypochlorite) and anhydrous or aqueous ammonia in ethereal solvents (such as THF or Et₂O) or water. Preferred is the reaction with NCS in a mixture of THF and anhydrous liquid ammonia at -33°C. For instance, a solution of the thiol **3** in THF is added to a solution of NCS (N-chlorosuccinimide) in THF/liquid ammonia at -78°C. The solution is warmed to -30°C and stirred until the ammonia has evaporated. Alternatively, at 0°C, a solution of the sodium thiolate (NaSR) in water is added to a mixture of aqueous ammonia (25%) and aqueous sodium hypochlorite (1 N). The one pot chlorination/amination reaction can be carried out as described, for example, in Tetrahedron 2010, 66, 7279-7287 or in J. Org. Chem. 1994, 59, 914-921.

Compound **2** can virtually not be isolated as it generally reacts spontaneously in a ring-closing reaction to compound **1.**

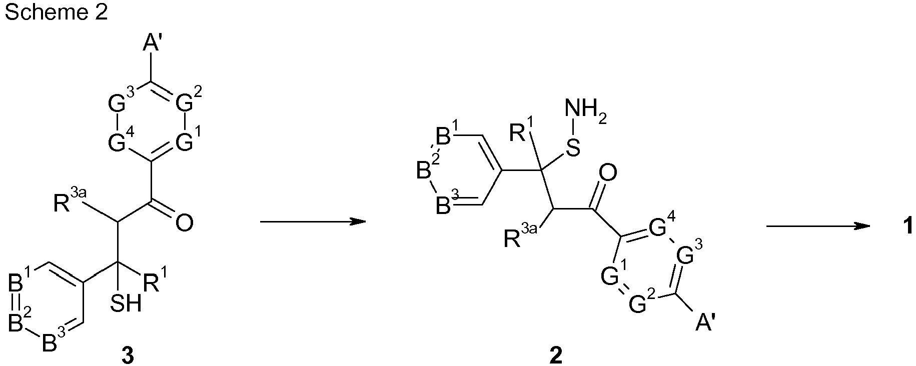

The compound of formula **3** can be prepared by reacting a compound of formula **4** 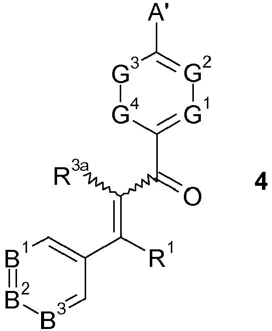 with a sulfur source. Suitable sulfur sources are for example H₂S, metal hydrogen sulfides, such as NaSH or KSH, metal sulfides, such as Na₂S, K₂S Li₂S, Cu₂S, MgS, CaS, CuS, FeS and the like, ammonium sulfide [(NH₄)₂S], tetraalkylammonium sulfides (R₄NSH), such as tetramethylammonium sulfide, tetraethylammonium sulfide, tetrapropylammonium sulfide and the like, or bistrimethylsilyl sulfide. H₂S as a sulfur source is generally used in the presence of a base, such as Na₂CO₃, K₂CO₃, Cs₂CO₃, sodium acetate, potassium acetate, cesium acetate, amines, such as diethylamine, dipropylamine, triethylamine, diisopropylethylamine and the like, or basic nitrogen-containing heterocycles, such as pyrrolidine, piperidine, piperazine, pyridine, lutidine and the like. Alternatively, H₂S as a sulfur source can be used in the presence of a Lewis acid, such as AlCl₃ or FeCl₃. The reaction of compound **4** with a sulfur source is generally carried out in a solvent, suitable solvents being for example chlorinated alkanes, such as methylene chloride or chloroform, and aromatic solvents, such as benzene, toluene, the xylenes, chlorobenzene or dichlorobenzene. The reaction temperature can vary over a wide range, such as -78°C to room temperature. In general, compound **4** is dissolved in a solvent, optionally cooled, then the base (if used) and subsequently the sulfur source is added. The compound **4** can alternatively be reacted with a sulfur source which provides a compound **3** which is protected at the thiol group SH by a protective group (S-PG). This is advantageous if compound **3** is for example subjected to harsher purification conditions or is derivatized, e.g. for converting the precursor group A' into a group A or for modifying group A' at this stage. Moreover, purification of the protected product is easier. Suitable sulfuration reagents which give such protected thiols are for example thiourea (NH₂-C(=S)-NH₂), optionally substituted benzyl thiols, such as benzylthiol, o- or p-methoxy-benzylthiol, o- or p-hydroxybenzylthiol, o- or p-acetoxybenzylthiol, o- or p-nitrobenzylthiol or 2,4,6-trimethylbenzylthiol, pyridin-4-yl-methylthiol, quinolin-2-yl-methylthiol, benzyl metal sulfides, such as sodium benzylsulfide, phenylthiol, 2,4-dinitrophenylthiol, tritylthiol, tert-butylthiol, compounds of formula R-C(=O)-NH-CH₃-SH, wherein R is methyl, tert-butyl, allyl, phenyl or benzyl, 2-trimethylsilanyl-ethanethiol, 2-(2,4-dinitrophenyl)-ethanethiol, 2-phenylsulfonyl-ethanethiol, acylated thiols, such as methylcarbonylthiol or phenylcarbonylthiol, and thiocarbamates R-NH-C(=O)-SH, wherein R is e.g. methyl or ethyl. The benzyl and alkyl thiols are generally used in the presence of a base, such as sodium hydroxide, potassium hydroxide, sodium phosphate, potassium phosphate, sodium hydrogenphosphate, potassium hydrogenphosphate, sodium carbonate, potassium carbonate, caesium carbonate, sodium hydride, potassium hydride, lithium diisopropyl amide (LDA), sodium methanolate, sodium ethanolate, potassium tert-butoxide, aqueous sodium tetraborate, n-butyllithium, tert-butylithium, tetrabutylammoniumfluoride (TBAF), NaHMDS and the like, or in the presence of a Lewis or Bronsted acid, such as FeCl₃, Zn(ClO₄)₂, Cu(BF₄)₂, HBF₄ or HClO₄. The reaction is generally carried out in a solvent, suitable solvents being for example chlorinated alkanes, such as methylene chloride or chloroform, and ethers, such as diethylether, dipropylether, methyl tert-butylether, methyl isobutylether, ethylenegylcol dimethylether, tetrahydrofuran (THF) or dioxane and the like. The reaction temperature can vary over a wide range, such as from -25°C to the boiling point of the reaction mixture. The acylated thiols can be reacted neat or in a solvent, suitable solvents being for example chlorinated alkanes, such as methylene chloride or chloroform, and ethers, such as diethylether, dipropylether, methyl tert-butylether, methyl isobutylether, ethylenegylcol dimethylether, tetrahydrofuran (THF) or dioxane and the like. They can be used with or without a base. The S-protected compound **3** can then be deprotected to the free thiol **3** under conditions generally known for the respective protective group, such as described, for example, in Peter G. M. Wuts, Theodora Greene, Protective Groups in Organic Synthesis, 4th edition, John Wiley & Sons, Inc., 2007, Chapter 6.

Compound **4** can be prepared in analogy to the method described in EP-A-2172462.

Compounds **1** (in which R^{3b} is not necessarily hydrogen) can be prepared alternatively by reacting a compound of formula **6** with an amination agent to a compound of formula **5,** which reacts spontaneously to the compound **1,** as shown in scheme 3. The reaction can be carried out in analogy to that of compounds **3** and **2.**

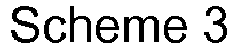

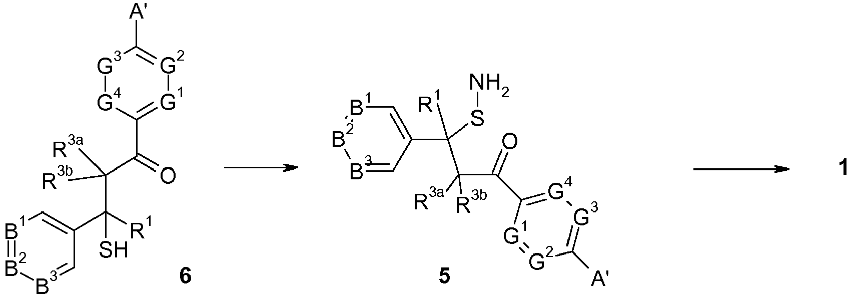

The compound of formula **6** can be obtained by reacting a compound of formula **7** with a compound of formula **8.**

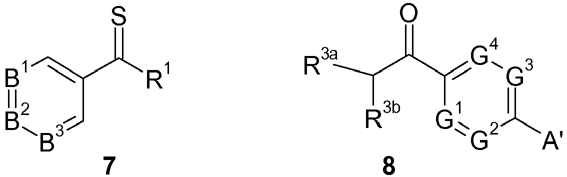

The reaction is preferably carried out as a Mukaiyama aldol reaction. To this purpose, the trialklysilyl-enolate derivative of **8** is reacted with **7** in the presence of a Lewis acid, such as TiCl₄ or BF₃[O(C₂H₅)₂]. Alternatively, the reaction can be carried out in the presence of a strong base, such as lithium diisopropylamide (LDA), sodium bistrimethylsilylamide (sodium hexamethyldisilazide; NaHMDS) and amines, such as triethylamine, tripropylamine or diisopropylethylamine. The reaction is generally carried out in a solvent. If a lithium or sodium base is used, the solvent is suitably an ether, such as diethylether, dipropylether, methyl tert-butylether, methyl isobutylether, ethylenegylcol dimethylether, tetrahydrofuran (THF) or dioxane and the like. Suitable reaction temperatures range from -78 to 25°C. If an amine base is used, the solvent is suitably an ether, such as diethylether, dipropylether, methyl tert-butylether, methyl isobutylether, ethylenegylcol dimethylether, tetrahydrofuran (THF) or dioxane, or an alkane, such as pentane, hexane or heptane. Suitable reaction temperatures range from 25 to 100°C.

The compound of formula **7** can be obtained by reacting a compound of formula **9** with a sulfuration agent, such as Lawesson's reagent or P₂S₅.

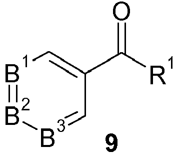

The reaction is generally carried out in a solvent, suitable solvents being for example aromatic solvents, such as benzene, toluene, the xylenes, chlorobenzene or dichlorobenzene, ethers, such as diethylether, dipropylether, methyl tert-butylether, methyl isobutylether, ethylenegylcol dimethylether, tetrahydrofuran (THF) or dioxane, and hexamethyl phosphoric acid triamide (HMPA). The reaction is generally carried out at a temperature of from 25°C to the boiling point of the reaction mixture.

Compounds of formula I wherein R¹ is CF₃ can moreover be prepared by reacting a compound of formula **10** with a fluorinating agent and, if necessary (i.e. if A' is a precursor of A), converting the group A' into a group A.

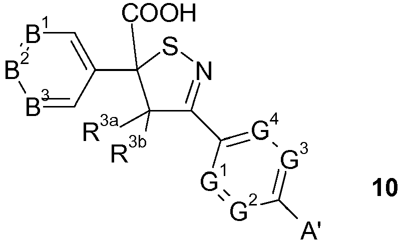

Suitable fluorinating agents are, for example, SF₄, preferably in combination with HF or BF₃[O(C₂H₅)₂], phenylsulfur trifluoride (Ph-SF₃), preferably in combination with HF and pyridine, 4-tert-butyl-2,6-dimethylphenylsulfur trifluoride ("Fluoled"), and bis(2-methoxyethyl)aminosulfur trifluoride [(CH₃OCH₂CH₂)₂NSF₃]. Among these, preference is given to SF₄ in combination with HF. If SF₄ in combination with HF is used, the reaction is carried out neat, i.e. without any further solvent. The reaction is generally carried out under elevated pressure stemming from the reactants, e.g. at a pressure of from 2 to 10 bar, preferably from 5 to 8 bar. The reaction temperature can vary over wide ranges, such as from 25 to 120°C, preferably from 60 to 100°C.

Alternatively, fluorination can be carried out by a two step method, in which the carboxyl group on the isothiazoline ring is first converted into a CCl₃ group, and this is subsequently fluorinated to the CF₃ group. The conversion of the COOH group to the CCl₃ group is preferably carried out by reacting the compound VI with PCl₅ and phenyl-phosphoroxy dichloride (Ph-P(=O)Cl₂). The reaction can be carried out neat, i.e. without any further solvent. Suitably, the reaction is carried out at elevated temperatures, for example at from 50°C to reflux and preferably at reflux. Fluorination agents for converting the CCl₃ group into a CF₃ group are those mentioned above, and further HF and HF in combination with SbCl₅ and HF in combination with Cl₂ and SbF₃. The reaction can be carried out neat, i.e. without any further solvent. The reaction temperature can vary over wide ranges, for examples from 25 to 300°C, preferably from 50 to 200°C and in particular from 80 to 120°C. If the fluorination agent is HF or HF in combination with a further agent, the reaction generally takes place at the pressure stemming from HF and ranging generally from 2 to 10 bar, preferably from 5 to 8 bar.

The compound of formula **10** is preferably obtained by hydrolyzing a compound of formula **11,** wherein R is C₁-C₄-alkyl.

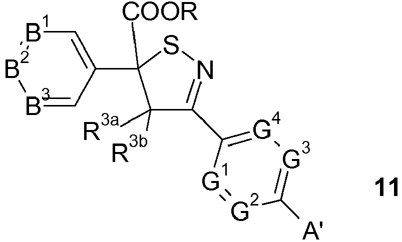

Hydrolysis can be carried out by any suitable means known for hydrolyzing ester groups, such as acidic conditions, e.g. using hydrochloric acid, hydrobromic acid, sulfuric acid, trifluoroacetic acid, etc., or by basic conditions, e.g. using an alkali metal hydroxide, such as LiOH, NaOH or KOH, or an alkali metal carbonate, such as sodium or potassium carbonate.

The compound of formula **11** can in turn be obtained by reacting a compound **12** with a compound **13**

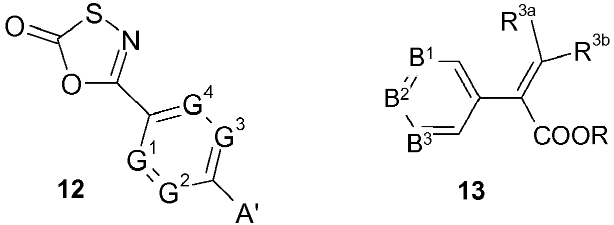

The reaction is carried out at elevated temperature, e.g. at from 90 to 200°C, preferably from 100 to 180°C and in particular from 120 to 160°C, e.g. at about 140°C.

The compound of formula **12** can in turn be obtained by reacting a compound **15** with a compound **16.**

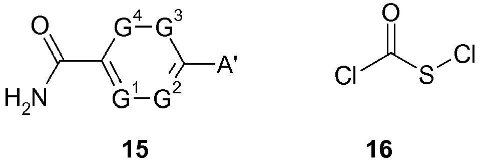

The reaction is generally carried out in a solvent, suitable solvents being for example aromatic solvents, such as benzene, toluene, the xylenes, chlorobenzene and dichlorobenzene. The reaction temperature is preferably from 80 to 140°C, more preferably from 100 to 120°C.

Compounds of formula I wherein however R¹ is CF₃ can moreover be prepared by reacting a compound of formula **12** as defined above with a compound of formula **14** and, if necessary, converting the group A' into a group A.

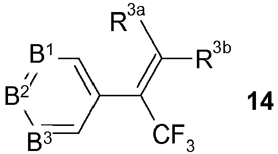

The reaction is carried out at elevated temperature, e.g. at from 90 to 200°C, preferably from 100 to 180°C and in particular from 120 to 160°C, e.g. at about 140°C.

Compounds I', in which A' is a precursor of A can be converted as shown below.

Compounds I', in which A' is Cl, Br, I or -OSO₂-R^{z1}, where R^{z1} is as defined above, can be converted to compounds I wherein A is a group C(=W)N(R⁵R⁶), by reaction with carbon monoxide and a hydride source, such as triethylsilane, in the presence of a transition metal complex catalyst, preferably a palladium catalyst, to a carbonyl compound **17.** This reaction converts the starting group A' into a carbonyl group-C(=O)H.

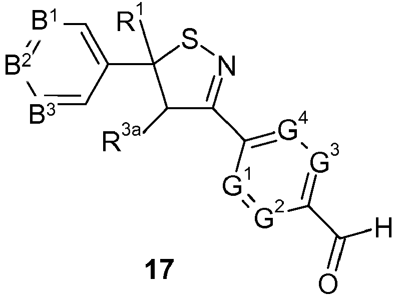

The aldehyde **17** can also be obtained by reducing the ester **18** (see below; R = C₁-C₄-alkyl) with diisobutylaluminium hydride (DIBAL-H) either directly to the aldehyde **17** or via the corresponding alcohol, which is then oxidized to the aldehyde.

For obtaining compounds I, such carbonyl compounds **17** are then reacted with a cyclic amine (derivative) NH(R⁵)R⁶. Alternatively, the compound I', in which A' is Cl, Br, I or -OSO₂-R^{z1}, where R^{z1} is as defined above, can be reacted in a one pot reaction with carbon monoxide and hydrogen in the presence of a transition metal complex catalyst and the cyclic amine NH(R⁵)R⁶.

Alternatively, compounds I', in which A' is Br or Cl can be converted to compounds I wherein A is a group C(=W)N(R⁵R⁶), by Pd-catalyzed aminocarbonylation in the sense of a Heck-carbonylation. The arylhalide compound I' is reacted with carbon monoxide and the cyclic amine NH(R⁵)R⁶ in the presence of a Pd catalyst, such as palladium(II) acetate in the presence of a phosphine ligand such as Xanthos (4,5-bis(diphenylphosphino)-9,9-dimethylxanthene). Usually, the reaction is conducted in the presence of a base. Suitable bases are alkali metal carbonates such as sodium carbonate or potassium carbonate or alkali metal phosphates such as potassium phosphate or sodium phenoxide.

Compounds I, wherein W is O can be prepared by reacting a compound I' wherein A' is Cl, Br, I or triflate with carbon monoxide in the presence of a palladium catalyst and an alcohol ROH, wherein R is C₁-C₄-alkyl, to a compound of formula **18.** Suitable palladium catalysts are for example those described in PCT/EP 2011/060388.

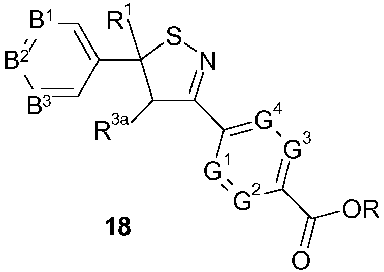

This ester is then hydrolyzed to the respective carboxylic acid, which is the reacted under standard amidation conditions with a cyclic amine NHR⁵R⁶. Hydrolyzation can be carried out under standard conditions, e.g. under acidic conditions using for example hydrochloric acid, sulfuric acid or trifluoroacetic acid, or under basic conditions using for example an alkali metal hydroxide, such as LiOH, NaOH or KOH. Amidation is preferably carried out by activation of the carboxylic acids with oxalylchloride [(COCl)₂] or thionylchloride (SOCl₂) to the respective acid chlorides, followed by reaction with an amine NHR⁵R⁶. Alternatively, amidation is carried out in the presence of a coupling reagent. Suitable coupling reagent (activators) are well known and are for instance selected from carbodiimides, such as DCC (dicyclohexylcarbodiimide) and DCI (diisopropylcarbodiimide), benzotriazol derivatives, such as HATU (O-(7-azabenzotriazol-1-yl)-N,N,N',N'-tetramethyluronium hexafluorophosphate), HBTU ((O-benzotriazol-1-yl)-N,N,N',N'-tetramethyluronium hexafluorophosphate) and HCTU (1H-benzotriazolium-1-[bis(dimethylamino)methylene]-5-chloro tetrafluoroborate) and phosphonium-derived activators, such as BOP ((benzotriazol-1-yloxy)-tris(dimethylamino)phosphonium hexafluorophosphate), Py-BOP ((benzotriazol-1-yloxy)-tripyrrolidinphosphonium hexafluorophosphate) and Py-BrOP (bromotripyrrolidinphosphonium hexafluorophosphate). Generally, the activator is used in excess. The benzotriazol and phosphonium coupling reagents are generally used in a basic medium.

Compounds I, wherein W is S, can be prepared by reacting the corresponding oxo-compound (W is O) with Lawesson's reagent (CAS 19172-47-5), see for example Jesberger et al., Synthesis, 2003, 1929-1958 and references therein. Solvents such as HMPA or THF at an elevated temperature such as 60°C to 100°C can be used. Preferred reaction conditions are THF at 65°C.

As a rule, the compounds of formula (I) including their stereoisomers, salts, and N-oxides, and their precursors in the synthesis process, can be prepared by the methods described above. If individual compounds can not be prepared via the above-described routes, they can be prepared by derivatization of other compounds (I) or the respective precursor or by customary modifications of the synthesis routes described. For example, in individual cases, certain compounds of formula (I) can advantageously be prepared from other compounds of formula (I) by derivatization, e.g. by ester hydrolysis, amidation, esterification, ether cleavage, olefination, reduction, oxidation and the like, or by customary modifications of the synthesis routes described.

The reaction mixtures are worked up in the customary manner, for example by mixing with water, separating the phases, and, if appropriate, purifying the crude products by chromatography, for example on alumina or on silica gel. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or trituration.

Due to their excellent activity, the compounds of the present invention may be used for controlling invertebrate pests.

Accordingly, the present invention also provides a non-therapeutic method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of the present invention or a composition as defined above. The invention also relates to a compound of the invention, a stereoisomer and/or an agriculturally or veterinarily acceptable salt thereof for use in combating invertebrate pests

Preferably, the method of the invention serves for protecting plant propagation material (such as seed) and the plant which grows therefrom from invertebrate pest attack or infestation and comprises treating the plant propagation material (such as seed) with a pesticidally effective amount of a compound of the present invention as defined above or with a pesticidally effective amount of an agricultural composition as defined above and below. The method of the invention is not limited to the protection of the "substrate" (plant, plant propagation materials, soil material etc.) which has been treated according to the invention, but also has a preventive effect, thus, for example, according protection to a plant which grows from a treated plant propagation materials (such as seed), the plant itself not having been treated.

Alternatively preferably, the method of the invention serves for protecting plants from attack or infestation by invertebrate pests, which method comprises treating the plants with a pesticidally effective amount of at least one compound of the invention, a stereoisomer thereof and/or at least one agriculturally acceptable salt thereof.

In the sense of the present invention, "invertebrate pests" are preferably selected from arthropods and nematodes, more preferably from harmful insects, arachnids and nematodes, and even more preferably from insects, acarids and nematodes. In the sense of the present invention, "invertebrate pests" are most preferably insects.

The invention further provides an agricultural composition for combating invertebrate pests, which comprises such an amount of at least one compound according to the invention and at least one inert liquid and/or solid agronomically acceptable carrier that has a pesticidal action and, if desired, at least one surfactant.

Such a composition may comprise a single active compound of the present invention or a mixture of several active compounds of the present invention. The composition according to the present invention may comprise an individual isomer or mixtures of isomers or a salt as well as individual tautomers or mixtures of tautomers.

The compounds of the present invention, including their salts, stereoisomers and tautomers, are in particular suitable for efficiently controlling arthropodal pests such as arachnids, myriapedes and insects as well as nematodes. They are especially suitable for efficiently combating or controlling the following pests:
insects from the order of the lepidopterans (Lepidoptera), for example Acronicta major, Adoxophyes orana, Aedia leucomelas, Agrotis spp. such as Agrotis fucosa, Agrotis segetum, Agrotis ypsilon; Alabama argillacea, Anticarsia gemmatalis, Anticarsia spp., Argyresthia conjugella, Autographa gamma, Barathra brassicae, Bucculatrix thurberiella, Bupalus piniarius, Cacoecia murinana, Cacoecia podana, Capua reticulana, Carpocapsa pomonella, Cheimatobia brumata, Chilo spp. such as Chilo suppressalis; Choristoneura fumiferana, Choristoneura occidentalis, Cirphis unipuncta, Clysia ambiguella, Cnaphalocerus spp., Cydia pomonella, Dendrolimus pini, Diaphania nitidalis, Diatraea grandiosella, Earias insulana, Elasmopalpus lignosellus, Ephestia cautella, Ephestia kuehniella, Eupoecilia ambiguella, Euproctis chrysorrhoea, Euxoa spp., Evetria bouliana, Feltia spp. such as Feltia subterranean; Galleria mellonella, Grapholitha funebrana, Grapholitha molesta, Helicoverpa spp. such as Helicoverpa armigera, Helicoverpa zea; Heliothis spp. such as Heliothis armigera, Heliothis virescens, Heliothis zea; Hellula undalis, Hibernia defoliaria, Hofmannophila pseudospretella, Homona magnanima, Hyphantria cunea, Hyponomeuta padella, Hyponomeuta malinellus, Keiferia lycopersicella, Lambdina fiscellaria, Laphygma spp. such as Laphygma exigua; Leucoptera coffeella, Leucoptera scitella, Lithocolletis blancardella, Lithophane antennata, Lobesia botrana, Loxagrotis albicosta, Loxostege sticticalis, Lymantria spp. such as Lymantria dispar, Lymantria monacha; Lyonetia clerkella, Malacosoma neustria, Mamestra spp. such as Mamestra brassicae; Mocis repanda, Mythimna separata, Orgyia pseudotsugata, Oria spp., Ostrinia spp. such as Ostrinia nubilalis; Oulema oryzae, Panolis flammea, Pectinophora spp. such as Pectinophora gossypiella; Peridroma saucia, Phalera bucephala, Phthorimaea spp. such as Phthorimaea operculella; Phyllocnistis citrella, Pieris spp. such as Pieris brassicae, Pieris rapae; Plathypena scabra, Plutella maculipennis, Plutella xylostella, Prodenia spp., Pseudaletia spp., Pseudoplusia includens, Pyrausta nubilalis, Rhyacionia frustrana, Scrobipalpula absoluta, Sitotroga cerealella, Sparganothis pilleriana, Spodoptera spp. such as Spodoptera frugiperda, Spodoptera littoralis, Spodoptera litura; Thaumatopoea pityocampa, Thermesia gemmatalis, Tinea pellionella, Tineola bisselliella, Tortrix viridana, Trichoplusia spp. such as Trichoplusia ni; Tuta absoluta, and Zeiraphera canadensis,
beetles (Coleoptera), for example Acanthoscehdes obtectus, Adoretus spp., Agelastica alni, Agrilus sinuatus, Agriotes spp. such as Agriotes fuscicollis, Agriotes lineatus, Agriotes obscurus; Amphimallus solstitialis, Anisandrus dispar, Anobium punctatum, Anomala rufocuprea, Anoplophora spp. such as Anoplophora glabripennis; Anthonomus spp. such as Anthonomus grandis, Anthonomus pomorum; Anthrenus spp., Aphthona euphoridae, Apogonia spp., Athous haemorrhoidalis, Atomaria spp. such as Atomaria linearis; Attagenus spp., Aulacophora femoralis, Blastophagus piniperda, Blitophaga undata, Bruchidius obtectus, Bruchus spp. such as Bruchus lentis, Bruchus pisorum, Bruchus rufimanus; Byctiscus betulae, Callosobruchus chinensis, Cassida nebulosa, Cerotoma trifurcata, Cetonia aurata, Ceuthorhynchus spp. such as Ceuthorrhynchus assimilis, Ceuthorrhynchus napi; Chaetocnema tibialis, Cleonus mendicus, Conoderus spp. such as Conoderus vespertinus; Cosmopolites spp., Costelytra zealandica, Crioceris asparagi, Cryptorhynchus lapathi, Ctenicera ssp. such as Ctenicera destructor; Curculio spp., Dectes texanus, Dermestes spp., Diabrotica spp. such as Diabrotica 12-punctata Diabrotica speciosa, Diabrotica longicornis, Diabrotica semipunctata, Diabrotica virgifera; Epilachna spp. such as Epilachna varivestis, Epilachna vigintioctomaculata; Epitrix spp. such as Epitrix hirtipennis; Eutinobothrus brasiliensis, Faustinus cubae, Gibbium psylloides, Heteronychus arator, Hylamorpha elegans, Hylobius abietis, Hylotrupes bajulus, Hypera brunneipennis, Hypera postica, Hypothenemus spp., lps typographus, Lachnosterna consanguinea, Lema bilineata, Lema melanopus, Leptinotarsa spp. such as Leptinotarsa decemlineata; Limonius californicus, Lissorhoptrus oryzophilus, Lissorhoptrus oryzophilus, Lixus spp., Lyctus spp. such as Lyctus bruneus; Melanotus communis, Meligethes spp. such as Meligethes aeneus; Melolontha hippocastani, Melolontha melolontha, Migdolus spp., Monochamus spp. such as Monochamus alternatus; Naupactus xanthographus, Niptus hololeucus, Oryctes rhinoceros, Oryzaephilus surinamensis, Otiorrhynchus sulcatus, Otiorrhynchus ovatus, Otiorrhynchus sulcatus, Oulema oryzae, Oxycetonia jucunda, Phaedon cochleariae, Phyllobius pyri, Phyllopertha horticola, Phyllophaga spp., Phyllotreta spp. such as Phyllotreta chrysocephala, Phyllotreta nemorum, Phyllotreta striolata; Phyllophaga spp., Phyllopertha horticola, Popillia japonica, Premnotrypes spp., Psylliodes chrysocephala, Ptinus spp., Rhizobius ventralis, Rhizopertha dominica, Sitona lineatus, Sitophilus spp. such as Sitophilus granaria, Sitophilus zeamais; Sphenophorus spp. such as Sphenophorus levis; Sternechus spp. such as Sternechus subsignatus; Symphyletes spp., Tenebrio molitor, Tribolium spp. such as Tribolium castaneum; Trogoderma spp., Tychius spp., Xylotrechus spp., and Zabrus spp. such as Zabrus tenebrioides,
flies, mosquitoes (Diptera), e.g. Aedes spp. such as Aedes aegypti, Aedes albopictus, Aedes vexans; Anastrepha ludens, Anopheles spp. such as Anopheles albimanus, Anopheles crucians, Anopheles freeborni, Anopheles gambiae, Anopheles leucosphyrus, Anopheles maculipennis, Anopheles minimus, Anopheles quadrimaculatus, Anopheles sinensis; Bibio hortulanus, Calliphora erythrocephala, Calliphora vicina, Cerafitis capitata, Ceratitis capitata, Chrysomyia spp. such as Chrysomya bezziana, Chrysomya hominivorax, Chrysomya macellaria; Chrysops atlanticus, Chrysops discalis, Chrysops silacea, Cochliomyia spp. such as Cochliomyia hominivorax; Contarinia spp. such as Contarinia sorghicola; Cordylobia anthropophaga, Culex spp. such as Culex nigripalpus, Culex pipiens, Culex quinquefasciatus, Culex tarsalis, Culex tritaeniorhynchus; Culicoides furens, Culiseta inornata, Culiseta melanura, Cuterebra spp., Dacus cucurbitae, Dacus oleae, Dasineura brassicae, Delia spp. such as Delia antique, Delia coarctata, Delia platura, Delia radicum; Dermatobia hominis, Drosophila spp., Fannia spp. such as Fannia canicularis; Gastraphilus spp. such as Gasterophilus intestinalis; Geomyza Tripunctata, Glossina fuscipes, Glossina morsitans, Glossina palpalis, Glossina tachinoides, Haematobia irritans, Haplodiplosis equestris, Hippelates spp., Hylemyia spp. such as Hylemyia platura; Hypoderma spp. such as Hypoderma lineata; Hyppobosca spp., Leptoconops torrens, Liriomyza spp. such as Liriomyza sativae, Liriomyza trifolii; Lucilia spp. such as Lucilia caprina, Lucilia cuprina, Lucilia sericata; Lycoria pectoralis, Mansonia titillanus, Mayetiola spp. such as Mayetiola destructor; Musca spp. such as Musca autumnalis, Musca domestica; Muscina stabulans, Oestrus spp. such as Oestrus ovis; Opomyza florum, Oscinella spp. such as Oscinella frit; Pegomya hysocyami, Phlebotomus argentipes, Phorbia spp. such as Phorbia antiqua, Phorbia brassicae, Phorbia coarctata; Prosimulium mixtum, Psila rosae, Psorophora columbiae, Psorophora discolor, Rhagoletis cerasi, Rhagoletis pomonella, Sarcophaga spp. such as Sarcophaga haemorrhoidalis; Simulium vittatum, Stomoxys spp. such as Stomoxys calcitrans; Tabanus spp. such as Tabanus atratus, Tabanus bovinus, Tabanus lineola, Tabanus similis; Tannia spp., Tipula oleracea, Tipula paludosa, and Wohlfahrtia spp.,
thrips (Thysanoptera), e.g. Baliothrips biformis, Dichromothrips corbetti, Dichromothrips ssp., Enneothrips flavens, Frankliniella spp. such as Frankliniella fusca, Frankliniella occidentalis, Frankliniella tritici; Heliothrips spp., Hercinothrips femoralis, Kakothrips spp., Rhipiphorothrips cruentatus, Scirtothrips spp. such as Scirtothrips citri; Taeniothrips cardamoni, Thrips spp. such as Thrips oryzae, Thrips palmi, Thrips tabaci;
termites (Isoptera), e.g. Calotermes flavicollis, Coptotermes formosanus, Heterotermes aureus, Heterotermes longiceps, Heterotermes tenuis, Leucotermes flavipes, Odontotermes spp., Reticulitermes spp. such as Reticulitermes speratus, Reticulitermes flavipes, Reticulitermes grassei, Reticulitermes lucifugus, Reticulitermes santonensis, Reticulitermes virginicus; Termes natalensis,
cockroaches (Blattaria - Blattodea), e.g. Acheta domesticus, Blatta orientalis, Blattella asahinae, Blattella germanica, Gryllotalpa spp., Leucophaea maderae, Locusta spp., Melanoplus spp., Periplaneta americana, Periplaneta australasiae, Periplaneta brunnea, Periplaneta fuligginosa, Periplaneta japonica,
bugs, aphids, leafhoppers, whiteflies, scale insects, cicadas (Hemiptera), e.g. Acrosternum spp. such as Acrosternum hilare; Acyrthosipon spp. such as Acyrthosiphon onobrychis, Acyrthosiphon pisum; Adelges laricis, Aeneolamia spp., Agonoscena spp., Aleurodes spp., Aleurolobus barodensis, Aleurothrixus spp., Amrasca spp., Anasa tristis, Antestiopsis spp., Anuraphis cardui, Aonidiella spp., Aphanostigma piri, Aphidula nasturtii, Aphis spp. such as Aphis fabae, Aphis forbesi, Aphis gossypii, Aphis grossulariae, Aphis pomi, Aphis sambuci, Aphis schneideri, Aphis spiraecola; Arboridia apicalis, Arilus critatus, Aspidiella spp., Aspidiotus spp., Atanus spp., Aulacorthum solani, Bemisia spp. such as Bemisia argentifolii, Bemisia tabaci; Blissus spp. such as Blissus leucopterus; Brachycaudus cardui, Brachycaudus helichrysi, Brachycaudus persicae, Brachycaudus prunicola, Brachycolus spp., Brevicoryne brassicae, Calligypona marginata, Calocoris spp., Campylomma livida, Capitophorus horni, Carneocephala fulgida, Cavelerius spp., Ceraplastes spp., Ceratovacuna lanigera, Cercopidae, Cerosipha gossypii, Chaetosiphon fragaefolii, Chionaspis tegalensis, Chlorita onukii, Chromaphis juglandicola, Chrysomphalus ficus, Cicadulina mbila, Cimex spp. such as Cimex hemipterus, Cimex lectularius; Coccomytilus halli, Coccus spp., Creontiades dilutus, Cryptomyzus ribis, Cryptomyzus ribis, Cyrtopeltis notatus, Dalbulus spp., Dasynus piperis, Dialeurades spp., Diaphorina spp., Diaspis spp., Dichelops furcatus, Diconocoris hewetti, Doralis spp., Dreyfusia nordmannianae, Dreyfusia piceae, Drosicha spp., Dysaphis spp. such as Dysaphis plantaginea, Dysaphis pyri, Dysaphis radicola; Dysaulacorthum pseudosolani, Dysdercus spp. such as Dysdercus cingulatus, Dysdercus intermedius; Dysmicoccus spp., Empoasca spp. such as Empoasca fabae, Empoasca solana; Eriosoma spp., Erythroneura spp., Eurygaster spp. such as Eurygaster integriceps; Euscelis bilobatus, Euschistus spp. such as Euschistuos heros, Euschistus impictiventris, Euschistus servus; Geococcus coffeae, Halyomorpha spp. such as Halyomorpha halys; Heliopeltis spp., Homalodisca coagulata, Horcias nobilellus, Hyalopterus pruni, Hyperomyzus lactucae, Icerya spp., Idiocerus spp., Idioscopus spp., Laodelphax striatellus, Lecanium spp., Lepidosaphes spp., Leptocorisa spp., Leptoglossus phyllopus, Lipaphis erysimi, Lygus spp. such as Lygus hesperus, Lygus lineolaris, Lygus pratensis; Macropes excavatus, Macrosiphum spp. such as Macrosiphum rosae, Macrosiphum avenae, Macrosiphum euphorbiae; Mahanarva fimbriolata, Megacopta cribraria, Megoura viciae, Melanaphis pyrarius, Melanaphis sacchari, Metcafiella spp., Metopolophium dirhodum, Miridae spp., Monellia costalis, Monelliopsis pecanis, Myzus spp. such as Myzus ascalonicus, Myzus cerasi, Myzus persicae, Myzus varians; Nasonovia ribis-nigri, Nephotettix spp. such as Nephotettix malayanus, Nephotettix nigropictus, Nephotettix parvus, Nephotettix virescens; Nezara spp. such as Nezara viridula; Nilaparvata lugens, Oebalus spp., Oncometopia spp., Orthezia praelonga, Parabemisia myricae, Paratrioza spp., Parlatoria spp., Pemphigus spp. such as Pemphigus bursarius; Pentomidae, Peregrinus maidis, Perkinsiella saccharicida, Phenacoccus spp., Phloeomyzus passerinii, Phorodon humuli, Phylloxera spp., Piesma quadrata, Piezodorus spp. such as Piezodorus guildinii, Pinnaspis aspidistrae, Planococcus spp., Protopulvinaria pyriformis, Psallus seriatus, Pseudacysta persea, Pseudaulacaspis pentagona, Pseudococcus spp. such as Pseudococcus comstocki; Psylla spp. such as Psylla mali, Psylla piri; Pteromalus spp., Pyrilla spp., Quadraspidiotus spp., Quesada gigas, Rastrococcus spp., Reduvius senilis, Rhodnius spp., Rhopalomyzus ascalonicus, Rhopalosiphum spp. such as Rhopalosiphum pseudobrassicas, Rhopalosiphum insertum, Rhopalosiphum maidis, Rhopalosiphum padi; Sagatodes spp., Sahlbergella singularis, Saissetia spp., Sappaphis mala, Sappaphis mali, Scaphoides titanus, Schizaphis graminum, Schizoneura lanuginosa, Scotinophora spp., Selenaspidus articulatus, Sitobion avenae, Sogata spp., Sogatella furcifera, Solubea insularis , Stephanitis nashi, Stictocephala festina, Tenalaphara malayensis, Thyanta spp. such as Thyanta perditor; Tibraca spp., Tinocallis caryaefoliae, Tomaspis spp., Toxoptera spp. such as Toxoptera aurantii; Trialeurodes spp. such as Trialeurodes vaporariorum; Triatoma spp., Trioza spp., Typhlocyba spp., Unaspis spp. such as Unaspis yanonensis; and Viteus vitifolii,
ants, bees, wasps, sawflies (Hymenoptera), e.g. Athalia rosae, Atta capiguara, Atta cephalotes, Atta cephalotes, Atta laevigata, Atta robusta, Atta sexdens, Atta texana, Bombus spp., Camponotus floridanus, Crematogaster spp., Dasymutilla occidentalis, Diprion spp., Dolichovespula maculata, Hoplocampa spp. such as Hoplocampa minuta, Hoplocampa testudinea; Lasius spp. such as Lasius niger, Linepithema humile, Monomorium pharaonis, Paravespula germanica, Paravespula pennsylvanica, Paravespula vulgaris, Pheidole megacephala, Pogonomyrmex barbatus, Pogonomyrmex californicus, Polistes rubiginosa, Solenopsis geminata, Solenopsis invicta, Solenopsis richteri, Solenopsis xyloni, Vespa spp. such as Vespa crabro, and Vespula squamosa,
crickets, grasshoppers, locusts (Orthoptera), e.g. Acheta domestica, Calliptamus italicus, Chortoicetes terminifera, Dociostaurus maroccanus, Gryllotalpa africana, Gryllotalpa gryllotalpa, Hieroglyphus daganensis, Kraussaria angulifera, Locusta migratoria, Locustana pardalina, Melanoplus bivittatus, Melanoplus femurrubrum, Melanoplus mexicanus, Melanoplus sanguinipes, Melanoplus spretus, Nomadacris septemfasciata, Oedaleus senegalensis, Schistocerca americana, Schistocerca gregaria, Tachycines asynamorus, and Zonozerus variegatus,
arachnids (Arachnida), such as acari,e.g. of the families Argasidae, Ixodidae and Sarcoptidae, such as Amblyomma spp. (e.g. Amblyomma americanum, Amblyomma variegatum, Amblyomma maculatum), Argas spp. (e.g. Argas persicus), Boophilus spp. (e.g. Boophilus annulatus, Boophilus decoloratus, Boophilus microplus), Dermacentor silvarum, Dermacentor andersoni, Dermacentor variabilis, Hyalomma spp. (e.g. Hyalomma truncatum), Ixodes spp. (e.g. Ixodes ricinus, Ixodes rubicundus, Ixodes scapularis, Ixodes holocyclus, Ixodes pacificus), Ornithodorus spp. (e.g. Ornithodorus moubata, Ornithodorus hermsi, Ornithodorus turicata), Ornithonyssus bacoti, Otobius megnini, Dermanyssus gallinae, Psoroptes spp. (e.g. Psoroptes ovis), Rhipicephalus spp. (e.g. Rhipicephalus sanguineus, Rhipicephalus appendiculatus, Rhipicephalus evertsi), Rhizoglyphus spp., Sarcoptes spp. (e.g. Sarcoptes scabiei), and Eriophyidae spp. such as Acaria sheldoni, Aculops spp. (e.g. Aculops pelekassi) Aculus spp. (e.g. Aculus schlechtendali), Epitrimerus pyri, Phyllocoptruta oleivora and Eriophyes spp. (e.g. Eriophyes sheldoni); Tarsonemidae spp. such as Hemitarsonemus spp., Phytonemus pallidus and Polyphagotarsonemus latus, Stenotarsonemus spp.; Tenuipalpidae spp. such as Brevipalpus spp. (e.g. Brevipalpus phoenicis); Tetranychidae spp. such as Eotetranychus spp., Eutetranychus spp., Oligonychus spp., Tetranychus cinnabarinus, Tetranychus kanzawai, Tetranychus pacificus, Tetranychus telarius and Tetranychus urticae; Bryobia praetiosa, Panonychus spp. (e.g. Panonychus ulmi, Panonychus citri), Metatetranychus spp. and Oligonychus spp. (e.g. Oligonychus pratensis), Vasates lycopersici; Araneida, e.g. Latrodectus mactans, and Loxosceles reclusa. And Acarus siro, Chorioptes spp., Scorpio maurus fleas (Siphonaptera), e.g. Ceratophyllus spp., Ctenocephalides felis, Ctenocephalides canis, Xenopsylla cheopis, Pulex irritans, Tunga penetrans, and Nosopsyllus fasciatus,
silverfish, firebrat (Thysanura), e.g. Lepisma saccharina and Thermobia domestica,
centipedes (Chilopoda), e.g. Geophilus spp., Scutigera spp. such as Scutigera coleoptrata;
millipedes (Diplopoda), e.g. Blaniulus guttulatus, Narceus spp.,
Earwigs (Dermaptera), e.g. forficula auricularia,
lice (Phthiraptera), e.g. Damalinia spp., Pediculus spp. such as Pediculus humanus capitis, Pediculus humanus corporis; Pthirus pubis, Haematopinus spp. such as Haematopinus eurysternus, Haematopinus suis; Linognathus spp. such as Linognathus vituli; Bovicola bovis, Menopon gallinae, Menacanthus stramineus and Solenopotes capillatus, Trichodectes spp.,
springtails (Collembola), e.g. Onychiurus ssp. such as Onychiurus armatus,

They are also suitable for controlling nematodes: plant parasitic nematodes such as root knot nematodes, Meloidogyne hapla, Meloidogyne incognita, Meloidogyne javanica, and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species such as Aphelenchoides besseyi ; Sting nematodes, Belonolaimus longicaudatus and other Belonolaimus species; Pine nematodes, Bursaphelenchus lignicolus Mamiya et Kiyohara, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Lesion nematodes, Pratylenchus brachyurus, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species such as Tylenchulus semipenetrans; Dagger nematodes, Xiphinema species; and other plant parasitic nematode species.

Examples of further pest species which may be controlled by compounds of fomula (I) include: from the class of the Bivalva, for example, Dreissena spp.; from the class of the Gastropoda, for example, Arion spp., Biomphalaria spp., Bulinus spp., Deroceras spp., Galba spp., Lymnaea spp., Oncomelania spp., Succinea spp.; from the class of the helminths, for example, Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. such as Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti; from the order of the Isopoda, for example, Armadillidium vulgare, Oniscus asellus, Porcellio scaber; from the order of the Symphyla, for example, Scutigerella immaculata;

Further examples of pest species which may be controlled by compounds of formula (I) include: Anisoplia austriaca, Apamea spp., Austroasca viridigrisea, Baliothrips biformis, Caenorhabditis elegans, Cephus spp., Ceutorhynchus napi, Chaetocnema aridula, Chilo auricilius, Chilo indicus , Chilo polychrysus, Chortiocetes terminifera, Cnaphalocroci medinalis, Cnaphalocrosis spp., Colias eurytheme, Collops spp., Cornitermes cumulans, Creontiades spp., Cyclocephala spp., Dalbulus maidis, Deraceras reticulatum , Diatrea saccharalis, Dichelops furcatus, Dicladispa armigera , Diloboderus spp. such as Diloboderus abderus; Edessa spp., Epinotia spp., Formicidae, Geocoris spp., Globitermes sulfureus, Gryllotalpidae, Halotydeus destructor, Hipnodes bicolor, Hydrellia philippina, Julus spp., Laodelphax spp., Leptocorsia acuta , Leptocorsia oratorius , Liogenys fuscus, Lucillia spp., Lyogenys fuscus, Mahanarva spp., Maladera matrida, Marasmia spp., Mastotermes spp., Mealybugs, Megascelis ssp, Metamasius hemipterus, Microtheca spp., Mocis latipes, Murgantia spp., Mythemina separata , Neocapritermes opacus, Neocapritermes parvus, Neomegalotomus spp., Neotermes spp., Nymphula depunctalis, Oebalus pugnax, Orseolia spp. such as Orseolia oryzae; Oxycaraenus hyalinipennis, Plusia spp., Pomacea canaliculata, Procornitermes ssp, Procornitermes triacifer , Psylloides spp., Rachiplusia spp., Rhodopholus spp., Scaptocoris castanea, Scaptocoris spp., Scirpophaga spp. such as Scirpophaga incertulas , Scirpophaga innotata; Scotinophara spp. such as Scotinophara coarctata; Sesamia spp. such as Sesamia inferens, Sogaella frucifera, Solenapsis geminata, Spissistilus spp., Stalk borer, Stenchaetothrips biformis, Steneotarsonemus spinki, Sylepta derogata, Telehin licus, Trichostrongylus spp..

The compounds of the present invention, including their salts, stereoisomers and tautomers, are particularly useful for controlling insects, preferably sucking or piercing and chewing and biting insects such as insects from the genera Lepidoptera, Coleoptera and Hemiptera, in particular Lepidoptera, Coleoptera and true bugs.

The compounds of the present invention, including their salts, stereoisomers and tautomers, are moreover useful for controlling insects of the orders Thysanoptera, Diptera (especially flies, mosquitos), Hymenoptera (especially ants) and Isoptera (especially termites.

The compounds of the present invention, including their salts, stereoisomers and tautomers, are particularly useful for controlling insects of the orders Lepidoptera and Coleoptera.

The invention also relates to agrochemical compositions comprising an auxiliary and at least one compound I according to the invention.

An agrochemical composition comprises a pesticidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the species to be controlled, the treated cultivated plant or material, the climatic conditions and the specific compound I used.

The compounds I, their N-oxides and salts can be converted into customary types of agrochemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.

### ii) Dispersible concentrates (DC)

5-25 wt% of a compound I according to the invention and 1-10 wt% dispersant (e.g. polyvinylpyrrolidone) are dissolved in organic solvent (e.g. cyclohexanone) ad 100 wt%. Dilution with water gives a dispersion.

### iii) Emulsifiable concentrates (EC)

15-70 wt% of a compound I according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.

### iv) Emulsions (EW, EO, ES)

5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.

### vi) Water-dispersible granules and water-soluble granules (WG, SG)

50-80 wt% of a compound I according to the invention are ground finely with addition of dispersants and wetting agents (e.g. sodium lignosulfonate and alcohol ethoxylate) ad 100 wt%and prepared as water-dispersible or water-soluble granules by means of technical appliances (e.g. extrusion, spray tower, fluidized bed). Dilution with water gives a stable dispersion or solution of the active substance.

### vii) Water-dispersible powders and water-soluble powders (WP, SP, WS)

50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.

### viii) Gel (GW, GF)

In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.

### iv) Microemulsion (ME)

5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water ad 100 %. This mixture is stirred for 1 h to produce spontaneously a thermodynamically stable microemulsion.

### iv) Microcapsules (CS)

An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of a polyurea microcapsules. The monomers amount to 1-10 wt%. The wt% relate to the total CS composition.

### ix) Dustable powders (DP, DS)

1-10 wt% of a compound I according to the invention are ground finely and mixed intimately with solid carrier (e.g. finely divided kaolin) ad 100 wt%.

### x) Granules (GR, FG)

0.5-30 wt% of a compound I according to the invention is ground finely and associated with solid carrier (e.g. silicate) ad 100 wt%. Granulation is achieved by extrusion, spray-drying or the fluidized bed.

### xi) Ultra-low volume liquids (UL)

1-50 wt% of a compound I according to the invention are dissolved in organic solvent (e.g. aromatic hydrocarbon) ad 100 wt%.

The compositions types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treamtent (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e.g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e.g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required. When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate. In a further embodiment, either individual components of the composition according to the invention or partially premixed components, e.g. components comprising compounds I and/or active substances from the groups M) or F) (see below), may be mixed by the user in a spray tank and further auxiliaries and additives may be added, if appropriate.

In a further embodiment, either individual components of the composition according to the invention or partially premixed components, e. g. components comprising compounds I and/or active substances from the groups M.1 to M.UN.X or F.I to F.XII, can be applied jointly (e.g. after tank mix) or consecutively.

The following list M of pesticides, grouped according the Mode of Action Classification of the Insecticide Resistance Action Committee (IRAC), together with which the compounds according to the invention can be used and with which potential synergistic effects might be produced, is intended to illustrate the possible combinations, but not to impose any limitation:
M.1 Acetylcholine esterase (AChE) inhibitors from the class of
   M.1A carbamates, for example aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; or from the class of
   M.1B organophosphates, for example acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O- (methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon and vamidothion;
M.2. GABA-gated chloride channel antagonists such as:
   M.2A cyclodiene organochlorine compounds, as for example endosulfan or chlordane; or
   M.2B fiproles (phenylpyrazoles), as for example ethiprole, fipronil, flufiprole, pyrafluprole and pyriprole;
M.3 Sodium channel modulators from the class of
   M.3A pyrethroids, for example acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, meperfluthrin,metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin and transfluthrin; or
   M.3B sodium channel modulators such as DDT or methoxychlor;
M.4 Nicotinic acetylcholine receptor agonists (nAChR) from the class of
   M.4A neonicotinoids, for example acteamiprid, chlothianidin, dinotefuran, imidacloprid, nitenpyram, thiacloprid and thiamethoxam; or the compounds
   M.4A.1: 1-[(6-chloro-3-pyridinyl)methyl]-2,3,5,6,7,8-hexahydro-9-nitro-(5S,8R)-5,8-Epoxy-1H-imidazo[1,2-a]azepine; or
   M.4A.2: 1-[(6-chloro-3-pyridyl)methyl]-2-nitro-1-[(E)-pentylideneamino]guanidine; or
   M4.A.3: 1-[(6-chloro-3-pyridyl)methyl]-7-methyl-8-nitro-5-propoxy-3,5,6,7-tetrahydro-2H-imidazo[1,2-a]pyridine;
   or M.4B nicotine.
M.5 Nicotinic acetylcholine receptor allosteric activators from the class of spinosyns, for example spinosad or spinetoram;
M.6 Chloride channel activators from the class of avermectins and milbemycins, for example abamectin, emamectin benzoate, ivermectin, lepimectin or milbemectin;
M.7 Juvenile hormone mimics, such as
   M.7A juvenile hormone analogues as hydroprene, kinoprene and methoprene; or others as M.7B fenoxycarb or M.7C pyriproxyfen;
M.8 miscellaneous non-specific (multi-site) inhibitors, for example
   M.8A alkyl halides as methyl bromide and other alkyl halides, or
   M.8B chloropicrin, or M.8C sulfuryl fluoride, or M.8D borax, or M.8E tartar emetic;
M.9 Selective homopteran feeding blockers, for example
   M.9B pymetrozine, or M.9C flonicamid;
M.10 Mite growth inhibitors, for example
   M.10A clofentezine, hexythiazox and diflovidazin, or M.10B etoxazole;
M.11 Microbial disruptors of insect midgut membranes, for example *bacillus thuringiensis* or *bacillus sphaericus* and the insecticdal proteins they produce such as *bacillus thuringiensis subsp. israelensis, bacillus sphaericus, bacillus thuringiensis subsp. aizawai, bacillus thuringiensis subsp. kurstaki* and *bacillus thuringiensis subsp. tenebrionis,* or the Bt crop proteins: Cry1Ab, Cry1Ac, Cry1Fa, Cry2Ab, mCry3A, Cry3Ab, Cry3Bb and Cry34/35Ab1;
M.12 Inhibitors of mitochondrial ATP synthase, for example
   M.12A diafenthiuron, or
   M.12B organotin miticides such as azocyclotin, cyhexatin or fenbutatin oxide, or M.12C propargite, or M.12D tetradifon;
M.13 Uncouplers of oxidative phosphorylation via disruption of the proton gradient, for example chlorfenapyr, DNOC or sulfluramid;
M.14 Nicotinic acetylcholine receptor (nAChR) channel blockers, for example nereistoxin analogues as bensultap, cartap hydrochloride, thiocyclam or thiosultap sodium;
M.15 Inhibitors of the chitin biosynthesis type 0, such as benzoylureas as for example bistrifluron, chlorfluazuron, diflubenzuron, flucycloxuron, flufenoxuron, hexaflumuron, lufenuron, novaluron, noviflumuron, teflubenzuron or triflumuron;
M.16 Inhibitors of the chitin biosynthesis type 1, as for example buprofezin;
M.17 Moulting disruptors, Dipteran, as for example cyromazine;
M.18 Ecdyson receptor agonists such as diacylhydrazines, for example methoxyfenozide, tebufenozide, halofenozide, fufenozide or chromafenozide;
M.19 Octopamin receptor agonists, as for example amitraz;
M.20 Mitochondrial complex III electron transport inhibitors, for example
   M.20A hydramethylnon, or M.20B acequinocyl, or M.20C fluacrypyrim;
M.21 Mitochondrial complex I electron transport inhibitors, for example
   M.21A METI acaricides and insecticides such as fenazaquin, fenpyroximate, pyrimidifen, pyridaben, tebufenpyrad or tolfenpyrad, or M.21B rotenone;
M.22 Voltage-dependent sodium channel blockers, for example
   M.22A indoxacarb, or M.22B metaflumizone, or M.22C 1-[(E)-[2-(4-cyanophenyl)-1-[3-(trifluoromethyl)phenyl]ethylidene]amino]-3-[4-(difluoromethoxy)phenyl]urea;
M.23 Inhibitors of the of acetyl CoA carboxylase, such as Tetronic and Tetramic acid derivatives, for example spirodiclofen, spiromesifen or spirotetramat;
M.24 Mitochondrial complex IV electron transport inhibitors, for example M.24A phosphine such as aluminium phosphide, calcium phosphide, phosphine or zinc phosphide, or M.24B cyanide.
M.25 Mitochondrial complex II electron transport inhibitors, such as beta-ketonitrile derivatives, for example cyenopyrafen or cyflumetofen;
M.28 Ryanodine receptor-modulators from the class of diamides, as for example flubendiamide, chlorantraniliprole (rynaxypyr®), cyantraniliprole (cyazypyr®), or the phthalamide compounds
   M.28.1: (R)-3-Chlor-N1-{2-methyl-4-[1,2,2,2-tetrafluor-1-(trifluormethyl)ethyl]phenyl}-N2-(1-methyl-2-methylsulfonylethyl)phthalamid and
   M.28.2: (S)-3-Chlor-N1-{2-methyl-4-[1,2,2,2-tetrafluor-1-(trifluormethyl)ethyl]phenyl}-N2-(1-methyl-2-methylsulfonylethyl)phthalamid, or the compound
   M.28.3: 3-bromo-N-{2-bromo-4-chloro-6-[(1-cyclopropylethyl)carbamoyl]phenyl}-1-(3-chlorpyridin-2-yl)-1H-pyrazole-5-carboxamide (proposed ISO name: cyclaniliprole), or the compound
   M.28.4: methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; or a compound selected from M.28.5a) to M.28.5l):
   M.28.5a) N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide;
   M.28.5b) N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide;
   M.28.5c) N-[4-chloro-2-[(di-2-propyl-lambda-4-sulfanylidene)carbamoyl]-6-methylphenyl]-2-(3-chloro-2-pyridyl)-5-(trifluoromethyl)pyrazole-3-carboxamide;
   M.28.5d) N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide;
   M.28.5e) N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide;
   M.28.5f) N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide;
   M.28.5g) N-[4-chloro-2-[(di-2-propyl-lambda-4-sulfanylidene)carbamoyl]-6-cyanophenyl]-2-(3-chloro-2-pyridyl)-5-(trifluoromethyl)pyrazole-3-carboxamide;
   M.28.5h) N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
   M.28.5i) N-[2-(5-amino-1,3,4-thiadiazol-2-yl)-4-chloro-6-methyl-phenyl]-5-bromo-2-(3-chloro-2-pyridyl)pyrazole-3-carboxamide;
   M.28.5j) 5-chloro-2-(3-chloro-2-pyridyl)-N-[2,4-dichloro-6-[(1-cyano-1-methylethyl)carbamoyl]phenyl]pyrazole-3-carboxamide;
   M.28.5k) 5-bromo-N-[2,4-dichloro-6-(methylcarbamoyl)phenyl]-2-(3,5-dichloro-2-pyridyl)pyrazole-3-carboxamide;
   M.28.5l) N-[2-(tert-butylcarbamoyl)-4-chloro-6-methyl-phenyl]-2-(3-chloro-2-pyridyl)-5-(fluoromethoxy)pyrazole-3-carboxamide; or a compound selected from
   M.28.6 N2-(1-cyano-1-methyl-ethyl)-N1-(2,4-dimethylphenyl)-3-iodo-phthalamide; or M.28.7 3-chloro-N2-(1-cyano-1-methyl-ethyl)-N1-(2,4-dimethylphenyl)phthalamide;
M.UN.X insecticidal active compounds of unknown or uncertain mode of action, as for example azadirachtin, amidoflumet, benzoximate, bifenazate, bromopropylate, chinomethionat, cryolite, dicofol, flufenerim, flometoquin, fluensulfone, flupyradifurone, piperonyl butoxide, pyridalyl, pyrifluquinazon, sulfoxaflor, pyflubumide or the compounds
   M.UN.X.1: 4-[5-(3,5-Dichloro-phenyl)-5-trifluoromethyl-4,5-dihydro-isoxazol-3-yl]-2-methyl-N-[(2,2,2-trifluoro-ethylcarbamoyl)-methyl]-benzamide, or the compound
   M.UN.X.2: cyclopropaneacetic acid, 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] ester, or the compound
   M.UN.X.3: 11-(4-chloro-2,6-dimethylphenyl)-12-hydroxy-1,4-dioxa-9-azadispiro[4.2.4.2]-tetradec-11-en-10-one, or the compound
   M.UN.X.4: 3-(4' -fluoro-2,4-dimethylbiphenyl-3-yl)-4-hydroxy-8-oxa-1-azaspiro[4.5]dec-3-en-2-one, or the compound
   M.UN.X.5: 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582); or
   M.UN.X.6; a compound selected from the group of
   M.UN.X.6a) (E/Z)-N-[1-[(6-chloro-3-pyridyl)methyl]-2-pyridylidene]-2,2,2-trifluoroacetamide;
   M.UN.X.6b) (E/Z)-N-[1-[(6-chloro-5-fluoro-3-pyridyl)methyl]-2-pyridylidene]-2,2,2-trifluoro-acetamide;
   M.UN.X.6c) (E/Z)-2,2,2-trifluoro-N-[1-[(6-fluoro-3-pyridyl)methyl]-2-pyridylidene]acetamide;
   M.UN.X.6d) (E/Z)-N-[1-[(6-bromo-3-pyridyl)methyl]-2-pyridylidene]-2,2,2-trifluoroacetamide;
   M.UN.X.6e) (E/Z)-N-[1-[1-(6-chloro-3-pyridyl)ethyl]-2-pyridylidene]-2,2,2-trifluoroacetamide;
   M.UN.X.6f) (E/Z)-N-[1-[(6-chloro-3-pyridyl)methyl]-2-pyridylidene]-2,2-difluoro-acetamide;
   M.UN.X.6g) (E/Z)-2-chloro-N-[1-[(6-chloro-3-pyridyl)methyl]-2-pyridylidene]-2,2-difluoro-acetamide;
   M.UN.X.6h) (E/Z)-N-[1-[(2-chloropyrimidin-5-yl)methyl]-2-pyridylidene]-2,2,2-trifluoroacetamide and
   M.UN.X.6i) (E/Z)-N-[1-[(6-chloro-3-pyridyl)methyl]-2-pyridylidene]-2,2,3,3,3-pentafluoro-propanamide.); or the compounds
   M.UN.X.7: 3-[3-chloro-5-(trifluoromethyl)phenyl]-4-oxo-1-(pyrimidin-5-ylmethyl)pyrido[1,2-a]pyrimidin-1-ium-2-olate; or
   M.UN.X.8: 8-chloro-N-[2-chloro-5-methoxyphenyl)sulfonyl]-6-trifluoromethyl)-imidazo[1,2-a]pyridine-2-carboxamide; or
   M.UN.X.9: 4-[5-(3,5-dichlorophenyl)-5-(trifluoromethyl)-4H-isoxazol-3-yl]-2-methyl-N-(1-oxothietan-3-yl)benzamide; or
   M.UN.X.10: 5-[3-[2,6-dichloro-4-(3,3-dichloroallyloxy)phenoxy]propoxy]-1H-pyrazole; or
   M.UN.X.11: 4-[5-(3,5-dichlorophenyl)-5-(trifluoromethyl)-4H-isoxazol-3-yl]-2-methyl-N-[2-oxo-2-(2,2,2-trifluoroethylamino)ethyl]benzamide; or
   M.UN.X.12: 4-[5-[3-chloro-5-(trifluoromethyl)phenyl]-5-(trifluoromethyl)-4H-isoxazol-3-yl]-N-[2-oxo-2-(2,2,2-trifluoroethylamino)ethyl]naphthalene-1-carboxamide.

The commercially available compounds of the group M listed above may be found in The Pesticide Manual, 15th Edition, C. D. S. Tomlin, British Crop Protection Council (2011) among other publications.

The quinoline derivative flometoquin is shown in WO2006/013896. The aminofuranone compounds flupyradifurone is known from WO 2007/115644. The sulfoximine compound sulfoxaflor is known from WO2007/149134. The acaricide pyflubumide is known from WO2007/020986. The isoxazoline compounds have been described: M.UN.X.1 in WO2005/085216, M.UN.X.9 in WO2013/050317, M.UN.X.11 in WO2005/085216 and M.UN.X. in WO2009/002809 and in WO2011/149749. The pyripyropene derivative M.UN.X.2 has been described in WO 2006/129714. The spiroketal-substituted cyclic ketoenol derivative M.UN.X.3 is known from WO2006/089633 and the biphenyl-substituted spirocyclic ketoenol derivative M.UN.X.4 from WO2008/067911. Finally triazoylphenylsulfide like M.UN.X.5 have been described in WO2006/043635 and biological control agents on basis of *bacillus firmus* in WO2009/124707. The neonicotionids 4A.1 is known from WO20120/069266 and WO2011/06946, the M.4.A.2 from WO2013/003977, the M4.A.3.from WO2010/069266.

The Metaflumizone analogue M.22C is described in CN 10171577. The phthalamides M.28.1 and M.28.2 are both known from WO 2007/101540. The anthranilamide M.28.3 has been described in WO2005/077934. The hydrazide compound M.28.4 has been described in WO 2007/043677. The anthranilamides M.28.5a) to M.28.5h) can be prepared as described in WO 2007/006670, WO2013/024009 and WO2013/024010, the anthranilamide M.28.5i) is described in WO2011/085575, the M.28.5j) in WO2008/134969, the M.28.5k) in US2011/046186 and the M.28.5l) in WO2012/034403. The diamide compounds M.28.6 and M.28.7 can be found in CN102613183.

The compounds M.UN.X.6a) to M.UN.X.6i) listed in M.UN.X.6 have been described in WO2012/029672. The mesoionic antagonist compound M.UN.X.7 was described in WO2012/092115, the nematicide M.UN.X.8 in WO2013/055584and the Pyridalyl-type analogue M.UN.X.10 in WO2010/060379.

Preferred additional pesticidally active ingredients are those selected from the IRAC group 1, the Acetylcholinesterase (AChE) inhibitors, herein from the group 1A (Carbamtes) Thiodicarb, Methomyl and Carbaryl, and from the group 1B(Organophosphates), especially Acephate, Chlorpyriphos and Dimethoate, from the group 2B, the fiproles, here especially ethiprole and fipronil, from the group 3, the pyrethroids, here especially lambda-cyhalothrin, alpha-cypermethrin or deltametrin, and from the group 4A, the neonicotinoids, here especially acetamiprid, clothianidin, dinotefuran, imidacloprid, nitenpyram, thiacloprid or thiomethoxam.

Especially combinations of compounds of the invention with fiproles, neonictinoids or pyrethroids may possibly exhibit synergistic control of stinkbugs (according to the Colby formula), in particular Euschistus, e.g. Euschistus heros.

The following list F of active substances, in conjunction with which the compounds according to the invention can be used, is intended to illustrate the possible combinations but does not limit them:
F.I) Respiration Inhibitors
   F.I-1) Inhibitors of complex III at Qo site:
      strobilurins: azoxystrobin, coumethoxystrobin, coumoxystrobin, dimoxystrobin, enestroburin, fluoxastrobin, kresoxim-methyl, metominostrobin, orysastrobin, picoxystrobin, pyraclostrobin, pyrametostrobin, pyraoxystrobin, pyribencarb, triclopyricarb/chlorodincarb, trifloxystrobin, 2-[2-(2,5-dimethyl-phenoxymethyl)-phenyl]-3-methoxy-acrylic acid methyl ester and 2 (2-(3-(2,6-dichlorophenyl)-1-methylallylideneaminooxymethyl)-phenyl)-2-methoxyimino-N methyl-acetamide; oxazolidinediones and imidazolinones: famoxadone, fenamidone;
   F.I-2) Inhibitors of complex II (e.g. carboxamides):
      carboxanilides: benodanil, benzovindiflupyr, bixafen, boscalid, carboxin, fenfuram, fenhexamid, fluopyram, flutolanil, furametpyr, isopyrazam, isotianil, mepronil, oxycarboxin, penflufen, penthiopyrad, sedaxane, tecloftalam, thifluzamide, tiadinil, 2-amino-4 methyl-thiazole-5-carboxanilide, N-(3',4',5' trifluorobiphenyl-2 yl)-3-difluoromethyl-1-methyl-1H-pyrazole-4 carboxamide (fluxapyroxad), N-(4'-trifluoromethylthiobiphenyl-2-yl)-3 difluoromethyl-1-methyl-1H pyrazole-4-carboxamide, N-(2-(1,3,3-trimethyl-butyl)-phenyl)-1,3-dimethyl-5 fluoro-1H-pyrazole-4 carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide;
   F.I-3) Inhibitors of complex III at Qi site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-di-oxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate, 3*S*,6*S*,7*R*,8*R*)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate;
   F.I-4) Other respiration inhibitors (complex I, uncouplers) diflumetorim; (5,8-difluoro-quinazolin-4-yl)-{2-[2-fluoro-4-(4-trifluoromethylpyridin-2-yloxy)-phenyl]-ethyl}-amine; tecnazen; ametoctradin; silthiofam; nitrophenyl derivates: binapacryl, dinobuton, dinocap, fluazinam, ferimzone, nitrthal-isopropyl,
      and including organometal compounds: fentin salts, such as fentin-acetate, fentin chloride or fentin hydroxide;
F.II) Sterol biosynthesis inhibitors (SBI fungicides)
   F.II-1) C14 demethylase inhibitors (DMI fungicides, e.g. triazoles, imidazoles) triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, paclobutrazole, penconazole, propiconazole, prothioconazole, simeconazole, tebuconazole, tetraconazole, triadimefon, triadimenol, triticonazole, uniconazole, 1-[*rel-*(2*S*;3*R*)-3-(2-chlorophenyl)-2-(2,4-difluorophenyl)-oxiranylmethyl]-5-thiocyanato-1H-[1,2,4]triazole, 2-[*rel*-(2*S*;3*R*)-3-(2-chlorophenyl)-2-(2,4-difluorophenyl)-oxiranylmethyl]-2H-[1,2,4]triazole-3-thiol; imidazoles: imazalil, pefurazoate, oxpoconazole, prochloraz, triflumizole; pyrimidines, pyridines and piperazines: fenarimol, nuarimol, pyrifenox, triforine, 1-[rel-(2*S*;3*R*)-3-(2-chlorophenyl)-2-(2,4-difluorophenyl)-oxiranylmethyl]-5-thiocyanato-1H-[1,2,4]triazole, 2-[rel-(2S;3R)-3-(2-chlorophenyl)-2-(2,4-difluorophenyl)-oxiranylmethyl]-2H-[1,2,4]triazole-3-thiol;
   F.II-2) Delta14-reductase inhitors (Amines, e.g. morpholines, piperidines) morpholines: aldimorph, dodemorph, dodemorph-acetate, fenpropimorph, tridemorph; piperidines: fenpropidin, piperalin; spiroketalamines: spiroxamine;
   F.II-3) Inhibitors of 3-keto reductase: hydroxyanilides: fenhexamid;
F.III) Nucleic acid synthesis inhibitors
   F.III-1) RNA, DNA synthesis
      phenylamides or acyl amino acid fungicides: benalaxyl, benalaxyl-M, kiralaxyl, metalaxyl, metalaxyl-M (mefenoxam), ofurace, oxadixyl;
      isoxazoles and iosothiazolones: hymexazole, octhilinone;
   F.III-2) DNA topisomerase inhibitors: oxolinic acid;
   F.III-3) Nucleotide metabolism (e.g. adenosin-deaminase), hydroxy (2-amino)-pyrimidines: bupirimate;
F.IV) Inhibitors of cell division and or cytoskeleton
   F.IV-1) Tubulin inhibitors: benzimidazoles and thiophanates: benomyl, carbendazim, fuberidazole, thiabendazole, thiophanate-methyl;
      triazolopyrimidines: 5-chloro-7 (4-methylpiperidin-1-yl)-6-(2,4,6-trifluorophenyl)-[1,2,4]triazolo[1,5 a]pyrimidine;
   F.IV-2) Other cell division inhibitors
      benzamides and phenyl acetamides: diethofencarb, ethaboxam, pencycuron, fluopicolide, zoxamide;
   F.IV-3) Actin inhibitors: benzophenones: metrafenone, pyriofenone;
F.V) Inhibitors of amino acid and protein synthesis
   F.V-1) Methionine synthesis inhibitors (anilino-pyrimidines)
      anilino-pyrimidines: cyprodinil, mepanipyrim, nitrapyrin, pyrimethanil;
   F.V-2) Protein synthesis inhibitors (anilino-pyrimidines)
      antibiotics: blasticidin-S, kasugamycin, kasugamycin hydrochloride-hydrate, mildiomycin, streptomycin, oxytetracyclin, polyoxine, validamycin A;
F.VI) Signal transduction inhibitors
   F.VI-1) MAP / Histidine kinase inhibitors (e.g. anilino-pyrimidines)
      dicarboximides: fluoroimid, iprodione, procymidone, vinclozolin;
      phenylpyrroles: fenpiclonil, fludioxonil;
   F.VI-2) G protein inhibitors: quinolines: quinoxyfen;
F.VII) Lipid and membrane synthesis inhibitors
   F.VII-1) Phospholipid biosynthesis inhibitors
      organophosphorus compounds: edifenphos, iprobenfos, pyrazophos;
      dithiolanes: isoprothiolane;
   F.VII-2) Lipid peroxidation: aromatic hydrocarbons: dicloran, quintozene, tecnazene, tolclofos-methyl, biphenyl, chloroneb, etridiazole;
   F.VII-3) Carboxyl acid amides (CAA fungicides)
      cinnamic or mandelic acid amides: dimethomorph, flumorph, mandiproamid, pyrimorph; valinamide carbamates: benthiavalicarb, iprovalicarb, pyribencarb, valifenalate and N-(1-(1-(4-cyano-phenyl)ethanesulfonyl)-but-2-yl) carbamic acid-(4-fluorophenyl) ester;
   F.VII-4) Compounds affecting cell membrane permeability and fatty acids: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone, carbamates: propamocarb, propamocarb-hydrochlorid,
   F.VII-5) fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone;
F.VIII) Inhibitors with Multi Site Action
   F.VIII-1) Inorganic active substances: Bordeaux mixture, copper acetate, copper hydroxide, copper oxychloride, basic copper sulfate, sulfur;
   F.VIII-2) Thio- and dithiocarbamates: ferbam, mancozeb, maneb, metam, methasulphocarb, metiram, propineb, thiram, zineb, ziram;
   F.VIII-3) Organochlorine compounds (e.g. phthalimides, sulfamides, chloronitriles): anilazine, chlorothalonil, captafol, captan, folpet, dichlofluanid, dichlorophen, flusulfamide, hexachlorobenzene, pentachlorphenole and its salts, phthalide, tolylfluanid, N-(4-chloro-2-nitro-phenyl)-N-ethyl-4-methyl-benzenesulfonamide;
   F.VIII-4) Guanidines and other: guanidine, dodine, dodine free base, guazatine, guazatine-acetate, iminoctadine, iminoctadine-triacetate, iminoctadine-tris(albesilate), 2,6-dimethyl-1H,5H-[1,4]dithiino[2,3-c:5,6-c']dipyrrole-1,3,5,7(2H,6H)-tetraone;
   F.VIII-5) Ahtraquinones: dithianon;
F.IX) Cell wall synthesis inhibitors
   F.IX-1) Inhibitors of glucan synthesis: validamycin, polyoxin B;
   F.IX-2) Melanin synthesis inhibitors: pyroquilon, tricyclazole, carpropamide, dicyclomet, fenoxanil;
F.X) Plant defence inducers
   F.X-1) Salicylic acid pathway: acibenzolar-S-methyl;
   F.X-2) Others: probenazole, isotianil, tiadinil, prohexadione-calcium;
      phosphonates: fosetyl, fosetyl-aluminum, phosphorous acid and its salts;
F.XI) Unknown mode of action:bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N methyl formamidine, N' (4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2 methyl-4-(3-trimethylsilanyl-propoxy)-phenyl)-N-ethyl-N-methyl formamidine, 2-{1-[2-(5-methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide,2-{1-[2-(5-methyl-3-trifluoromethyl-pyrazole-1-yl)-acetyl]-piperidin-4-yl}-thiazole-4-carboxylic acid methyl-(R)-1,2,3,4-tetrahydro-naphthalen-1-yl-amide, methoxy-acetic acid 6-tert-butyl-8-fluoro-2,3-dimethyl-quinolin-4-yl ester and N-Methyl-2-{1-[(5-methyl-3-trifluoromethyl-1H-pyrazol-1-yl)-acetyl]-piperidin-4-yl}-N-[(1*R*)-1,2,3,4-tetrahydronaphthalen-1-yl]-4-thiazolecarboxamide, 3-[5-(4-chloro-phenyl)-2,3-dimethyl-isoxazolidin-3 yl]-pyridine, pyrisoxazole, 5-amino-2-isopropyl-3-oxo-4-ortho-tolyl-2,3-dihydro-pyrazole-1 carbothioic acid *S*-allyl ester, N-(6-methoxy-pyridin-3-yl) cyclopropanecarboxylic acid amide, 5-chloro-1 (4,6-dimethoxy-pyrimidin-2-yl)-2-methyl-1H-benzoimidazole, 2-(4-chloro-phenyl)-N-[4-(3,4-dimethoxy-phenyl)-isoxazol-5-yl]-2-prop-2-ynyloxy-acetamide,.
F.XII) Growth regulators: abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat (mepiquat chloride), naphthaleneacetic acid, N 6-benzyladenine, paclobutrazol, prohexadione (prohexadione-calcium), prohydrojasmon, thidiazuron, triapenthenol, tributyl phosphorotrithioate, 2,3,5 tri iodobenzoic acid, trinexapac-ethyl and uniconazole;
F.XIII) Biological control agents
   *Ampelomyces quisqualis* (e.g. AQ 10® from Intrachem Bio GmbH & Co. KG, Germany), *Aspergillus flavus* (e.g. AFLAGUARD® from Syngenta, CH), *Aureobasidium pullulans* (e.g. BOTECTOR® from bio-ferm GmbH, Germany), *Bacillus pumilus* (e.g. NRRL Accession No. B-30087 in SONATA® and BALLAD® Plus from AgraQuest Inc., USA), *Bacillus subtilis* (e.g. isolate NRRL-Nr. B-21661 in RHAPSODY®, SERENADE® MAX and SERENADE® ASO from AgraQuest Inc., USA), *Bacillus subtilis* var. *amylolique-faciens* FZB24 (e.g. TAEGRO® from Novozyme Biologicals, Inc., USA), *Candida oleophila* I-82 (e.g. ASPIRE® from Ecogen Inc., USA), *Candida saitoana* (e.g. BIOCURE® (in mixture with lysozyme) and BIOCOAT® from Micro Flo Company, USA (BASF SE) and Arysta), Chitosan (e.g. ARMOUR-ZEN from BotriZen Ltd., NZ), *Clonostachys rosea* f. *catenulata,* also named *Gliocladium catenulatum* (e.g. isolate J1446: PRESTOP® from Verdera, Finland), *Coniothyrium minitans* (e.g. CONTANS® from Prophyta, Germany), *Cryphonectria parasitica* (e.g. *Endothia parasitica* from CNICM, France), *Cryptococcus albidus* (e.g. YIELD PLUS® from Anchor BioTechnologies, South Africa), *Fusarium oxysporum* (e.g. BIOFOX® from S.I.A.P.A., Italy, FUSACLEAN® from Natural Plant Protection, France), *Metschnikowia fructicola* (e.g. SHEMER® from Agrogreen, Israel), *Microdochium dimerum* (e.g. ANTIBOT® from Agrauxine, France), *Phlebiopsis gigantea* (e.g. ROTSOP® from Verdera, Finland), *Pseudozyma flocculosa* (e.g. SPORODEX® from Plant Products Co. Ltd., Canada), *Pythium oligandrum* DV74 (e.g. POLYVERSUM® from Remeslo SSRO, Biopreparaty, Czech Rep.), *Reynoutria sachlinensis* (e.g. REGALIA® from Marrone Biolnnovations, USA), *Talaromyces flavus* V117b (e.g. PROTUS® from Prophyta, Germany), *Trichoderma asperellum* SKT-1 (e.g. ECO-HOPE® from Kumiai Chemical Industry Co., Ltd., Japan), *T. atroviride* LC52 (e.g. SENTINEL® from Agrimm Technologies Ltd, NZ), *T. harzianum* T-22 (e.g. PLANTSHIELD® der Firma BioWorks Inc., USA), *T. harzianum* TH 35 (e.g. ROOT PRO® from Mycontrol Ltd., Israel), *T. harzianum* T-39 (e.g. TRICHODEX® and TRICHODERMA 2000® from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER® WP from Isagro Ricerca, Italy), *T. polysporum* and *T. harzianum* (e.g. BINAB® from BINAB Bio-Innovation AB, Sweden), *T. stromaticum* (e.g. TRICOVAB® from C.E.P.L.A.C., Brazil), *T. virens* GL-21 (e.g. SOILGARD® from Certis LLC, USA), *T. viride* (e.g. TRIECO® from Ecosense Labs. (India) Pvt. Ltd., Indien, BIO-CURE® F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN® from Botry-Zen Ltd, NZ).

The commercially available compounds II of the group F listed above may be found in The Pesticide Manual, 15th Edition, C. D. S. Tomlin, British Crop Protection Council (2011) among other publications. Their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EPA 141 317; EP-A 152 031; EP-A 226 917; EP A243 970; EP A 256 503; EP-A428 941; EP-A 532 022; EP-A 1 028 125; EP-A 1 035 122; EPA 1 201 648; EP A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657).

The compounds of the invention may be mixed with soil, peat or other rooting media for the protection of plants against seed-borne, soil-borne or foliar fungal diseases.

Examples of suitable synergists for use in the compositions include piperonyl butoxide, sesamex, safroxan and dodecyl imidazole.

Suitable herbicides and plant-growth regulators for inclusion in the compositions will depend upon the intended target and the effect required.

An example of a rice selective herbicide which may be included is propanil. An example of a plant growth regulator for use in cotton is PIX™.

Some mixtures may comprise active ingredients which have significantly different physical, chemical or biological properties such that they do not easily lend themselves to the same

The invertebrate pest (also referred to as "animal pest"), i.e. the insects, arachnids and nematodes, the plant, soil or water in which the plant is growing or may grow can be contacted with the compounds of the present invention or composition(s) comprising them by any application method known in the art. As such, "contacting" includes both direct contact (applying the compounds/compositions directly on the invertebrate pest or plant - typically to the foliage, stem or roots of the plant) and indirect contact (applying the compounds/compositions to the locus of the invertebrate pest or plant). The compounds of the present invention or the pesticidal compositions comprising them may be used to protect growing plants and crops from attack or infestation by animal pests, especially insects, acaridae or arachnids by contacting the plant/crop with a pesticidally effective amount of compounds of the present invention. The term "crop" refers both to growing and harvested crops.

The compounds of the present invention and the compositions comprising them are particularly important in the control of a multitude of insects on various cultivated plants, such as cereal, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

The compounds of the present invention are employed as such or in form of compositions by treating the insects or the plants, plant propagation materials, such as seeds, soil, surfaces, materials or rooms to be protected from insecticidal attack with an insecticidally effective amount of the active compounds. The application can be carried out both before and after the infection of the plants, plant propagation materials, such as seeds, soil, surfaces, materials or rooms by the insects.

Moreover, invertebrate pests may be controlled by contacting the target pest, its food supply, habitat, breeding ground or its locus with a pesticidally effective amount of compounds of the present invention. As such, the application may be carried out before or after the infection of the locus, growing crops, or harvested crops by the pest.

The compounds of the present invention can also be applied preventively to places at which occurrence of the pests is expected.

The compounds of the present invention may be also used to protect growing plants from attack or infestation by pests by contacting the plant with a pesticidally effective amount of compounds of the present invention. As such, "contacting" includes both direct contact (applying the compounds/compositions directly on the pest and/or plant - typically to the foliage, stem or roots of the plant) and indirect contact (applying the compounds/compositions to the locus of the pest and/or plant).

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

For use in treating crop plants, the rate of application of the active ingredients of this invention may be in the range of 0.1 g to 4000 g per hectare, desirably from 5 g to 500 g per hectare, more desirably from 5 g to 200 g per hectare.

The compounds of the present invention are effective through both contact (via soil, glass, wall, bed net, carpet, plant parts or animal parts), and ingestion (bait, or plant part).

The compounds of the present invention may also be applied against non-crop insect pests, such as ants, termites, wasps, flies, mosquitos, crickets, or cockroaches. For use against said non-crop pests, compounds of the present invention are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics. The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

For use in bait compositions, the typical content of active ingredient is from 0.001 weight % to 15 weight %, desirably from 0.001 weight % to 5% weight % of active ingredient.

Formulations of compounds of the present invention as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250°C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of the present invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the present invention and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder. Suitable repellents for example are N,N-Diethyl-meta-toluamide (DEET), N,N-diethylphenylacetamide (DEPA), 1-(3-cyclohexan-1-yl-carbonyl)-2-methylpiperine, (2-hydroxymethylcyclohexyl) acetic acid lactone, 2-ethyl-1,3-hexandiol, indalone, Methylneodecanamide (MNDA), a pyrethroid not used for insect control such as {(+/-)-3-allyl-2-methyl-4-oxocyclopent-2-(+)-enyl-(+)-trans-chrysantemate (Esbiothrin), a repellent derived from or identical with plant extracts like limonene, eugenol, (+)-Eucamalol (1), (-)-1-epi-eucamalol or crude plant extracts from plants like Eucalyptus maculata, Vitex rotundifolia, Cymbopogan martinii, Cymbopogan citratus (lemon grass), Cymopogan nartdus (citronella). Suitable binders are selected for example from polymers and copolymers of vinyl esters of aliphatic acids (such as such as vinyl acetate and vinyl versatate), acrylic and methacrylic esters of alcohols, such as butyl acrylate, 2-ethylhexylacrylate, and methyl acrylate, mono- and di-ethylenically unsaturated hydrocarbons, such as styrene, and aliphatic diens, such as butadiene.

The impregnation of curtains and bednets is done in general by dipping the textile material into emulsions or dispersions of the insecticide or spraying them onto the nets. The compounds of the present invention and their compositions can be used for protecting wooden materials such as trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of the present invention are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied to the crops or the surrounding soil, or is directly applied to the nest of ants or the like.

The compounds of the present invention are also suitable for the treatment of plant propagation material, especially seeds, in order to protect them from insect pest, in particular from soil-living insect pests and the resulting plant's roots and shoots against soil pests and foliar insects.

The compounds of the present invention are particularly useful for the protection of the seed from soil pests and the resulting plant's roots and shoots against soil pests and foliar insects. The protection of the resulting plant's roots and shoots is preferred. More preferred is the protection of resulting plant's shoots from piercing and sucking insects, wherein the protection from aphids is most preferred.

The present invention therefore comprises a method for the protection of seeds from insects, in particular from soil insects and of the seedlings' roots and shoots from insects, in particular from soil and foliar insects, said method comprising contacting the seeds before sowing and/or after pregermination with a compound of the present invention, including a salt thereof. Particularly preferred is a method, wherein the plant' s roots and shoots are protected, more preferably a method, wherein the plants shoots are protected form piercing and sucking insects, most preferably a method, wherein the plants shoots are protected from aphids.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term seed treatment comprises all suitable seed treatment techniques known in the art, such as seed dressing, seed coating, seed dusting, seed soaking and seed pelleting.

The present invention also comprises seeds coated with or containing the active compound.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is seed of cereals, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment seeds from plants, which tolerate the action of herbicides or fungicides or insecticides owing to breeding, including genetic engineering methods.

For example, the active compound can be employed in treatment of seeds from plants, which are resistant to herbicides from the group consisting of the sulfonylureas, imidazolinones, glufosinate-ammonium or glyphosate-isopropylammonium and analogous active substances (see for example, EP-A 242 236, EP-A 242 246) (WO 92/00377) (EP-A 257 993, U.S. 5,013,659) or in transgenic crop plants, for example cotton, with the capability of producing Bacillus thuringiensis toxins (Bt toxins) which make the plants resistant to certain pests (EP-A 142 924, EP-A 193 259), Furthermore, the active compound can be used also for the treatment of seeds from plants, which have modified characteristics in comparison with existing plants consist, which can be generated for example by traditional breeding methods and/or the generation of mutants, or by recombinant procedures). For example, a number of cases have been described of recombinant modifications of crop plants for the purpose of modifying the starch synthesized in the plants (e.g. WO 92/11376, WO 92/14827, WO 91/19806) or of transgenic crop plants having a modified fatty acid composition (WO 91/13972).

The seed treatment application of the active compound is carried out by spraying or by dusting the seeds before sowing of the plants and before emergence of the plants.

Compositions which are especially useful for seed treatment are e.g.:
- A: Soluble concentrates (SL, LS)
- D: Emulsions (EW, EO, ES)
- E: Suspensions (SC, OD, FS)
- F: Water-dispersible granules and water-soluble granules (WG, SG)
- G: Water-dispersible powders and water-soluble powders (WP, SP, WS)
- H: Gel-Formulations (GF)
- I: Dustable powders (DP, DS)

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter.

In a preferred embodiment a FS formulation is used for seed treatment. Typcially, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of compounds of the present invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker /adhesion agent), optionally up to 5% by weight, e.g. from 0.1 to 5% by weight of a thickener, optionally from 0.1 to 2% of an anti-foam agent, and optionally a preservative such as a biocide, antioxidant or the like, e.g. in an amount from 0.01 to 1% by weight and a filler/vehicle up to 100% by weight.

Seed Treatment formulations may additionally also comprise binders and optionally colorants.

Binders can be added to improve the adhesion of the active materials on the seeds after treatment. Suitable binders are homo- and copolymers from alkylene oxides like ethylene oxide or propylene oxide, polyvinylacetate, polyvinylalcohols, polyvinylpyrrolidones, and copolymers thereof, ethylene-vinyl acetate copolymers, acrylic homo- and copolymers, polyethyleneamines, polyethyleneamides and polyethyleneimines, polysaccharides like celluloses, tylose and starch, polyolefin homo- and copolymers like olefin/maleic anhydride copolymers, polyurethanes, polyesters, polystyrene homo and copolymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

### Examples of a gelling agent is carrageen (Satiagel®)

In the treatment of seed, the application rates of the compounds of the present invention are generally from 0.01 g to 10 kg per 100 kg of seed, preferably from 0.05 g to 5 kg per 100 kg of seed, more preferably from 0.1 g to 1000 g per 100 kg of seed and in particular from 0.1 g to 200 g per 100 kg of seed.

The invention therefore also relates to seed comprising a compound of the present invention, including an agriculturally useful salt of it, as defined herein. The amount of the compound of the present invention, including an agriculturally useful salt thereof will in general vary from 0.01 g to 10 kg per 100 kg of seed, preferably from 0.05 g to 5 kg per 100 kg of seed, in particular from 0.1 g to 1000 g per 100 kg of seed. For specific crops such as lettuce the rate can be higher.

Methods which can be employed for treating the seed are, in principle, all suitable seed treatment and especially seed dressing techniques known in the art, such as seed coating (e.g. seed pelleting), seed dusting and seed imbibition (e.g. seed soaking). Here, "seed treatment" refers to all methods that bring seeds and the compounds of the present invention into contact with each other, and "seed dressing" to methods of seed treatment which provide the seeds with an amount of the compounds of the present invention, i.e. which generate a seed comprising a compound of the present invention. In principle, the treatment can be applied to the seed at any time from the harvest of the seed to the sowing of the seed. The seed can be treated immediately before, or during, the planting of the seed, for example using the "planter's box" method. However, the treatment may also be carried out several weeks or months, for example up to 12 months, before planting the seed, for example in the form of a seed dressing treatment, without a substantially reduced efficacy being observed.

Expediently, the treatment is applied to unsown seed. As used herein, the term "unsown seed" is meant to include seed at any period from the harvest of the seed to the sowing of the seed in the ground for the purpose of germination and growth of the plant.

Specifically, a procedure is followed in the treatment in which the seed is mixed, in a suitable device, for example a mixing device for solid or solid/liquid mixing partners, with the desired amount of seed treatment formulations, either as such or after previous dilution with water, until the composition is distributed uniformly on the seed. If appropriate, this is followed by a drying step.

The compounds of the present invention, including their stereoisomers, veterinarily acceptable salts or N-oxides, are in particular also suitable for being used for combating parasites in and on animals.

An object of the present description is therefore also to provide new methods to control parasites in and on animals. Another object of the invention is to provide safer pesticides for animals. Another object of the invention is further to provide pesticides for animals that may be used in lower doses than existing pesticides. And another object of the invention is to provide pesticides for animals, which provide a long residual control of the parasites.

The invention also relates to compositions comprising a parasiticidally effective amount of compounds of the present invention, including their stereoisomers, veterinarily acceptable salts or N-oxides, and an acceptable carrier, for combating parasites in and on animals.

The present invention also provides a compound of the present invention, including its stereoisomers, veterinarily acceptable salts or N-oxides, or a composition comprising it, for use in a method for treating, controlling, preventing and protecting animals against infestation and infection by parasites, which comprises orally, topically or parenterally administering or applying to the animals a parasiticidally effective amount of a compound of the present invention, including its stereoisomers, veterinarily acceptable salts or N-oxides, or a composition comprising it.

The invention also provides a compound of the present invention, including its stereoisomers, veterinarily acceptable salts or N-oxides, for use in treating or protecting an animal from infestation or infection by invertebrate pests.

The invention also provides a process for the preparation of a composition for treating, controlling, preventing or protecting animals against infestation or infection by parasites which comprises a parasiticidally effective amount of a compound of the present invention, including its stereoisomers, veterinarily acceptable salts or N-oxides, or a composition comprising it.

Activity of compounds against agricultural pests does not suggest their suitability for control of endo- and ectoparasites in and on animals which requires, for example, low, non-emetic dosages in the case of oral application, metabolic compatibility with the animal, low toxicity, and a safe handling.

Surprisingly it has now been found that compounds of formula (I) and their stereoisomers, veterinarily acceptable salts, tautomers and N-oxides, are suitable for combating endo- and ectoparasites in and on animals.

The compounds of the present invention, especially compounds of formula (I) and their stereoisomers, veterinarily acceptable salts, tautomers and N-oxides, and compositions comprising them are preferably used for controlling and preventing infestations of and infections in animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Compounds of the present invention, including their stereoisomers, veterinarily acceptable salts or N-oxides, and compositions comprising them are preferably used for controlling and preventing infestations and infections in domestic animals, such as dogs or cats.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of the present invention, including their stereoisomers, veterinarily acceptable salts or N-oxides, and compositions comprising them are suitable for systemic and/or non-systemic control of ecto- and/or endoparasites. They are active against all or some stages of development.

The compounds of the present invention are especially useful for combating parasites of the following orders and species, respectively:
fleas (Siphonaptera), e.g. Ctenocephalides felis, Ctenocephalides canis, Xenopsylla cheopis, Pulex irritans, Tunga penetrans, and Nosopsyllus fasciatus, cockroaches (Blattaria - Blattodea), e.g. Blattella germanica, Blattella asahinae, Periplaneta americana, Periplaneta japonica, Periplaneta brunnea, Periplaneta fuligginosa, Periplaneta australasiae, and Blatta orientalis,
flies, mosquitoes (Diptera), e.g. Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola, and Tabanus similis,
lice (Phthiraptera), e.g. Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.
ticks and parasitic mites (Parasitiformes): ticks (Ixodida), e.g. Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata and parasitic mites (Mesostigmata), e.g. Ornithonyssus bacoti and Dermanyssus gallinae,
Actinedida (Prostigmata) und Acaridida (Astigmata) e.g. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp., and Laminosioptes spp., Bugs (Heteropterida): Cimex lectularius, Cimex hemipterus, Reduvius senilis, Triatoma spp., Rhodnius ssp., Panstrongylus ssp. and Arilus critatus,
Anoplurida, e.g. Haematopinus spp., Linognathus spp., Pediculus spp., Phtirus spp., and Solenopotes spp,
Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp,
Roundworms Nematoda:
   Wipeworms and Trichinosis (Trichosyringida), e.g. Trichinellidae (Trichinella spp.), (Trichuridae) Trichuris spp., Capillaria spp,
   Rhabditida, e.g. Rhabditis spp, Strongyloides spp., Helicephalobus spp,
   Strongylida, e.g. Strongylus spp., Ancylostoma spp., Necator americanus, Bunostomum spp. (Hookworm), Trichostrongylus spp., Haemonchus contortus., Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp. Aleurostrongylus abstrusus, and Dioctophyma renale,
   Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi,
   Camallanida, e.g. Dracunculus medinensis (guinea worm)
   Spirurida, e.g. Thelazia spp. Wuchereria spp., Brugia spp., Onchocerca spp., Dirofilari spp.a, Dipetalonema spp., Setaria spp., Elaeophora spp., Spirocerca lupi, and Habronema spp.,
   Thorny headed worms (Acanthocephala), e.g. Acanthocephalus spp., Macracanthorhynchus hirudinaceus and Oncicola spp.,
Planarians (Plathelminthes):
   Flukes (Trematoda), e.g. Faciola spp., Fascioloides magna, Paragonimus spp.,
   Dicrocoelium spp., Fasciolopsis buski, Clonorchis sinensis, Schistosoma spp.,
   Trichobilharzia spp., Alaria alata, Paragonimus spp., and Nanocyetes spp.,
   Cercomeromorpha, in particular Cestoda (Tapeworms), e.g. Diphyllobothrium spp.,
   Tenia spp., Echinococcus spp., Dipylidium caninum, Multiceps spp., Hymenolepis spp.,
   Mesocestoides spp., Vampirolepis spp., Moniezia spp., Anoplocephala spp., Sirometra spp., Anoplocephala spp., and Hymenolepis spp.

The present description relates to the therapeutic and the non-therapeutic use of compounds of the present invention and compositions comprising them for controlling and/or combating parasites in and/or on animals. The compounds of the present invention and compositions comprising them may be used to protect the animals from attack or infestation by parasites by contacting them with a parasiticidally effective amount of compounds of the present invention and compositions containing them. The compounds of the present invention and compositions comprising them can be effective through both contact (via soil, glass, wall, bed net, carpet, blankets or animal parts) and ingestion (e.g. baits). As such, "contacting" includes both direct contact (applying the pesticidal mixtures/compositions containing the compounds of the present invention directly on the parasite, which may include an indirect contact at its locus-P, and optionally also administrating the pesticidal mixtures/composition directly on the animal to be protected) and indirect contact (applying the compounds/compositions to the locus of the parasite). The contact of the parasite through application to its locus is an example of a non-therapeutic use of compounds of the present invention. "Locus-P" as used above means the habitat, food supply, breeding ground, area, material or environment in which a parasite is growing or may grow outside of the animal.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions of the present invention. A parasiticidally effective amount of the compositions will also vary according to the prevailing conditions such as desired parasiticidal effect and duration, target species, mode of application, and the like.

The compounds of the present invention can also be applied preventively to places at which occurrence of the pests or parasites are expected.

Administration can be carried out both prophylactically and therapeutically. Administration of the active compounds is carried out directly or in the form of suitable preparations, orally, topically/dermally or parenterally.

The isothiazoline compounds of the present invention are less persistent, bioaccumulative and/or toxic than the compounds of the prior art, and especially the isoxazoline insecticides of the prior art, which show a high persistency in the soil and thus accumulate there.

### Examples

The present invention is now illustrated in further details by the following examples, without imposing any limitation thereto.

### Preparation Examples

Compounds can be characterized e.g. by coupled High Performance Liquid Chromatography / mass spectrometry (HPLC/MS), by ¹H-NMR and/or by their melting points.

### Analytical HPLC column:

Method: Analytical UPLC column: Phenomenex Kinetex 1,7 µm XB-C18 100A; 50 x2.1 mm from Phenomenex, Germany. Elution: acetonitrile + 0.1% trifluoroacetic acid (TFA) / water + 0.1% trifluoroacetic acid (TFA) in a ratio from 5:95 to 100:0 in 1.5 min at 60 °C. Flow: 0.8 mL/min to 1 mL/min in 1.5 min. MS-method: ESI positive.
¹H-NMR: The signals are characterized by chemical shift (ppm, δ [delta]) vs. tetramethylsilane, respectively CDCl₃ for ¹³C-NMR, by their multiplicity and by their integral (relative number of hydrogen atoms given). The following abbreviations are used to characterize the multiplicity of the signals: m = multiplet, q = quartet, t = triplet, d = doublet and s = singlet.

### C.1 Compound examples

Compound examples 1-1 to 1-42 correspond to compounds of formula C.1: 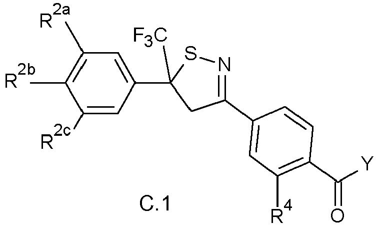 wherein R^{2a}, R^{2b}, R^{2c}, R⁴, and Y of each synthesized compound is defined in one row of table C.1 below (whereby compounds 1-18, 1-23, and 1-33 are not part of the invention). The compounds were synthesized in analogy to Synthesis Example S.1.

**Table C.1**

| Ex. | R^{2a}, R^{2b}, R^{2c} | R⁴ | Y | **HPLC-MS:** | |
|---|---|---|---|---|---|
| | | | | Rₜ (min) & [M+H]⁺ | |
| 1-1 | Cl, Cl, Cl | H | azetidin-1-yl | 1.431 | 495.1 |
| 1-2 | Cl, Cl, Cl | H | pyrrolidin-1-yl | 1.461 | 507.1 |
| 1-3 | Cl, F, Cl | Cl | 3,3-difluoro-pyrrolidin-1-yl | 1.447 | 562.9 |
| 1-4 | Cl, F, Cl | Cl | 3,3,4,4-tetrafluoro-pyrrolidin-1-yl | 1.498 | 597.0 |
| 1-5 | Cl, F, Cl | Cl | 4,4-difluoro-piperidine-1-yl | 1.485 | 575.1 |
| 1-6 | Cl, F, Cl | Cl | 3,3-difluoro-azetidin-1-yl | 1.451 | 549.1 |
| 1-7 | Cl, F, Cl | Cl | 4-cyano-1-piperidyl | 1.408 | 566.1 |

| | | | | Rₜ (min) & [M+H]⁺ | |
|---|---|---|---|---|---|
| 1-8 | Cl, F, Cl | Cl | 3-acetamidopyrrolidin-1-yl | 1.33 | 584.4 |
| 1-9 | Cl, F, Cl | Cl | 2-methoxycarbonylaziridin-1-yl | 1.517 | 557.3 |
| 1-10 | Cl, F, Cl | Cl | 3-[(2,2,2-trifluoroacetyl)-amino]-pyrrolidin-1-yl | 1.455 | 638.4 |
| 1-11 | Cl, F, Cl | Cl | 4-acetylpiperazin-1-yl | 1.366 | 584.5 |
| 1-12 | Cl, F, Cl | Cl | 3-cyanopyrrolidin-1-yl | 1.424 | 550.6 |
| 1-13 | Cl, F, Cl | Cl | 2-(methylcarbamoyl)-pyrrolidin-1-yl | 1.362 | 583.6 |
| 1-14 | Cl, F, Cl | Cl | 2-(2,2,2-trifluoroethyl-carbamoyl)-pyrrolidin-1-yl | 1.447 | 651.6 |
| 1-15 | Cl, F, Cl | Cl | 2,2,6,6-tetrafluoromorpholin-4-yl | 1.54 | 612.7 |
| 1-16 | Cl, F, Cl | Cl | 3-cyanoazetidin-1-yl | 1.418 | 538.4 |
| 1-17 | Cl, F, Cl | Cl | 2-methoxycarbonylazetidin-1-yl | 1.451 | 571.4 |
| 1-18 | Cl, F, Cl | Cl | 3-methyl-4-oxo-imidazolidin-1-yl | 1.351 | 555.6 |
| 1-19 | Cl, F, Cl | Cl | 3-cyano-1-piperidyl | 1.447 | 563.8 |
| 1-20 | Cl, F, Cl | Cl | 3-(methylcarbamoyl)-pyrrolidin-1-yl | 1.33 | 584.4 |
| 1-21 | Cl, F, Cl | Cl | 3-(methylcarbamoyl)-1-piperidyl | 1.377 | 598.5 |
| 1-22 | Cl, F, Cl | Cl | 2-(2,2,2-trifluoroethyl-carbamoyl)-azetidin-1-yl | 1.434 | 636.7 |
| 1-23 | Cl, F, Cl | Cl | 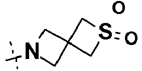 | 1.35 | 602.6 |
| 1-24 | Cl, F, Cl | Cl | 4-(methylcarbamoyl)-1-piperidyl | 1.341 | 596.7 |
| 1-25 | Cl, F, Cl | Cl | 2-(methylcarbamoyl)-1-azetidinyl | 1.330 | 569.6 |
| 1-26 | Cl, F, Cl | Cl | 3-(ethylcarbamoylamino)-1-pyrrolidinyl | 1.326 | 612.6 |
| 1-27 | Cl, F, Cl | Cl | 3-(3,3,3-trifluoropropanoylamino)-1-pyrrolidinyl | 1.393 | 651.5 |
| 1-28 | Cl, F, Cl | Cl | 3-(propanoylamino)-1-pyrrolidinyl | 1.349 | 598.7 |
| 1-29 | Cl, F, Cl | Cl | 3-(methoxycarbonylamino)-1-pyrrolidinyl | 1.374 | 600.7 |
| 1-30 | Cl, F, Cl | Cl | 3-acetamidoazetidin-1-yl | 1.298 | 567.7 |
| 1-31 | Cl, F, Cl | Cl | 3-(methylcarbamoylamino)-pyrrolidin-1-yl | 1.311 | 599.7 |
| 1-32 | Cl, F, Cl | Cl | 3-(2,2,2-trifluoroethyl-carbamoyl)pyrrolidin-1-yl | 1.377 | 651.5 |
| 1-33 | Cl, F, Cl | Cl | 2-oxo-3-(2,2,2-trifluoroethyl)imidazolidin-1-yl | 1.514 | 623.6 |
| 1-34 | Cl, F, Cl | Cl | aziridin-1-yl | ¹H NMR (400 MHz, CDCl₃): δ 7.9-7.8 (m, 2H), 7.8-7.6 (m, 1H), 7.35-7.25 (m, 2H), 4.6-4.4 (m, 2H), 4.3-4.1 (m, 3H), 3.9 (d, 1H) | |
| 1-35 | Cl, F, Cl | Cl | azetidin-1-yl | 1.480 | 513.1 |
| 1-36 | Cl, F, Cl | Cl | 2-cyanopyrrolidin-1-yl | 1.478 | 552.1 |
| 1-37 | Cl, F, Cl | Cl | 2-(2,2,2-trifluoroethyl-carbamoyl)aziridin-1-yl | 1.507 | 624.0 |
| 1-38 | Cl, F, Cl | Cl | 3-[(2,2,2-trifluoroacetyl)-amino]azetidin-1-yl | 1.417 | 621.8 |
| 1-39 | Cl, F, Cl | Cl | 3-(cyclobutanecarbonylamino)azetidin-1-yl | 1.395 | 609.8 |
| 1-40 | Cl, F, Cl | Cl | 3-[(2-methoxyacetyl)amino]-azetidin-1-yl | 1.329 | 599.8 |
| 1-41 | Cl, F, Cl | Cl | 3-(propanoylamino)azetidin-1-yl | 1.364 | 584.0 |
| 1-42 | Cl, F, Cl | Cl | 3-(cyclopropanecarbonylamino)azetidin-1-yl | 1.357 | 594.8 |

### Synthesis Example S.1

### [2-Chloro-4-[5-(3,5-dichloro-4-fluoro-phenyl)-5-(trifluoromethyl)-4H-isothiazol-3-yl]phenyl]-(4,4-difluoro-1-piperidyl)methanone

(Compound example 1-5; compound of formula IA, wherein R^{2a} and R^{2c} are Cl, R^{2b} is F, R⁴ is Cl and R⁵ and R⁶, together with the nitrogen atom to which they are bound, are 4,4-difluoro-1-piperidyl.

### Step 1: 1-(3,5-Dichloro-4-fluoro-phenyl)-2,2,2-trifluoro-ethanone

To a solution of 1,3-dichloro-2-fluoro-5-iodo-benzene (CAS 133307-08-1, 50 g) and 2,2,2-trifluoro-*N*-methoxy-*N*-methyl-acetamide (40.5 g) in THF (800 mL) at -78°C was added n-BuLi (82.5 mL, 2.5 M in hexanes). The reaction was stirred for 20 min, then quenched by saturated aqueous NH₄Cl solution and extracted with ethyl acetate. The organic layer was dried (Na₂SO₄), filtered, and concentrated. The residue was purified by flash chromatography on silica gel to afford the product (24 g, 53%).
¹H NMR (400 MHz, CDCl₃): δ 8.0 (m, 2H).

### Step 2: tert-Butyl 4-acetyl-2-chloro-benzoate

To a solution of tert-butyl 4-bromo-2-chloro-benzoate (CAS 929000-18-0, 85 g) in xylene (600 mL) were added tris(dibenzylideneacetone)dipalladium(0) ("Pd₂(dba)₃", 4.3 g), 1,1'-binaphthalene-2,2'-diyl)bis(diphenylphosphine) ("BINAP", 3.7 g) and tributyl(1-ethoxyvinyl)tin (126.6 g). The mixture was stirred under N₂ at 150°C overnight, then the mixture was concentrated, and the residue was dissolved in THF and aqueous 2 M HCI solution and stirred at r.t. for 2 h. The mixture was extracted with ethyl acetate, and the organic layer was concentrated to give a residue, which was purified by flash chromatography on silica gel to afford the product (60 g, 81%).
¹H NMR (400 MHz, CDCl₃): δ 7.9 (s, 1H), 7.8 (d, 1H), 7.7 (d, 1H), 2.6 (s, 3H), 1.6 (s, 9H).

### Step 3: tert-Butyl 2-chloro-4-[3-(3,5-dichloro-4-fluoro-phenyl)-4,4,4-trifluoro-3-hydroxy-butanoyl]benzoate

A solution of the product of step 1 (30 g) and the product of step 2 (23.4 g) in a mixture of triethylamine (10 mL) and heptane (300 mL) was stirred at 70 °C overnight. Then, the mixture was concentrated. The residue was purified by flash chromatography on silica gel to afford the product (37.5 g, 63%).
¹H NMR (400 MHz, CDCl₃): δ 7.9 (s, 1H), 7.8 (m, 2H), 7.5 (m, 2H), 5.5 (s, 1H), 3.7 (d, 1H), 3.6 (d, 1H), 1.6 (s, 9H).

### Step 4: tert-Butyl 2-chloro-4-[3-(3,5-dichloro-4-fluoro-phenyl)-4,4,4-trifluoro-but-2-enoyl]benzoate

To the product of step 4 (50 g) in pyridine (27 g) and toluene (400 mL) was added thionyl chloride (45 g) at 60°C. Then, the mixture was heated at 80°C for 5 h, and concentrated. The residue was purified by flash chromatography on silica gel to afford the product as a mixture of *E*/*Z*-isomers (42 g, 87%).
¹H NMR (400 MHz, CDCl₃): δ 7.9- 6.8 (m, 6H), 1.6 (s, 9H).

### Step 5: tert-Butyl 2-chloro-4-[3-(3,5-dichloro-4-fluoro-phenyl)-4,4,4-trifluoro-3-sulfanyl-butanoyl]benzoate

The product of step 4 (56 g, mixture of *E*/*Z*-isomers) in CH₂Cl₂ (500 mL) was treated with triethylamine (114 g). At 0°C, gaseous hydrogen sulfide (H₂S) was bubbled through the solution until the solution was saturated. The mixture was stirred for another 1 h at 0°C, and then diluted with CH₂Cl₂ (200 mL). The organic layer was washed with 10% aqueous hydrochloric acid solution (3x), dried (Na₂SO₄), filtered, and concentrated to afford the crude product (59.9 g, quant.), which was used in the next step without any further purification.
¹H NMR (400 MHz, CDCl₃): δ 7.9 (s, 1H), 7.8 (m, 2H), 7.6 (d, 2H), 4.2 (d, 1H), 3.9 (d, 1H), 3.2 (s, 1H (SH)), 1.6 (s, 9H).

### Step 6: tert-Butyl 2-chloro-4-[5-(3,5-dichloro-4-fluoro-phenyl)-5-(trifluoromethyl)-4H-isothiazol-3-yl]benzoate

At -15°C, the product of step 5 (59.9 g) in CH₂Cl₂ (600 mL) was treated with triethylamine (45.6 g) and with a solution of hydroxylamine-*O*-sulfonic acid ("HOSA", 15.3 g) in water (20 mL). The reaction was warmed to 0°C and stirred at 0°C for 1 h, and then diluted with CH₂Cl₂ (300 mL). The organic layer was washed with saturated aqueous NH₄Cl solution (3x), dried (Na₂SO₄), and filtered. To the obtained solution, para-toluene sufonic acid ("p-TsOH", 0.5 g) was added and the mixture was stirred for 2 h at r.t. Then, the reaction was washed with 5% aqueous K₂CO₃ solution (3x), dried (Na₂SO₄), and concentrated. The obtained residue was purified by trituration (hexanes/CH₂Cl₂) to afford a white solid (34.4 g, 58%).
¹H NMR (400 MHz, CDCl₃): δ 7.8 (m, 2H), 7.7 (m, 1H), 7.4 (m, 2H), 4.2 (d, 1H), 3.9 (d, 1H), 1.6 (s, 9H).

### Step 7: 2-Chloro-4-[5-(3,5-dichloro-4-fluoro-phenyl)-5-(trifluoromethyl)-4H-isothiazol-3-yl]benzoic acid

At 0°C, the product of step 6 (16.2 g) in CH₂Cl₂ (120 mL) was treated with trifluoroacetic acid ("TFA", 60 mL), and the mixture stirred overnight at r.t. The reaction was concentrated, and co-evaporated with CH₂Cl₂ (5x) to afford a pale yellow solid. The residue was purified by trituration (petroleum ether/ethyl acetate) to afford the product (14.1 g, 97%).
¹H NMR (400 MHz, CDCl₃): δ 8.1 (d, 1H), 7.8 (s, 1H), 7.7 (d, 1H), 7.4 (m, 2H), 4.2 (d, 1H), 3.9 (d, 1H).

### Step 8: [2-Chloro-4-[5-(3,5-dichloro-4-fluoro-phenyl)-5-(trifluoromethyl)-4H-isothiazol-3-yl]phenyl]-(4,4-difluoro-1-piperidyl)methanone

To a solution of the product of step 7 (0.4 g) in CH₂Cl₂/toluene (1:1, 20 mL) at r.t. was added N,N-dimethylformamid ("DMF", 1 drop) and oxalyl chloride (0.21 g). The reaction was stirred overnight at r.t., and concentrated. The residue was co-evaporated with CH₂Cl₂ (5x) and used in the next step without any further purification (0.41 g, "acid chloride").

A solution of the "acid chloride" (0.2 g) in THF (10 mL) was added to a solution of 4,4-difluoropiperidine hydrochloride (80 mg) in THF (10 mL) at 0 °C. The resulting solution was stirred at r.t. for 66 h, then filtered and concentrated. The residue was purified by flash chromatography on silica gel (ethyl acetate/cyclohexane) to afford the product (0.12 g, 51%).
¹H NMR (400 MHz, CDCl₃): δ 7.9-7.6 (m, 2H), 7.4 (m, 3H), 4.2 (d, 1H), 3.8 (m, 2H), 3.4 (m, 1H), 3.3 (m, 1H), 2.2-2.0 (m, 3H), 2.0-1.8 (m, 1H).

### II. Evaluation of pesticidal activity:

The activity of the compounds of formula I of the present invention can be demonstrated and evaluated by the following biological test.

### B.1 Diamond back moth (Plutella xylostella)

Leaves of Chinese cabbage were dipped in test solution and air-dried. Treated leaves were placed in petri dished lined with moist filter paper. Mortality was recorded 24, 72, and 120 hours after treatment.

In this test, the compounds 1-1, 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-25, 1-26, 1-27, 1-28, 1-29, 1-30, 1-31, 1-32, 1-33 and 1-34 at 500 ppm, respectively, showed a mortality of at least 75% in comparison with untreated controls.

### B.2 Green Peach Aphid (Myzus persicae)

For evaluating control of green peach aphid (Myzus persicae) through systemic means the test unit consisted of 96-well-microtiter plates containing liquid artificial diet under an artificial membrane.

The compounds were formulated using a solution containing 75% v/v water and 25% v/v DMSO. Different concentrations of formulated compounds were pipetted into the aphid diet, using a custom built pipetter, at two replications.

After application, 5 - 8 adult aphids were placed on the artificial membrane inside the microtiter plate wells. The aphids were then allowed to suck on the treated aphid diet and incubated at about 23 + 1 °C and about 50 + 5 % relative humidity for 3 days. Aphid mortality and fecundity was then visually assessed.

In this test, the compounds 1-1, 1-2, 1-3, 1-4, 1-5, 1-6, 1-7, 1-8, 1-9, 1-10, 1-11, 1-14, 1-15, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36 and 1-37 at 2500 ppm, respectively, showed a mortality of at least 75% in comparison with untreated controls.

### B.3 Mediterranean fruitfly (Ceratitis capitata)

For evaluating control of Mediterranean fruitfly (Ceratitis capitata) the test unit consisted of microtiter plates containing an insect diet and 50-80 C. capitata eggs. The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 28 ± 1°C and about 80 ± 5 % relative humidity for 5 days. Egg and larval mortality was then visually assessed.

In this test, the compounds 1-1, 1-3, 1-4, 1-5, 1-6, 1-7, 1-8, 1-9, 1-10, 1-12, 1-13, 1-16, 1-18, 1-20, 1-21, 1-22, 1-23, 1-24, 1-25, 1-26, 1-27, 1-28, 1-29, 1-30, 1-32, 1-34, 1-35 and 1-36 at 2500 ppm, respectively, showed a mortality of at least 75% in comparison with untreated controls.

### B.4 Orchid thrips (dichromothrips corbetti)

Dichromothrips corbetti adults used for bioassay were obtained from a colony maintained continuously under laboratory conditions. For testing purposes, the test compound was diluted to a concentration of 500 ppm (wt compound: vol diluent) in a 1:1 mixture of acetone:water (vol:vol), plus 0.01 % vol/vol Kinetic® surfactant.

Thrips potency of each compound was evaluated by using a floral-immersion technique. Plastic petri dishes were used as test arenas. All petals of individual, intact orchid flowers were dipped into treatment solution and allowed to dry. Treated flowers were placed into individual petri dishes along with 10 - 15 adult thrips. The petri dishes were then covered with lids. All test arenas were held under continuous light and a temperature of about 28oC for duration of the assay. After 4 days, the numbers of live thrips were counted on each flower, and along inner walls of each petri dish. The level of thrips mortality was extrapolated from pre-treatment thrips numbers.

In this test, the compounds 1-1, 1-2, 1-3, 1-4, 1-5, 1-6, 1-7, 1-8, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-29, 1-31, 1-32, 1-33 and 1-34 at 500 ppm, respectively, showed a mortality of at least 75% in comparison with untreated controls.

### B.5 Rice green leafhopper (Nephotettix virescens)

Rice seedlings were cleaned and washed 24 hours before spraying. The active compounds were formulated in 50:50 acetone:water (vol:vol), and 0.1% vol/vol surfactant (EL 620) was added. Potted rice seedlings were sprayed with 5 ml test solution, air dried, placed in cages and inoculated with 10 adults. Treated rice plants were kept at about 28-29°C and relative humidity of about 50-60%. Percent mortality was recorded after 72 hours.

In this test, the compounds 1-3, 1-4, 1-5, 1-12, 1-16, 1-17, 1-18, 1-22, 1-25 and 1-29 at 500 ppm, respectively, showed a mortality of at least 75% in comparison with untreated controls.

### B.6 Vetch aphid (Megoura viciae)

For evaluating control of vetch aphid (Megoura viciae) through contact or systemic means the test unit consisted of 24-well-microtiter plates containing broad bean leaf disks.

The compounds were formulated using a solution containing 75% v/v water and 25% v/v DMSO. Different concentrations of formulated compounds were sprayed onto the leaf disks at 2.5 µl, using a custom built micro atomizer, at two replications.

After application, the leaf disks were air-dried and 5 - 8 adult aphids placed on the leaf disks inside the microtiter plate wells. The aphids were then allowed to suck on the treated leaf disks and incubated at about 23 ± 1 °C and about 50 ± 5 % relative humidity for 5 days. Aphid mortality and fecundity was then visually assessed.

In this test, the compounds 1-1, 1-3, 1-4, 1-5, 1-6, 1-7, 1-9, 1-10, 1-12, 1-13, 1-16, 1-17, 1-18, 1-19, 1-22, 1-25, 1-28, 1-29, 1-33, 1-34, 1-35, 1-36 and 1-37 at 2500 ppm, respectively, showed a mortality of at least 75% in comparison with untreated controls.

### B.7 Tobacco budworm (Heliothis virescens)

For evaluating control of tobacco budworm (Heliothis virescens) the test unit consisted of 96-well-microtiter plates containing an insect diet and 15-25 H. virescens eggs.

The compounds were formulated using a solution containing 75% v/v water and 25% v/v DMSO. Different concentrations of formulated compounds were sprayed onto the insect diet at 10 µl, using a custom built micro atomizer, at two replications.

After application, microtiter plates were incubated at about 28 ± 1°C and about 80 ± 5 % relative humidity for 5 days. Egg and larval mortality was then visually assessed.

In this test, the compounds 1-1, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34 and 1-35 at 2500 ppm, respectively, showed a mortality of at least 75% in comparison with untreated controls.

### B.8 Boll weevil (Anthonomus grandis)

For evaluating control of boll weevil (Anthonomus grandis) the test unit consisted of 24-well-microtiter plates containing an insect diet and 20-30 A. grandis eggs.

The compounds were formulated using a solution containing 75% v/v water and 25% v/v DMSO. Different concentrations of formulated compounds were sprayed onto the insect diet at 20 µl, using a custom built micro atomizer, at two replications.

After application, microtiter plates were incubated at about 23 ± 1°C and about 50 ± 5 % relative humidity for 5 days. Egg and larval mortality was then visually assessed.

In this test, the compounds 1-1, 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36 and 1-37 at 2500 ppm, respectively, showed a mortality of at least 75% in comparison with untreated controls.

### B.9 Red spider Mite (Tetranychus kanzawai)

The active compound was dissolved at the desired concentration in a mixture of 1:1 (v/v) distilled water: acetone. A surfactant (Alkamuls® EL 620) was added at the rate of 0.1% (v/v).

Potted cowpea beans of 7-10 days of age were cleaned with tap water and sprayed with 5 ml of the test solution using air driven hand atomizer. The treated plants were allowed to air dry and afterwards inculated with 20 or more mites by clipping a cassava leaf section with known mite population. Treated plants were placed inside a holding room at about 25-27°C and about 50-60% relatice humidity.

Mortality was detremined by counting the live mites 72 HAT. Percent mortality was assessed after 72 h.

In this test, the compounds 1-3, 1-4, 1-5, 1-6, 1-17, 1-18, 1-22, 1-25, 1-29 and 1-34 at 500 ppm, respectively, showed a mortality of at least 75% in comparison with untreated controls.

## Claims

1. Isothiazoline compounds of the formula I 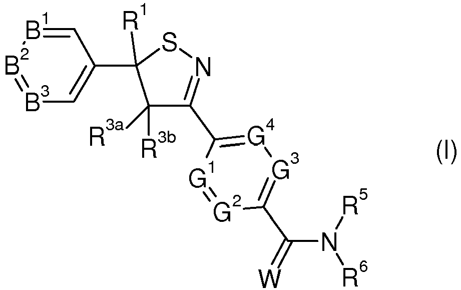 wherein:
B¹, B² and B³ are each independently CR²;
G¹, G³ and G⁴ are CH and G² is CR⁴;
W is O;
R¹ is CF₃;
each R² is independently selected from hydrogen, halogen and C₁-C₂-haloalkyl;
R^{3a}, R^{3b} are each independently selected from hydrogen and halogen;
R⁴ is selected from hydrogen, halogen and methyl;
R⁵ and R⁶, together with the nitrogen atom to which they are bound, form a 3-, 4-, 5- or 6-membered saturated heteromonocyclic ring, where the ring may further contain 1 or 2 heteroatoms or heteroatom-containing groups selected from O, S, SO, SO₂, and NH as ring members, wherein the heteromonocyclic ring may be substituted with 1, 2, 3, 4, 5 or 6 substituents R⁷;
each R⁷ is independently selected from halogen, cyano, oxo, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, wherein the 8 last-mentioned substituents may carry a radical R⁸; C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, -N(R^{10a})R^{10b}, -C(=O)N(R^{10a})R^{10b}, -C(=S)N(R^{10a})R^{10b}, and -C(=O)R⁸;
R⁸ as a substituent on an aliphatic or cycloaliphatic group is selected from cyano, C₃-C₆-cycloalkyl which may be substituted by 1 or 2 substituents selected from CN, methyl and oxo; C₃-C₆-halocycloalkyl, and -OR⁹; and
R⁸ in the group -5(=O)R⁸ is selected from hydrogen, C₁-C₆-alkyl, C₁-C₆-haloalkyl, C₁-C₆-alkyl carrying a CN group; C₃-C₈-cycloalkyl, C₃-C₈-halocycloalkyl, C₃-C₈-cycloalkyl-C₁-C₄-alkyl, C₃-C₈-halocycloalkyl-C₁-C₄-alkyl, where the cycloalkyl moieties in the 4 last-mentioned radicals may carry a CN group; C₂-C₆-alkenyl, C₂-C₆-haloalkenyl, C₂-C₆-alkynyl, C₂-C₆-haloalkynyl, -OR⁹ and -N(R^{10a})R^{10b};
each R⁹ is independently selected from the group consisting of hydrogen, C₁-C₆-alkyl and C₁-C₆-haloalkyl;
R^{10a} is hydrogen or methyl; and R^{10b} is selected from hydrogen, C₁-C₆-alkyl, C₁-C₆-haloalkyl, -C(=O)R¹³, and -C(=O)N(R^{14a})R^{14b};
R¹³ is selected from C₁-C₆-alkyl, C₁-C₆-haloalkyl, C₃-C₆-cycloalkyl, C₃-C₆-halocycloalkyl, where the cycloalkyl moieties in the two last-mentioned groups may be substituted by a cyano group; C₁-C₄-alkoxy-C₁-C₄-alkyl, C₁-C₄-alkoxy and C₁-C₄-haloalkoxy;
R^{14a} is selected from hydrogen and C₁-C₆-alkyl;
R^{14b} is selected from hydrogen, C₁-C₆-alkyl and C₁-C₆-haloalkyl;
and the N-oxides, stereoisomers and agriculturally or veterinarily acceptable salts thereof;
except for compounds I wherein B¹ and B³ are C-CI, and simultaneously B² is C-H, G² is C-F, C-Cl or C-CH₃, and R⁵ and R⁶, together with the nitrogen atom they are bound to, form a ring selected from aziridin-1-yl, azetidin-1-yl, pyrrolidin-1-yl, piperidin-1-yl, thiazolidin-3-yl, morpholin-4-yl, thiomorpholin-4-yl, 1-oxo-1,4-thiazinan-4-yl and 1,1-dioxo-1,4-thiazinan-4-yl.

2. The compounds as claimed in claim 1, where R⁸ in the group -C(=O)R⁸ is selected from C₁-C₆-alkyl, C₁-C₆-haloalkyl, -OR⁹ and -N(R^{10a})R^{10b}.

3. The compounds as claimed in claim 1, where B¹ and B³ are CR², where R² is not hydrogen, and B² is CR², where R² has one of the meanings given in claim 1.

4. The compounds as claimed in claim 3, where B¹ and B³ are C-Cl and B² is C-F; or B¹ and B³ are C-CF₃ and B² is C-H; or B¹ and B³ are C-Br and B² is C-F; or B¹, B² and B³ are C-Cl; or B¹ is C-CI, B² is C-H and B³ is C-CF₃; or B¹ is C-Br, B² is C-H and B³ is C-CF³.

5. A compound according to claim 1 of formula C.1, which is selected from the following: 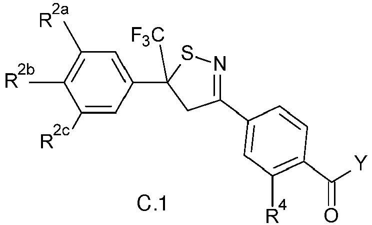
| Ex. | R^{2a}, R^{2b}, R^{2c} | R⁴ | Y |
|---|---|---|---|
| 1-1 | Cl, Cl, Cl | H | azetidin-1-yl |
| 1-2 | Cl, Cl, Cl | H | pyrrolidin-1-yl |
| 1-3 | Cl, F, Cl | Cl | 3,3-difluoro-pyrrolidin-1-yl |
| 1-4 | Cl, F, Cl | Cl | 3,3,4,4-tetrafluoro-pyrrolidin-1-yl |
| 1-5 | Cl, F, Cl | Cl | 4,4-difluoro-piperidine-1-yl |
| 1-6 | Cl, F, Cl | Cl | 3,3-difluoro-azetidin-1-yl |
| 1-7 | Cl, F, Cl | Cl | 4-cyano-1-piperidyl |
| 1-8 | Cl, F, Cl | Cl | 3-acetamidopyrrolidin-1-yl |
| 1-9 | Cl, F, Cl | Cl | 2-methoxycarbonylaziridin-1-yl |
| 1-10 | Cl, F, Cl | Cl | 3-[(2,2,2-trifluoroacetyl)amino]-pyrrolidin-1-yl |
| 1-11 | Cl, F, Cl | Cl | 4-acetylpiperazin-1-yl |
| 1-12 | Cl, F, Cl | Cl | 3-cyanopyrrolidin-1-yl |
| 1-13 | Cl, F, Cl | Cl | 2-(methylcarbamoyl)-pyrrolidin-1-yl |
| 1-14 | Cl, F, Cl | Cl | 2-(2,2,2-trifluoroethylcarbamoyl)-pyrrolidin-1-yl |
| 1-15 | Cl, F, Cl | Cl | 2,2,6,6-tetrafluoromorpholin-4-yl |
| 1-16 | Cl, F, Cl | Cl | 3-cyanoazetidin-1-yl |
| 1-17 | Cl, F, Cl | Cl | 2-methoxycarbonylazetidin-1-yl |
| 1-19 | Cl, F, Cl | Cl | 3-cyano-1-piperidyl |
| 1-20 | Cl, F, Cl | Cl | 3-(methylcarbamoyl)-pyrrolidin-1-yl |
| 1-21 | Cl, F, Cl | Cl | 3-(methylcarbamoyl)-1-piperidyl |
| 1-22 | Cl, F, Cl | Cl | 2-(2,2,2-trifluoroethylcarbamoyl)-azetidin-1-yl |
| 1-24 | Cl, F, Cl | Cl | 4-(methylcarbamoyl)-1-piperidyl |
| 1-25 | Cl, F, Cl | Cl | 2-(methylcarbamoyl)-1-azetidinyl |
| 1-26 | Cl, F, Cl | Cl | 3-(ethylcarbamoylamino)-1-pyrrolidinyl |
| 1-27 | Cl, F, Cl | Cl | 3-(3,3,3-trifluoropropanoylamino)-1-pyrrolidinyl |
| 1-28 | Cl, F, Cl | Cl | 3-(propanoylamino)-1-pyrrolidinyl |
| 1-29 | Cl, F, Cl | Cl | 3-(methoxycarbonylamino)-1-pyrrolidinyl |
| 1-30 | Cl, F, Cl | Cl | 3-acetamidoazetidin-1-yl |
| 1-31 | Cl, F, Cl | Cl | 3-(methylcarbamoylamino)pyrrolidin-1-yl |
| 1-32 | Cl, F, Cl | Cl | 3-(2,2,2-trifluoroethylcarbamoyl)pyrrolidin-1-yl |
| 1-34 | Cl, F, Cl | Cl | aziridin-1-yl |
| 1-35 | Cl, F, Cl | Cl | azetidin-1-yl |
| 1-36 | Cl, F, Cl | Cl | 2-cyanopyrrolidin-1-yl |
| 1-37 | Cl, F, Cl | Cl | 2-(2,2,2-trifluoroethylcarbamoyl)aziridin-1-yl |
| 1-38 | Cl, F, Cl | Cl | 3-[(2,2,2-trifluoroacetyl)amino]azetidin-1-yl |
| 1-39 | Cl, F, Cl | Cl | 3-(cyclobutanecarbonylamino)azetidin-1-yl |
| 1-40 | Cl, F, Cl | Cl | 3-[(2-methoxyacetyl)amino]azetidin-1-yl |
| 1-41 | Cl, F, Cl | Cl | 3-(propanoylamino)azetidin-1-yl |
| 1-42 | Cl, F, Cl | Cl | 3-(cyclopropanecarbonylamino)azetidin-1-yl |

6. An agricultural composition comprising at least one compound of the formula I, as defined in any of claims 1 to 5, a stereoisomer thereof and/or at least one agriculturally acceptable salt thereof, and at least one inert liquid and/or solid agriculturally acceptable carrier.

7. A veterinary composition comprising at least one compound of the formula I, as defined in any of claims 1 to 5, a stereoisomer thereof and/or at least one veterinarily acceptable salt thereof, and at least one inert liquid and/or solid veterinarily acceptable carrier.

8. A compound as defined in any of claims 1 to 5, or a stereoisomer and/or a veterinarily acceptable salt thereof, for use in treating or protecting an animal from infestation or infection by invertebrate pests.

9. A non-therapeutic method for controlling invertebrate pests which method comprises treating the pests, their food supply, their habitat or their breeding ground or a plant, plant propagation material, soil, area, material or environment in which the pests are growing or may grow, or the materials, plants, plant propagation material, soils, surfaces or spaces to be protected from invertebrate pest attack or infestation with a pesticidally effective amount of at least one imine compound of the formula I as defined in any of claims 1 to 5, a stereoisomer thereof and/or at least one agriculturally acceptable salt thereof.

10. The method as claimed in claim 9, for:
(i) protecting plants from attack or infestation by invertebrate pests, which method comprises treating the plants with a pesticidally effective amount of at least one compound of the formula I as defined in any of claims 1 to 5, a stereoisomer thereof and/or at least one agriculturally acceptable salt thereof; or
(ii) for protecting plant propagation material and/or the plants which grow therefrom from attack or infestation by invertebrate pests, which method comprises treating the plant propagation material with a pesticidally effective amount of at least one compound of the formula I as defined in any of claims 1 to 5, a stereoisomer thereof and/or at least one agriculturally acceptable salt thereof.

11. Plant propagation material, comprising at least one compound of the formula I as defined in any of claims 1 to 5, a stereoisomer thereof and/or at least one agriculturally acceptable salt thereof.

## Patentansprüche

1. Isothiazolinverbindungen der Formel I 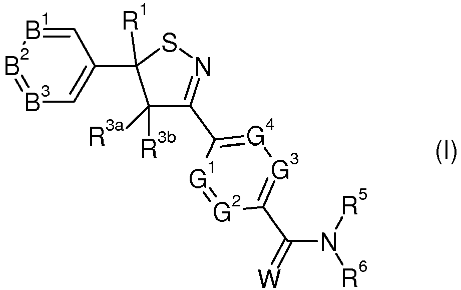 wobei:
B¹, B² und B³ jeweils unabhängig CR² sind;
G¹, G³ und G⁴ gleich CH sind und G² gleich CR⁴ ist;
W gleich O ist;
R¹ gleich CF₃ ist;
jedes R² unabhängig aus Wasserstoff, Halogen und C₁-C₂-Halogenalkyl ausgewählt ist;
R^{3a}, R^{3b} jeweils unabhängig aus Wasserstoff und Halogen ausgewählt sind;
R⁴ aus Wasserstoff, Halogen und Methyl ausgewählt ist;
R⁵ und R⁶ zusammen mit dem Stickstoffatom, an das sie gebunden sind, einen 3-, 4-, 5- oder 6-gliedrigen gesättigten heteromonocyclischen Ring bilden, wobei der Ring ferner 1 oder 2 Heteroatome oder Heteroatom enthaltende Gruppen, ausgewählt aus O, S, SO, SO₂ und NH, als Ringmitglieder enthalten kann, wobei der heteromonocyclische Ring mit 1, 2, 3, 4, 5 oder 6 R⁷-Substituenten substituiert sein kann;
jedes R⁷ unabhängig aus Halogen, Cyano, Oxo, C₁-C₆-Alkyl, C₁-C₆-Halogenalkyl, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, wobei die 8 zuletzt genannten Substituenten einen R⁸-Rest enthalten können; C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, -N(R^{10a})R^{10b}, -C(=O)N(R^{10a})R^{10b}, -C(=S)N(R^{10a})R^{10b} und -C(=O)R⁸ ausgewählt ist;
R⁸ als ein Substituent auf einer aliphatischen oder cycloaliphatischen Gruppe aus Cyano, C₃-C₆-Cycloalkyl, welches mit 1 oder 2 Substituenten, ausgewählt aus CN, Methyl und Oxo, substituiert sein kann; C₃-C₆-Halogencycloalkyl und -OR⁹ ausgewählt ist; und
R⁸ in der Gruppe -C(=O)R⁸ aus Wasserstoff, C₁-C₆-Alkyl, C₁-C₆-Halogenalkyl, einem CN-Gruppe enthaltenden C₁-C₆-Alkyl; C₃-C₈-Cycloalkyl, C₃-C₈-Halogencycloalkyl, C₃-C₈-Cycloalkyl-C₁-C₄-alkyl, C₃-C₈-Halogencycloalkyl-C₁-C₄-alkyl, wobei die Cycloalkyleinheiten in den 4 zuletzt genannten Resten eine CN-Gruppe enthalten können; C₂-C₆-Alkenyl, C₂-C₆-Halogenalkenyl, C₂-C₆-Alkinyl, C₂-C₆-Halogenalkinyl, -OR⁹ und -N(R^{10a})R^{10b} ausgewählt ist;
jedes R⁹ unabhängig aus der Gruppe bestehend aus Wasserstoff, C₁-C₆-Alkyl und C₁-C₆-Halogenalkyl ausgewählt ist;
R^{10a} Wasserstoff oder Methyl ist; und R^{10b} aus Wasserstoff, C₁-C₆-Alkyl, C₁-C₆-Halogenalkyl, -C(=O)R¹³ und -C(=O)N(R^{14a})R^{14b} ausgewählt ist;
R¹³ aus C₁-C₆-Alkyl, C₁-C₆-Halogenalkyl, C₃-C₆-Cycloalkyl, C₃-C₆-Halogencycloalkyl, wobei die Cycloalkyleinheiten in den zwei zuletzt genannten Gruppen mit einer Cyanogruppe substituiert sein können; C₁-C₄-Alkoxy-C₁-C₄-alkyl, C₁-C₄-Alkoxy und C₁-C₄-Halogenalkoxy ausgewählt ist;
R^{14a} aus Wasserstoff und C₁-C₆-Alkyl ausgewählt ist;
R^{14b} aus Wasserstoff, C₁-C₆-Alkyl und C₁-C₆-Halogenalkyl ausgewählt ist;
und die N-Oxide, Stereoisomere und landwirtschaftlich oder tiermedizinisch verträglichen Salze davon;
mit Ausnahme von Verbindungen I, wobei B¹ und B³ gleich C-Cl sind und gleichzeitig B² gleich C-H ist, G² gleich C-F, C-Cl oder C-CH₃ ist, und R⁵ und R⁶ zusammen mit dem Stickstoffatom, an das sie gebunden sind, einen Ring, ausgewählt aus Aziridin-1-yl, Azetidin-1-yl, Pyrrolidin-1-yl, Piperidin-1-yl, Thiazolidin-3-yl, Morpholin-4-yl, Thiomorpholin-4-yl, 1-Oxo-1,4-thiazinan-4-yl und 1,1-Dioxo-1,4-thiazinan-4-yl, bilden.

2. Die Verbindungen wie in Anspruch 1 beansprucht, wo R⁸ in der Gruppe -C(=O)R⁸ aus C₁-C₆-Alkyl, C₁-C₆-Halogenalkyl, -OR⁹ und -N(R^{10a})R^{10b} ausgewählt ist.

3. Die Verbindungen wie in Anspruch 1 beansprucht, wobei B¹ und B³ gleich CR² sind, wobei R² nicht Wasserstoff ist, und B² gleich CR² ist, wobei R² eine der in Anspruch 1 angegebenen Bedeutungen hat.

4. Die Verbindungen wie in Anspruch 3 beansprucht, wo B¹ und B³ gleich C-Cl sind und B² gleich C-F ist; oder B¹ und B³ gleich C-CF₃ sind und B² gleich C-H ist; oder B¹ und B³ gleich C-Br sind und B² gleich C-F ist; oder B¹, B² und B³ gleich C-Cl sind; oder B¹ gleich C-Cl ist, B² gleich C-H ist und B³ gleich C-CF₃ ist; oder B¹ gleich C-Br ist, B² gleich C-H ist und B³ gleich C-CF₃ ist.

5. Eine Verbindung nach Anspruch 1 der Formel C.1, welche aus den folgenden ausgewählt ist: 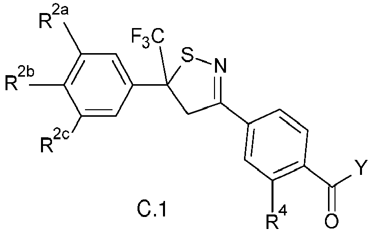
| Bsp. | R^{2a}, R^{2b}, R^{2c} | R⁴ | Y |
|---|---|---|---|
| 1-1 | Cl, Cl, Cl | H | Azetidin-1-yl |
| 1-2 | Cl, Cl, Cl | H | Pyrrolidin-1-yl |
| 1-3 | Cl, F, Cl | Cl | 3,3-Difluor-pyrrolidin-1-yl |
| 1-4 | Cl, F, Cl | Cl | 3,3,4,4-Tetrafluor-pyrrolidin-1-yl |
| 1-5 | Cl, F, Cl | Cl | 4,4-Difluor-piperidin-1-yl |
| 1-6 | Cl, F, Cl | Cl | 3,3-Difluor-azetidin- 1-yl |
| 1-7 | Cl, F, Cl | Cl | 4-Cyano-1-piperidyl |
| 1-8 | Cl, F, Cl | Cl | 3-Acetamidopyrrolidin-1-yl |
| 1-9 | Cl, F, Cl | Cl | 2-Methoxycarbonylaziridin- 1-yl |
| 1-10 | Cl, F, Cl | Cl | 3-[(2,2,2-Trifluoracetyl)amino]pyrrolidin-1-yl |
| 1-11 | Cl, F, Cl | Cl | 4-Acetylpiperazin-1-yl |
| 1-12 | Cl, F, Cl | Cl | 3-Cyanopyrrolidin-1-yl |
| 1-13 | Cl, F, Cl | Cl | 2-(Methylcarbamoyl)-pyrrolidin-1-yl |
| 1-14 | Cl, F, Cl | Cl | 2-(2,2,2-Trifluorethylcarbamoyl)-pyrrolidin-1-yl |
| 1-15 | Cl, F, Cl | Cl | 2,2,6,6-Tetrafluormorpholin-4-yl |
| 1-16 | Cl, F, Cl | Cl | 3-Cyanoazetidin-1-yl |
| 1-17 | Cl, F, Cl | Cl | 2-Methoxycarbonylazetidin-1-yl |
| 1-19 | Cl, F, Cl | Cl | 3-Cyano-1-piperidyl |
| 1-20 | Cl, F, Cl | Cl | 3-(Methylcarbamoyl)-pyrrolidin-1-yl |
| 1-21 | Cl, F, Cl | Cl | 3-(Methylcarbamoyl)-1-piperidyl |
| 1-22 | Cl, F, Cl | Cl | 2-(2,2,2-Trifluorethylcarbamoyl)-azetidin-1-yl |
| 1-24 | Cl, F, Cl | Cl | 4-(Methylcarbamoyl)-1-piperidyl |
| 1-25 | Cl, F, Cl | Cl | 2-(Methylcarbamoyl)-1-azetidinyl |
| 1-26 | Cl, F, Cl | Cl | 3-(Ethylcarbamoylamino)-1-pyrrolidinyl |
| 1-27 | Cl, F, Cl | Cl | 3-(3,3,3-Trifluorpropanoylamino)-1-pyrrolidinyl |
| 1-28 | Cl, F, Cl | Cl | 3-(Propanoylamino)-1-pyrrolidinyl |
| 1-29 | Cl, F, Cl | Cl | 3-(Methoxycarbonylamino)-1-pyrrolidinyl |
| 1-30 | Cl, F, Cl | Cl | 3-Acetamidoazetidin-1-yl |
| 1-31 | Cl, F, Cl | Cl | 3-(Methylcarbamoylamino)pyrrolidin-1-yl |
| 1-32 | Cl, F, Cl | Cl | 3-(2,2,2-Trifluorethylcarbamoyl)pyrrolidin-1-yl |
| 1-34 | Cl, F, Cl | Cl | Aziridin-1-yl |
| 1-35 | Cl, F, Cl | Cl | Azetidin-1-yl |
| 1-36 | Cl, F, Cl | Cl | 2-Cyanopyrrolidin-1-yl |
| 1-37 | Cl, F, Cl | Cl | 2-(2,2,2-Trifluorethylcarbamoyl)aziridin-1-yl |
| 1-38 | Cl, F, Cl | Cl | 3-[(2,2,2-Trifluoracetyl)amino]azetidin-1-yl |
| 1-39 | Cl, F, Cl | Cl | 3-(Cyclobutancarbonylamino)azetidin-1-yl |
| 1-40 | Cl, F, Cl | Cl | 3-[(2-Methoxyacetyl)amino]azetidin-1-yl |
| 1-41 | Cl, F, Cl | Cl | 3-(Propanoylamino)azetidin-1-yl |
| 1-42 | Cl, F, Cl | Cl | 3-(Cyclopropancarbonylamino)azetidin-1-yl |

6. Eine landwirtschaftliche Zusammensetzung, umfassend mindestens eine Verbindung der Formel I wie in einem der Ansprüche 1 bis 5 definiert, ein Stereoisomer davon und/oder mindestens ein landwirtschaftlich verträgliches Salz davon, und mindestens eine inerte Flüssigkeit und/oder einen festen landwirtschaftlich verträglichen Träger.

7. Eine tierärztliche Zusammensetzung, umfassend mindestens eine Verbindung der Formel I wie in einem der Ansprüche 1 bis 5 definiert, ein Stereoisomer davon und/oder mindestens ein tierärztlich verträgliches Salz davon, und mindestens eine inerte Flüssigkeit und/oder einen festen tierärztlich verträglichen Träger.

8. Eine Verbindung wie in einem der Ansprüche 1 bis 5 definiert oder ein Stereoisomer und/oder ein tierärztlich verträgliches Salz davon zur Verwendung zur Behandlung oder zum Schutz eines Tiers vor einem Befall oder einer Infektion durch wirbellose Schädlinge.

9. Ein nicht-therapeutisches Verfahren zur Bekämpfung von wirbellosen Schädlingen, wobei das Verfahren Behandeln der Schädlinge, ihrer Nahrungsmittelquelle, ihres Habitats oder ihrer Brutstätte oder einer Pflanze, eines Pflanzenwachstumsmaterials, Bodens, Gebiets, Materials oder einer Umgebung, in der Schädlinge wachsen oder wachsen können, oder der Materialien, Pflanzen, des Pflanzenwachstumsmaterials, der Böden, Oberflächen oder Bereiche, die vor dem Angriff oder Befall durch wirbellose Schädlinge zu schützen sind, mit einer pestizidisch wirksamen Menge mindestens einer Iminverbindung der Formel I wie in einem der Ansprüche 1 bis 5 definiert, einem Stereoisomer davon und/oder mindestens einem landwirtschaftlich verträglichen Salz davon.

10. Das Verfahren wie in Anspruch 9 beansprucht zum:
(i) Schutz von Pflanzen vor dem Angriff oder Befall durch wirbellose Schädlinge, wobei das Verfahren Behandeln der Pflanzen mit einer pestizidisch wirksamen Menge mindestens einer Verbindung der Formel I wie in einem der Ansprüche 1 bis 5 definiert, einem Stereoisomer davon und/oder mindestens einem landwirtschaftlich verträglichen Salz davon umfasst; oder
(ii) zum Schutz von Pflanzenwachstumsmaterial und/oder der Pflanzen, die daraus wachsen, vor dem Angriff oder Befall durch wirbellose Schädlinge, wobei das Verfahren Behandeln des Pflanzenwachstumsmaterials mit einer pestizidisch wirksamen Menge mindestens einer Verbindung der Formel I wie in einem der Ansprüche 1 bis 5 definiert, einem Stereoisomer davon und/oder mindestens einem landwirtschaftlich verträglichen Salz davon umfasst.

11. Pflanzenwachstumsmaterial, umfassend mindestens eine Verbindung der Formel I wie in einem der Ansprüche 1 bis 5 definiert, ein Stereoisomer davon und/oder mindestens ein landwirtschaftlich verträgliches Salz davon.

## Revendications

1. Composés d'isothiazoline de formule I 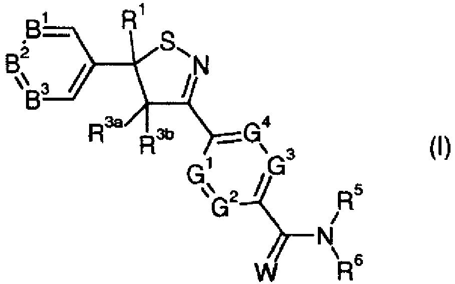 où:
B¹, B² et B³ sont chacun indépendamment CR²;
G¹, G³ et G⁴ sont CH et G² est CR⁴;
W est O;
R¹ est CF₃;
chaque R² est choisi indépendamment parmi l'hydrogène, un halogène et C₁-C₂-haloalkyle;
R^{3a}, R^{3b} sont choisis chacun indépendamment parmi l'hydrogène et un halogène;
R⁴ est choisi parmi l'hydrogène, un halogène et méthyle;
R⁵ et R⁶, avec l'atome d'azote auquel ils sont liés, forment un cycle hétéromonocyclique saturé à 3, 4, 5 ou 6 chaînons, où le cycle peut contenir en outre 1 ou 2 hétéroatomes ou groupes contenant un ou des hétéroatomes choisis parmi O, S, SO, SO₂ et NH comme chaînons du cycle, où le cycle hétéromonocyclique peut être substitué avec 1, 2, 3, 4, 5 ou 6 substituants R⁷;
chaque R⁷ est choisi indépendamment parmi un halogène, cyano, oxo, C₁-C₆-alkyle, C₁-C₆-haloalkyle, C₃-C₈-cycloalkyle, C₃-C₈-halocycloalkyle, C₂-C₆-alcényle, C₂-C₆-haloalcényle, C₂-C₆-alcynyle, C₂-C₆-haloalcynyle, où les 8 substituants mentionnés en dernier lieu peuvent porter un radical R⁸; C₁-C₆-alcoxy, C₁-C₆-haloalcoxy, C₁-C₆-alkylthio, C₁-C₆-haloalkylthio, C₁-C₆-alkylsulfinyle, C₁-C₆-haloalkylsulfinyle, C₁-C₆-alkylsulfonyle, C₁-C₆-haloalkylsulfonyle, -N(R^{10a})R^{10b}, -C(=O)N(R^{10a})R^{10b}, -C(=S)N(R^{10a})R^{10b} et -C(=O)R⁸;
R⁸ en tant que substituant sur un groupe aliphatique ou cycloaliphatique est choisi parmi cyano, C₃-C₆-cycloalkyle qui peut être substitué par 1 ou 2 substituants choisis parmi CN, méthyle et oxo; C₃-C₆-halocycloalkyle et -OR⁹; et
R⁸ dans le groupe -C(=O)R⁸ est choisi parmi l'hydrogène, C₁-C₆-alkyle, C₁-C₆-haloalkyle, C₁-C₆-alkyle portant un groupe CN; C₃-C₈-cycloalkyle, C₃-C₈-halocycloalkyle, C₃-C₈-cycloalkyl-C₁-C₄-alkyle, C₃-C₈-halocycloalkyl-C₁-C₄-alkyle, où les groupements cycloalkyle dans les 4 radicaux mentionnés en dernier lieu peuvent porter un groupe CN; C₂-C₆-alcényle, C₂-C₆-haloalcényle, C₂-C₆-alcynyle, C₂-C₆-haloalcynyle, -OR⁹ et -N(R^{10a})R^{10b};
chaque R⁹ est choisi indépendamment dans le groupe consistant en l'hydrogène, C₁-C₆-alkyle et C₁-C₆-haloalkyle;
R^{10a} est l'hydrogène ou méthyle; et R^{10b} est choisi parmi l'hydrogène, C₁-C₆-alkyle, C₁-C₆-haloalkyle, -C(=O)R¹³ et -C(=O)N(R^{14a})R^{14b};
R¹³ est choisi parmi C₁-C₆-alkyle, C₁-C₆-haloalkyle, C₃-C₆-cycloalkyle, C₃-C₆-halocycloalkyle, où les groupements cycloalkyle dans les deux groupes mentionnés en dernier lieu peuvent être substitués par un groupe cyano; C₁-C₄-alcoxy-C₁-C₄-alkyle, C₁-C₄-alcoxy et C₁-C₄-haloalcoxy;
R^{14a} est choisi parmi l'hydrogène et C₁-C₆-alkyle;
R^{14b} est choisi parmi l'hydrogène, C₁-C₆-alkyle et C₁-C₆-haloalkyle;
et leurs N-oxydes, stéréo-isomères et sels acceptables du point de vue agricole ou vétérinaire;
excepté pour les composés I où B¹ et B³ sont C-Cl, et simultanément B² est C-H, G² est C-F, C-Cl ou C-CH₃, et R⁵ et R⁶, avec l'atome d'azote auquel ils sont liés, forment un cycle choisi parmi aziridin-1-yle, azétidin-1-yle, pyrrolidin-1-yle, pipéridin-1-yle, thiazolidin-3-yle, morpholin-4-yle, thiomorpholin-4-yle, 1-oxo-1,4-thiazinan-4-yle et 1,1-dioxo-1,4-thiazinan-4-yle.

2. Composés tels que revendiqués dans la revendication 1, où R⁸ dans le groupe -C(=O)R⁸ est choisi parmi C₁-C₆-alkyle, C₁-C₆-haloalkyle, -OR⁹ et -N(R^{10a})R^{10b}.

3. Composés tels que revendiqués dans la revendication 1, où B¹ et B³ sont CR², où R² n'est pas l'hydrogène, et B² est CR², où R² a l'une des significations données dans la revendication 1.

4. Composés tels que revendiqués dans la revendication 3, où B¹ et B³ sont C-Cl et B² est C-F; ou B¹ et B³ sont C-CF₃ et B² est C-H; ou B¹ et B³ sont C-Br et B² est C-F; ou B¹, B² et B³ sont C-Cl; ou B¹ est C-Cl, B² est C-H et B³ est C-CF₃; ou B¹ est C-Br, B² est C-H et B³ est C-CF₃.

5. Composé selon la revendication 1 de formule C.1, qui est choisi parmi les suivants: 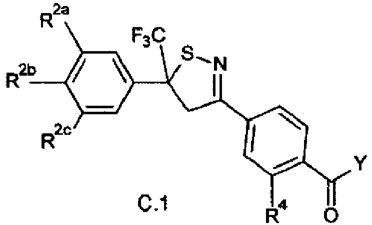
| Ex. | R^{2a}, R^{2b}, R^{2c} | R⁴ | Y |
|---|---|---|---|
| 1-1 | Cl, Cl, Cl | H | azétidin-1-yle |
| 1-2 | Cl, Cl, Cl | H | pyrrolidin-1-yle |
| 1-3 | Cl, F, Cl | Cl | 3,3-difluoro-pyrrolidin-1-yle |
| 1-4 | Cl, F, Cl | Cl | 3,3,4,4-tétrafluoro-pyrrolidin-1-yle |
| 1-5 | Cl, F, Cl | Cl | 4,4-difluoro-pipéridin-1-yle |
| 1-6 | Cl, F, Cl | Cl | 3,3-difluoro-azétidin-1-yle |
| 1-7 | Cl, F, Cl | Cl | 4-cyano-1-pipéridyle |
| 1-8 | Cl, F, Cl | Cl | 3-acétamidopyrrolidin-1-yle |
| 1-9 | Cl, F, Cl | Cl | 2-méthoxycarbonylaziridin-1-yle |
| 1-10 | Cl, F, Cl | Cl | 3-[(2,2,2-trifluoroacétyl)amino]-pyrrolidin-1-yle |
| 1-11 | Cl, F, Cl | Cl | 4-acétylpipérazin-1-yle |
| 1-12 | Cl, F, Cl | Cl | 3-cyanopyrrolidin-1-yle |
| 1-13 | Cl, F, Cl | Cl | 2-(méthylcarbamoyl)-pyrrolidin-1-yle |
| 1-14 | Cl, F, Cl | Cl | 2-(2,2,2-trifluoroéthylcarbamoyl)-pyrrolidin-1-yle |
| 1-15 | Cl, F, Cl | Cl | 2,2,6,6-tétrafluoromorpholin-4-yle |
| 1-16 | Cl, F, Cl | Cl | 3-cyanoazétidin-1-yle |
| 1-17 | Cl, F, Cl | Cl | 2-méthoxycarbonylazétidin-1-yle |
| 1-19 | Cl, F, Cl | Cl | 3-cyano-1-pipéridyle |
| 1-20 | Cl, F, Cl | Cl | 3-(méthylcarbamoyl)-pyrrolidin-1-yle |
| 1-21 | Cl, F, Cl | Cl | 3-(méthylcarbamoyl)-1-pipéridyle |
| 1-22 | Cl, F, Cl | Cl | 2-(2,2,2-trifluoroéthylcarbamoyl)-azétidin-1-yle |
| 1-24 | Cl, F, Cl | Cl | 4-(méthylcarbamoyl)-1-pipéridyle |
| 1-25 | Cl, F, Cl | Cl | 2-(méthylcarbamoyl)-1-azétidinyle |
| 1-26 | Cl, F, Cl | Cl | 3-(éthylcarbamoylamino)-1-pyrrolidinyle |
| 1-27 | Cl, F, Cl | Cl | 3-(3,3,3-trifluoropropanoylamino)-1-pyrrolidinyle |
| 1-28 | Cl, F, Cl | Cl | 3-(propanoylamino)-1-pyrrolidinyle |
| 1-29 | Cl, F, Cl | Cl | 3-(méthoxycarbonylamino)-1-pyrrolidinyle |
| 1-30 | Cl, F, Cl | Cl | 3-acétamidoazétidin-1-yle |
| 1-31 | Cl, F, Cl | Cl | 3-(méthylcarbamoylamino)pyrrolidin-1-yle |
| 1-32 | Cl, F, Cl | Cl | 3-(2,2,2-trifluoroéthylcarbamoyl)pyrrolidin-1-yle |
| 1-34 | Cl, F, Cl | Cl | aziridin-1-yle |
| 1-35 | Cl, F, Cl | Cl | azétidin-1-yle |
| 1-36 | Cl, F, Cl | Cl | 2-cyanopyrrolidin-1-yle |
| 1-37 | Cl, F, Cl | Cl | 2-(2,2,2-trifluoroéthylcarbamoyl)aziridin-1-yle |
| 1-38 | Cl, F, Cl | Cl | 3-[(2,2,2-trifluoroacétyl)amino]azétidin-1-yle |
| 1-39 | Cl, F, Cl | Cl | 3-(cyclobutanecarbonylamino)azétidin-1-yle |
| 1-40 | Cl, F, Cl | Cl | 3-[(2-méthoxyacétyl)amino]azétidin-1-yle |
| 1-41 | Cl, F, Cl | Cl | 3-(propanoylamino)azétidin-1-yle |
| 1-42 | Cl, F, Cl | Cl | 3-(cyclopropanecarbonylamino)azétidin-1-yle |

6. Composition agricole comprenant au moins un composé de formule I, tel que défini dans l'une quelconque des revendications 1 à 5, un stéréo-isomère de celui-ci et/ou au moins un sel acceptable du point de vue agricole de celui-ci, et au moins un vecteur acceptable du point de vue agricole liquide et/ou solide inerte.

7. Composition vétérinaire comprenant au moins un composé de formule I, tel que défini dans l'une quelconque des revendications 1 à 5, un stéréo-isomère de celui-ci et/ou au moins un sel acceptable du point de vue vétérinaire de celui-ci, et au moins un vecteur acceptable du point de vue vétérinaire liquide et/ou solide inerte.

8. Composé tel que défini dans l'une quelconque des revendications 1 à 5, ou stéréo-isomère de celui-ci et/ou sel acceptable du point de vue vétérinaire de celui-ci, destiné à être utilisé dans le traitement ou la protection d'un animal contre une infestation ou une infection par des nuisibles invertébrés.

9. Procédé non thérapeutique pour lutter contre les nuisibles invertébrés, lequel procédé comprend le traitement des nuisibles, de leur apport de nourriture, de leur habitat ou de leur aire de reproduction ou d'une plante, d'un matériel de propagation des plantes, d'un sol, d'une aire, d'un matériau ou d'un environnement dans lequel les nuisibles se développement ou peuvent se développer, ou des matériaux, des plantes, d'un matériel de propagation des plantes, de sols, de surfaces ou d'espaces destinés à être protégés contre une attaque ou une infestation par des nuisibles invertébrés avec une quantité efficace du point de vue pesticide d'au moins un composé d'imine de formule I tel que défini dans l'une quelconque des revendications 1 à 5, d'un stéréo-isomère de celui-ci et/ou d'au moins un sel acceptable du point de vue agricole de celui-ci.

10. Procédé selon la revendication 9, pour:
(i) protéger des plantes contre une attaque ou une infestation par des nuisibles invertébrés, lequel procédé comprend le traitement des plantes avec une quantité efficace du point de vue pesticide d'au moins un composé de formule I tel que défini dans l'une quelconque des revendications 1 à 5, d'un stéréo-isomère de celui-ci et/ou d'au moins un sel acceptable du point de vue agricole de celui-ci; ou
(ii) pour protéger un matériel de propagation des plantes et/ou les plantes qui poussent à partir de celui-ci contre une attaque ou une infestation par des nuisibles invertébrés, lequel procédé comprend le traitement du matériel de propagation des plantes avec une quantité efficace du point de vue pesticide d'au moins un composé de formule I tel que défini dans l'une quelconque des revendications 1 à 5, d'un stéréo-isomère de celui-ci et/ou d'au moins un sel acceptable du point de vue agricole de celui-ci.

11. Matériel de propagation des plantes, comprenant au moins un composé de formule I tel que défini dans l'une quelconque des revendications 1 à 5, un stéréo-isomère de celui-ci et/ou au moins un sel acceptable du point de vue agricole de celui-ci.
